# EUROPEAN PATENT APPLICATION

(11) **EP 4 534 566 A1**
(43) Date of publication of application: **09.04.2025**
(21) Application number: 23816133.5
(22) Date of filing: 31.05.2023
(51) Int. Cl.: C08B 37/08, A61K 39/00, A61K 39/39, A61K 47/36, A61P 31/00, A61P 35/00, A61P 37/04

(54) **HYALURONIC ACID DERIVATIVE, PHARMACEUTICAL COMPOSITION, AND METHOD FOR PRODUCING PHARMACEUTICAL COMPOSITION**

(30) Priority: 31.05.2022 JP 2022088995
(71) Applicant: Asahi Kasei Kabushiki Kaisha, Tokyo 1000006 (JP)
(72) Inventor: YABUUCHI Kohei, Tokyo 100-0006 (JP); NAKAI Takashi, Tokyo 100-0006 (JP); MOMOSE Fumiyasu, Tsu-shi, Mie 514-8507 (JP)
(74) Representative: Hoffmann Eitle
(86) International application number: PCT/JP2023/020372
(87) International publication number: WO 2023/234376

(57) **Abstract**

A hyaluronic acid derivative into which a steryl group is introduced according to the present invention is such that a ratio A1/A2 of areas A1 and A2, calculated by the following method from a chromatogram derived through gel permeation chromatography measurement, is 0.90 or greater. A2 is an area value surrounded by: a curve from an intersection point (Ub), at which a perpendicular line drawn from a refractive index intensity maximum point (Kb) on a chromatogram of 50 kDa polyacrylic acid serving as a standard substance to a baseline (B) intersects a chromatogram of the hyaluronic acid derivative, to the end point of the chromatogram of the hyaluronic acid derivative; a perpendicular line drawn from the refractive index intensity maximum point (Kb) to the baseline (B); and the baseline (B). A1 is an area value surrounded by a curve from the starting point of the chromatogram of the hyaluronic acid derivative to the intersection point (Ub), a perpendicular line drawn from the refractive index intensity maximum point (Kb) to the baseline (B), and the baseline (B).

## Description

### TECHNICAL FIELD

The present invention relates to a hyaluronic acid derivative, a pharmaceutical composition, and a method for producing a pharmaceutical composition.

The present invention claims priority on the basis of Japanese Patent Application No. 2022-088995 filed in Japan on May 31, 2022, the contents of which are incorporated herein by reference.

### BACKGROUND ART

In recent years, biopharmaceuticals that contain proteins, peptides, or nucleic acids as active ingredients have been put into practical use, and their number continues to increase year by year. Biopharmaceuticals can fill unmet medical needs that could not be satisfied by conventional small molecular drugs. However, there are problems in that biopharmaceuticals are difficult to be absorbed from the gastrointestinal tract or mucous membranes, are unstable in vivo, and have a short half-life in the blood. Therefore, biopharmaceuticals require frequent administration by injection, which places a heavy burden on both patients and medical personnel. Therefore, drug base materials (base materials of a sustained release drug delivery system) that can encapsulate biopharmaceuticals without impairing pharmacological activity and gradually release active ingredients thereof in vivo are required.

Against such a background, Patent Document 1 proposes a base material for a sustained release drug delivery system, the material being made of a highly safe hyaluronic acid derivative. This hyaluronic acid derivative spontaneously associates in an aqueous solution, can efficiently encapsulate drugs, especially biopharmaceuticals, while maintaining biological activities thereof, and aggregates under physiological saline concentrations (or disperses even under physiological saline concentrations) and exhibits good retention in the blood. When biopharmaceuticals are used as active ingredients, especially, the hyaluronic acid derivative can be used as a carrier that can efficiently encapsulate many drugs while maintaining pharmacological activities thereof, a sustained release carrier into the blood that exhibits a good retention in the blood, or a targeting carrier, and is considered usable as a local (such as subcutaneous) sustained release carrier that allows continuous sustained-release of drugs.

Furthermore, cancer vaccines using the above-mentioned hyaluronic acid derivatives as carriers have also been reported (see, for example, Patent Document 2).

### Citation List

### Patent Document

Patent Document 1: International Patent Application Publication No. 2010/053140
Patent Document 2: International Patent Application Publication No. 2020/158771

### SUMMARY OF INVENTION

### Technical Problem

However, the hyaluronic acid derivative used in Patent Document 2 or the like has a broad and non-uniform particle size distribution, and the relationship between the particle size distribution of the hyaluronic acid derivative and its delivery to the immune cells in lymph nodes or activation ability of the immune cells has not been specifically investigated, and there is a room for improvement.

The present invention has been made in view of the above-mentioned circumstances, and provides a hyaluronic acid derivative which exhibits excellent delivery properties to immune cells in lymph nodes and activation ability against the immune cells, when formulated with a medicinal ingredient, as well as a pharmaceutical composition using the hyaluronic acid derivative and a method for producing the same.

### Solution to Problem

The present invention encompasses the following aspects.
(1) A hyaluronic acid derivative having an introduced steryl group, wherein
   the ratio A1/A2 of areas A1 and A2 calculated by the following method from chromatograms obtained by gel permeation chromatography measurements is 0.90 or more,
   (i) an intersection point Ub is determined by: a perpendicular line drawn from the maximum point Kb of the refractive index intensity on the chromatogram of a 50 kDa polyacrylic acid serving as a standard substance to the baseline B; and the chromatogram of the hyaluronic acid derivative; and
   (ii) the area A2 surrounded by: a curve from the intersection point Ub to the end point on the chromatogram of the hyaluronic acid derivative; a perpendicular line drawn from the maximum point Kb of the refractive index intensity to the baseline B; and the baseline is determined, and the area A1 surrounded by: a curve from the starting point to the intersection point Ub on the chromatogram of the hyaluronic acid derivative; the perpendicular line drawn from the maximum point Kb of the refractive index intensity to the baseline B; and the baseline is determined.
(2) The hyaluronic acid derivative according to (1), wherein the ratio Da/Db of distances Da and Db calculated by the following method from chromatograms obtained by a gel permeation chromatography measurement is more than 0.00 and 1.20 or less.
   (i) an intersection point Ba is determined by: a perpendicular line drawn from the maximum point Ka of the refractive index intensity on the chromatogram of a 150 kDa polyacrylic acid serving as a standard substance to the baseline B; and the baseline, and the length La between the maximum point Ka of the refractive index intensity and the intersection point Ba is determined;
   (ii) among two points at which the refractive index intensity on the chromatogram reaches La/20, one point at which an elution time is earlier is determined as the point R1, and the other point at which the elution time is later is determined as the point S1;
   (iv) an intersection point Ta is determined by: a straight line D1 that connects the point R1 and the point S1; and the perpendicular line drawn from the maximum point Ka of the refractive index intensity to the baseline B, and an intersection point Tb is determined by: the perpendicular line drawn from the maximum point Kb of the refractive index intensity to the baseline B; and the straight line D1; and
   (v) the distance Da is determined as the distance between the point R1 and the intersection point Ta, and the distance Db is determined as the distance between the intersection point Ta and the intersection point Tb.
(3) The hyaluronic acid derivative according to (1) or (2), wherein the ratio A1/A2 of the areas A1 and A2 is 1.70 or more.
(4) A hyaluronic acid derivative having an introduced steryl group, wherein
   the ratio Pt/Pr of the retention time Pt at the maximum point of the refractive index intensity of the hyaluronic acid derivative relative to the retention time Pr at the maximum point of the refractive index intensity of a 50 kDa polyacrylic acid as a standard substance, calculated from chromatograms obtained by gel permeation chromatography measurements, is 0.5 or more and less than 1.0.
(5) A hyaluronic acid derivative having an introduced steryl group, wherein
   the weight-average molecular weight of the hyaluronic acid derivative is 110,000 or more and less than 500,000, the weight-average molecular weight being calculated from a chromatogram obtained by a gel permeation chromatography measurement based on a calibration curve obtained using polyacrylic acids having a molecular weight of 2 kDa, 4 kDa, 8 kDa, 18 kDa, 40 kDa, or 150 kDa as standard substances.
(6) The hyaluronic acid derivative according to any one of (1) to (5), wherein the hyaluronic acid derivative has at least one repeating unit of the following general formula (I). 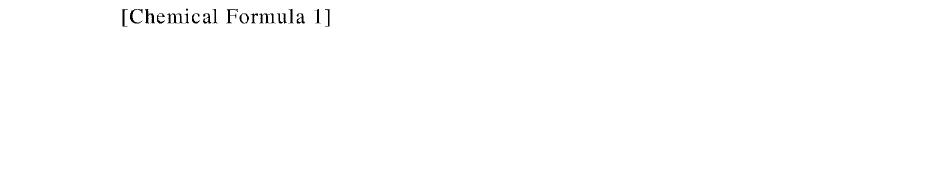 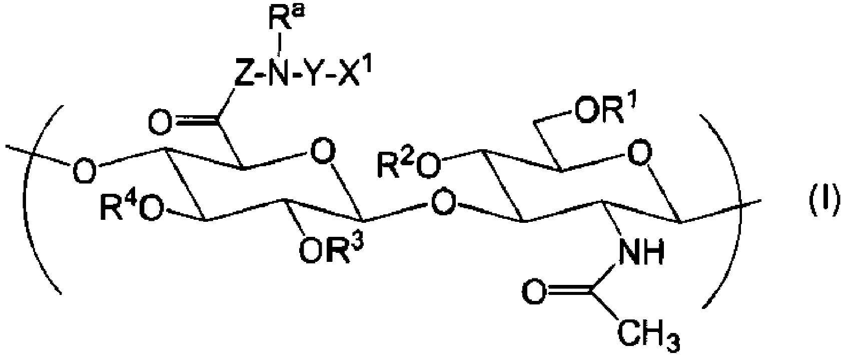
   (In the formula, each of R¹, R², R³ and R⁴ is independently selected from the group consisting of a hydrogen atom, C₁₋₆ alkyls, a formyl and C₁₋₆ alkylcarbonyls;
   Z is a direct bond or a peptide linker consisting of 2 to 30 arbitrary amino acid residues;
   X¹ is a group selected from the group consisting of:
      -NR^{b}-R,
      -NR^{b}-COO-R,
      -NR^{b}-CO-R,
      -NR^{b}-CO-NR^{c}-R,
      -COO-R,
      -O-COO-R,
      -S-R,
      -CO-Y^{a}-S-R,
      -O-CO-Y^{b}-S-R,
      -NR^{b}-CO-Y^{b}-S-R, and
      -S-S-R;
      each of R^{a}, R^{b} and R^{c} is independently selected from the group consisting of a hydrogen atom, C₁₋₂₀ alkyls, amino C₂₋₂₀ alkyls and hydroxy C₂₋₂₀ alkyls, in an alkyl moiety of which a group selected from the group consisting of -O- and -NR^{f}- may be inserted;
      R^{f} is selected from the group consisting of a hydrogen atom, C₁₋₁₂ alkyls, amino C₂₋₁₂ alkyls and hydroxy C₂₋₁₂ alkyls, in an alkyl moiety of which a group selected from the group consisting of -O- and -NH- may be inserted;
      R is a steryl group;
      Y is C₂₋₃₀ alkylene or (CH₂CH ₂O)ₘ-CH₂CH₂-, and a group selected from the group consisting of -O-, -NR^{g}- and -S-S- may be inserted in the alkylene;
      R^{g} is selected from the group consisting of a hydrogen atom, C₁₋₂₀ alkyls, amino C₂₋₂₀ alkyls and hydroxy C₂₋₂₀ alkyls, in an alkyl moiety of which a group selected from the group consisting of -O- and -NH- may be inserted;
      Y^{a} is a C₁₋₅ alkylene;
      Y^{b} is a C₂₋₈ alkylene or a C₂₋₈ alkenylene; and
      m is an integer of 1 or more and 100 or less.)
(7) The hyaluronic acid derivative according to any one of (1) to (6), wherein the steryl group is a cholesteryl group.
(8) The hyaluronic acid derivative according to any one of (1) to (7), wherein the introduction ratio of the steryl group relative to a disaccharide repeating unit that constitutes the hyaluronic acid derivative is 30% or more and 60% or less.
(9) The hyaluronic acid derivative according to any one of (1) to (8), wherein the weight-average molecular weight (absolute molecular weight) of the hyaluronic acid derivative is 4,000 or more and 1,000,000 or less.
(10) The hyaluronic acid derivative according to (9), wherein the weight-average molecular weight (absolute molecular weight) of the hyaluronic acid derivative is 5000 or more and 25,000 or less.
(11) A pharmaceutical composition containing: the hyaluronic acid derivative of any one of (1) to (10); and a medicinal ingredient.
(12) The pharmaceutical composition according to (11), wherein the pharmaceutical composition is used to prevent or treat at least one disease selected from the group consisting of cancers, infectious diseases and immune diseases.
(13) The pharmaceutical composition according to (11) or (12), wherein the medicinal ingredient contains at least one selected from the group consisting of cancer antigens, antigens derived from infectious diseases, and autoantigens in immune diseases; and the pharmaceutical composition further contains an adjuvant.
(14) The pharmaceutical composition according to any one of (11) to (13), wherein the medicinal ingredient contains: a cancer antigen or an antigen derived from infectious diseases; and the pharmaceutical composition further contains an adjuvant.
(15) A method for producing a pharmaceutical composition containing: the hyaluronic acid derivative of any one of (1) to (10); and a medicinal ingredient, the method including:
   a preparation step in which the medicinal ingredient is dissolved in an organic solvent or an organic solvent containing water to prepare an oil phase containing the medicinal ingredient; and
   a mixing step in which the oil phase and an aqueous phase containing the hyaluronic acid derivative are mixed such that the mixing ratio of the oil phase and the aqueous phase, based on volume, becomes 20:100 to 0.01:100.
(16) The method for producing a pharmaceutical composition according to (15), wherein the pH of the aqueous phase containing the hyaluronic acid derivative is 6.00 or more and 11.00 or less.

### Advantageous Effects of Invention

The hyaluronic acid derivative according to the above-mentioned aspect makes it possible to provide a hyaluronic acid derivative that exhibits excellent delivery properties to immune cells in lymph nodes and activation ability against the immune cells, when formulated with a medicinal ingredient. The pharmaceutical composition according to the above-mentioned aspect contains the above-mentioned hyaluronic acid derivative and exhibits excellent delivery properties to immune cells in lymph nodes and activation ability against the immune cells. The method for producing a pharmaceutical composition according to the above-mentioned aspect uses the above-mentioned hyaluronic acid derivative, and provides a pharmaceutical composition having excellent delivery properties to immune cells in lymph nodes and activation ability against the immune cells.

### BRIEF DESCRIPTION OF DRAWINGS

[Fig. 1] One example of a gel permeation chromatogram of a hyaluronic acid derivative of the present embodiment is shown.
[Fig. 2] One example of a gel permeation chromatogram of a hyaluronic acid derivative of the present embodiment is shown.
[Fig. 3A] A gel permeation chromatogram of a hyaluronic acid derivative of Synthesis Example 1-1 is shown.
[Fig. 3B] A gel permeation chromatogram of a hyaluronic acid derivative of Synthesis Example 1-2 is shown.
[Fig. 3C] A gel permeation chromatogram of a hyaluronic acid derivative of Synthesis Example 1-3 is shown.
[Fig. 4A] A gel permeation chromatogram of a hyaluronic acid derivative of Example 1-1 is shown.
[Fig. 4B] A gel permeation chromatogram of a hyaluronic acid derivative of Example 1-2 is shown.
[Fig. 4C] A gel permeation chromatogram of a hyaluronic acid derivative of Example 1-3 is shown.
[Fig. 5A] A gel permeation chromatogram of a hyaluronic acid derivative of Comparative Example 1-1 is shown.
[Fig. 5B] A gel permeation chromatogram of a hyaluronic acid derivative of Comparative Example 1-2 is shown.
[Fig. 5C] A gel permeation chromatogram of a hyaluronic acid derivative of Comparative Example 1-3 is shown.
[Fig. 5D] A gel permeation chromatogram of a cholesteryl-modified pullulan (CHP) of Comparative Example 1-4 is shown.
[Fig. 6A] Gel permeation chromatograms of a hyaluronic acid derivative of Example 1-1 and a hyaluronic acid derivative in a pharmaceutical composition of Example 2-1 are shown.
[Fig. 6B] Gel permeation chromatograms of a hyaluronic acid derivative of Example 1-2 and a hyaluronic acid derivative in a pharmaceutical composition of Example 2-2 are shown.
[Fig. 6C] Gel permeation chromatograms of a hyaluronic acid derivative of Example 1-3 and a hyaluronic acid derivative in a pharmaceutical composition of Example 2-3 are shown.
[Fig. 7A] A graph of one example of a FACS analysis when the ratio (%) of peptide-uptake dendritic cells (DC) expressing CD80 relative to peptide-uptake DC in Test Example 1-1 was calculated is shown.
[Fig. 7B] A graph of one example of a FACS analysis when the ratio (%) of peptide-uptake DC expressing CD86 relative to peptide-uptake DC in Test Example 1-1 was calculated is shown.
[Fig. 7C] A graph of one example of a FACS analysis when the ratio (%) of peptide-uptake conventional type 1 dendritic cells (cDC1) expressing CD80 relative to peptide-uptake cDC1 in Test Example 1-1 was calculated is shown.
[Fig. 7D] A graph of one example of a FACS analysis when the ratio (%) of peptide-uptake cDC1 expressing CD86 relative to peptide-uptake cDC1 in Test Example 1-1 was calculated is shown.
[Fig. 7E] A graph of one example of a FACS analysis when the ratio (%) of peptide-uptake macrophage cells (Mph) expressing CD80 relative to peptide-uptake Mph in Test Example 1-1 was calculated is shown.
[Fig. 7F] A graph of one example of a FACS analysis when the ratio (%) of peptide-uptake macrophage (Mph) expressing CD86 relative to peptide-uptake Mph in Test Example 1-1 was calculated is shown.
[Fig. 8] Gel permeation chromatograms of hyaluronic acid derivatives of Test Examples 2-1 to 2-8 are shown.
[Fig. 9] Gel permeation chromatograms of hyaluronic acid derivatives of Test Examples 3-1 to 3-6 are shown.
[Fig. 10] A gel permeation chromatogram of a hyaluronic acid derivative of Comparative Example 5-1 is shown.
[Fig. 11] Photographs of solutions obtained when a complex of an antigenic peptide and a hyaluronic acid was tried to be prepared in Comparative Example 5-1 or 5-2, and a solution obtained when a complex of an antigenic peptide and a hyaluronic acid derivative was prepared in Example 7-1-1 are shown.
[Fig. 12] A gel permeation chromatogram of a hyaluronic acid in Comparative Example 5-2 is shown.
[Fig. 13] A gel permeation chromatogram of a hyaluronic acid derivative in Comparative Example 6-1 is shown.
[Fig. 14] A gel permeation chromatogram of a hyaluronic acid derivative in Example 6-1 is shown.
[Fig. 15] Graphs of IgG antibody titer (OD value) in Example 6-1-1 and Comparative Example 6-1-1 are shown.
[Fig. 16] Graphs of IgG antibody titer (OD value) in Example 6-1-1 and Comparative Example 6-1-1 are shown.
[Fig. 17] Graphs of IgG2a antibody titer (OD value) in Example 6-1-1 and Comparative Example 6-1-1 are shown.
[Fig. 18A] Graphs of IgG antibody titer (OD value) at 10-fold dilution (10⁻¹ titer) in Example 6-1-1 and Comparative Example 6-1-1 are shown.
[Fig. 18B] Graphs of IgG1 antibody titer (OD value) at 10-fold dilution (10⁻¹ titer) in Example 6-1-1 and Comparative Example 6-1-1 are shown.
[Fig. 18C] Graphs of IgG2a antibody titer (OD value) at 10-fold dilution (10⁻¹ titer) in Example 6-1-1 and Comparative Example 6-1-1 are shown.
[Fig. 19] A gel permeation chromatogram of a hyaluronic acid derivative in Example 7-1 is shown.
[Fig. 20] A gel permeation chromatogram of a pharmaceutical composition containing a complex of an antigenic peptide and a hyaluronic acid derivative in Example 7-1-1 is shown.
[Fig. 21] A gel permeation chromatogram of a hyaluronic acid derivative obtained in Example 1-1 is shown.
[Fig. 22] A gel permeation chromatogram of a hyaluronic acid derivative obtained in Example 1-1 is shown.
[Fig. 23] Graphs of tumor areas (average values) over time in Test Example 6 are shown.

### DESCRIPTION OF EMBODIMENTS

Hereinafter, an embodiment of the present invention (hereinafter, referred to as "the present embodiment") will be described in detail, but the present invention is not limited thereto, and various modifications may be made without departing from the gist thereof.

Hereinafter, terms used in the present specification will be explained.

The term "C₁₋₂₀ alkyl" used in the present specification refers to a linear or branched alkyl group having 1 to 20 carbon atoms, and encompasses "C₁₋₄ alkyls" such as methyl, ethyl, n-propyl, iso-propyl, n-butyl, sec-butyl, iso-butyl, and tert-butyl; and n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, and 2-ethylbutyl. The term "C₁₋₂₀ alkyl" encompasses C₁₋₁₂ alkyls having 1 to 12 carbon atoms, and C₁₋₆ alkyl groups having 1 to 6 carbon atoms.

The term "C₁₋₆ alkylcarbonyl" used in the present specification refers to an alkylcarbonyl group in which the alkyl moiety is the above-mentioned C₁₋₆ alkyl, and encompasses "C₁₋₄ alkylcarbonyls" such as acetyl, propionyl, n-propylcarbonyl, iso-propylcarbonyl, n-butylcarbonyl, sec-butylcarbonyl, iso-butylcarbonyl, and tert-butylcarbonyl.

The term "amino C₂₋₂₀ alkyl" used in the present specification refers to a linear or branched alkyl having 2 to 20 carbon atoms with an amino group as a substituent, and the amino group may be located on the terminal carbon atom of the alkyl group, for example. The term "amino C₂₋₂₀ alkyl" encompasses amino C₂₋₁₂ alkyls having 2 to 12 carbon atoms.

The term "hydroxy C₂₋₂₀ alkyl" used in the present specification refers to a linear or branched alkyl group having 2 to 20 carbon atoms with a hydroxy group as a substituent, and the hydroxy group may be located on the terminal carbon atom of the alkyl group, for example. The term "hydroxy C₂₋₂₀ alkyl" encompasses hydroxyC₂₋₁₂ alkyls having 2 to 12 carbon atoms.

The term "C₂₋₃₀ alkylene" used in the present specification refers to a linear or branched divalent saturated hydrocarbon group having 2 to 30 carbon atoms, such as ethylene and propylene, and encompasses C₂₋₂₀ alkylenes having 2 to 20 carbon atoms, C₂₋₈ alkylenes having 2 to 8 carbon atoms, and a group of "-(CH₂)ₙ-" (in which n is 2 to 30, preferably 2 to 20, and more preferably 2 to 15).

The term "C₁₋₅ alkylene" used in the present specification refers to a linear or branched divalent saturated hydrocarbon group having 1 to 5 carbon atoms, such as methylene, ethylene, and propylene.

The term "C₂₋₈ alkenylene" used in the present specification refers to a linear or branched divalent saturated hydrocarbon group having at least one double bond with 2 to 8 carbon atoms, such as -CH=CH-, -C(CH₃)=CH-, 2-butene-1,4-diyl, hepta-2,4-diene-1,6-diyl, and octa-2,4,6-triene-1,8-diyl. When geometrical isomers exist, isomers and mixtures thereof are also included.

### <<Hyaluronic acid derivative>>

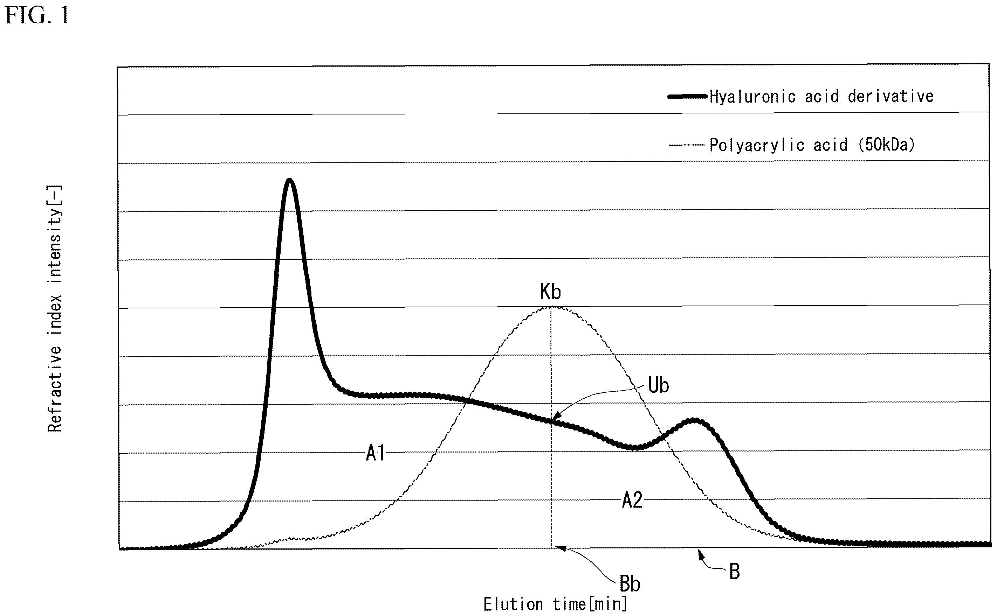

A hyaluronic acid derivative of the first embodiment of the present invention is a hyaluronic acid derivative having an introduced steryl group, wherein
the ratio A1/A2 of areas A1 and A2 calculated by the following method from chromatograms shown in Fig. 1, the chromatograms being obtained by gel permeation chromatography measurements, is 0.90 or more.
(i) An intersection point Ub is determined by: a perpendicular line drawn from the maximum point Kb of the refractive index intensity on a chromatogram of a 50 kDa polyacrylic acid serving as a standard substance to the baseline B; and the chromatogram of the hyaluronic acid derivative, and an intersection point Bb is determined by: a perpendicular line drawn from the maximum point Kb of the refractive index intensity to the baseline B; and the baseline; and
(ii) the area A2 surrounded by: a curve from the intersection point Ub to the end point on the chromatogram of the hyaluronic acid derivative; a perpendicular line drawn from the maximum point Kb of the refractive index intensity to the baseline B; and the baseline is determined, and the area A1 surrounded by: a curve from the starting point to the intersection point Ub on the chromatogram of the hyaluronic acid derivative; a perpendicular line drawn from the maximum point Kb of the refractive index intensity to the baseline B; and the baseline is determined.

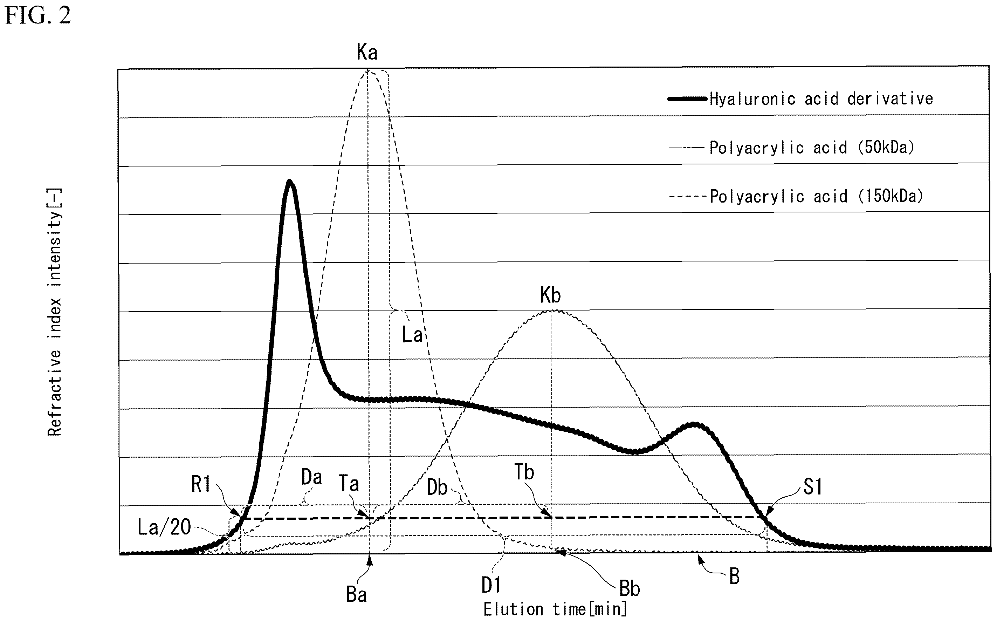

Furthermore, it is preferable in the hyaluronic acid derivative of the present embodiment that the ratio Da/Db of the distances Da and Db calculated by the following method from chromatograms shown in Fig. 2 and obtained by a gel permeation chromatography measurement be more than 0.00 and 1.20 or less.
(i) An intersection point Ba is determined by: a perpendicular line drawn from the maximum point Ka of the refractive index intensity on the chromatogram of a 150 kDa polyacrylic acid serving as a standard substance to the baseline B; and the baseline, and the length La between the maximum point Ka of the refractive index intensity and the intersection point Ba is determined;
(ii) among two points at which the refractive index intensity on the chromatogram reaches La/20, one point at which an elution time is earlier is determined as the point R1, and the other point at which the elution time is later is determined as the point S1;
(iii) an intersection point Bb is determined by: a perpendicular line drawn from the maximum point Kb of the refractive index intensity on a chromatogram of a 50 kDa polyacrylic acid serving as a standard substance to the baseline B; and the baseline;
(iv) an intersection point Ta is determined by: a straight line D1 that connects the point R1 and the point S1; and the perpendicular line drawn from the maximum point Ka of the refractive index intensity to the baseline B, and an intersection point Tb is determined by: the perpendicular line drawn from the maximum point Kb of the refractive index intensity to the baseline B; and the straight line D1; and
(v) the distance Da is determined as a distance between the point R1 and the intersection point Ta, and the distance Db is determined as a distance between the intersection point Ta and the intersection point Tb.

When there are at least three points on the chromatogram where the refractive index intensity reaches La/20 in (ii), the point R1 is determined as an intersection point nearest to the starting point, and the point S1 is determined as an intersection point nearest to the end point.

The hyaluronic acid derivative of the present embodiment differs from conventional hyaluronic acid derivatives in that hyaluronic acid derivatives having a particular particle size are contained in a relatively large amount in the particle size distribution, and the particle size distribution thereof is controlled sharply. The present inventors found that the delivery properties to immune cells in lymph nodes and the activation ability against the immune cells are significantly improved by formulating the hyaluronic acid derivative having a controlled particle size distribution with a medicinal ingredient, in comparison with hyaluronic acid derivatives having uncontrolled particle size distributions or cholesterol-bearing pullulan (CHP) conventionally used as a carrier, as shown in the below-mentioned examples, thereby completing the present invention.

The term "immune cells" mentioned here preferably refers to myeloid cells, more preferably macrophages or dendritic cells (DC), even more preferably DC, and particularly preferably conventional type 1 dendritic cells (cDC1).

Both macrophages and dendritic cells have an antigen-presenting ability. Dendritic cells are the most potent antigen-presenting cells and are responsible at regulating the proliferation and function of T cells and natural killer cells in lymphoid tissues or non-lymphoid tissues. Although there are several subtypes of dendritic cells, cDC1, which specifically expresses the chemokine receptor XCR1 and selectively expresses the C-type lectin endocytosis receptor CLEC9A, has a high cross-presentation ability, and therefore antigens are efficiently loaded on MHC class I molecules (Reference Document 1: Noubade R et al., "Beyond cDC1: Emerging Roles of DC Crosstalk in Cancer Immunity", Front Immunol., Vol. 10, Article 1014, pp. 1-13. 2019.). T cells (cytotoxic T cells (CTL)) that attack cells infected with viruses or bacteria or cancer cells can be activated by simultaneously expressing co-stimulatory molecules.

Namely, effects of preventing or treating cancers or infectious diseases are improved by enhancing the antigen-delivery properties to macrophages or DC (preferably cDC1).

In addition, macrophages and DC can load antigens on MHC class II molecules. This activates helper T cells and promotes antibody production by B cells.

Namely, an enhancement in the antigen-delivery properties to macrophages or DC (preferably cDC1) improves effects of preventing or treating infectious diseases or treating immune diseases.

The term "activation ability against the immune cells" refers to the property of improving, promoting, or enhancing the activity of the above-mentioned cells, and preferably the property of improving, promoting, or maintaining the expression of co-stimulatory molecules in DC (preferably cDC1). Examples of the co-stimulatory molecules include CD80 and CD86. T cells (cytotoxic T cells (CTL)) can be significantly induced by improving, promoting or maintaining the expression of such co-stimulatory molecules. Therefore, the hyaluronic acid derivative of the present embodiment is preferably one that can improve, promote, or maintain the expression of both CD80 and CD86 in DC (preferably cDC1) when formulated with a medicinal ingredient.

When the hyaluronic acid derivative of the present embodiment is formulated with a medicinal ingredient, the hyaluronic acid derivative can form a stable association state with the medicinal ingredient, realize stable and efficient delivery of the medicinal ingredient into immune cells (preferably DC, and more preferably cDC1), and further improve, promote, or enhance an uptake of the medicinal ingredient into the immune cells. Thus, it is assumed that the activation of immune cells is improved, promoted or enhanced. Even a case in which the desired effect is obtained by a mechanism different from the above-mentioned mechanism is also encompassed in the technical scope.

Namely, the hyaluronic acid derivative of the present embodiment may also be referred to as a delivery-improver, delivery-promoter, or delivery-enhancer of a medicinal ingredient to immune cells. Alternatively, the hyaluronic acid derivative may also be referred to as an uptake-improver, -promoter, or -enhancer of a medicinal ingredient into immune cells.

Furthermore, the below-mentioned pharmaceutical composition containing the hyaluronic acid derivative of the present embodiment and a medicinal ingredient may be referred to as a composition for activating immune cells, or improving, promoting or maintaining the expression of a co-stimulatory molecule in DC (preferably cDC1).

The particle size distribution of the hyaluronic acid derivative of the present embodiment can be measured by gel permeation chromatography, and it is shown that the particle size distribution is controlled as described above by the ratio A1/A2 of the areas A1 and A2 calculated by the following method from the gel permeation chromatograms.

In the gel permeation chromatography measurement, a polyacrylic acid used as a standard substance is preferably a sodium polyacrylate, and more preferably ORDER No. PSS-Paa series (sodium polyacrylate) manufactured by Polymer Standards Service-USA.

The measurement of the particle size distribution of the hyaluronic acid derivative by gel permeation chromatography can be carried out by the following method.

1 mg/mL of an aqueous solution of a hyaluronic acid derivative and 2 mg/mL of aqueous solutions of polyacrylic acids as standard substances are prepared, and the measurement by gel permeation chromatography is carried out under the following conditions.

### (Measurement conditions)

Device: High-speed GPC (gel permeation chromatography) device
Column: G4000SWXL (manufactured by TOSOH CORPORATION, particle size 8 µm, inner diameter 7.8 mm, length 30 cm, product number:8542)
Eluent: 10 mM phosphate buffer (pH 7.4)
Flow rate: 1 mL/min
Injection amount: 50 µL
Detector: RI (differential refractive index detector)
Temperature: 30°C

FIG. 1 is an example of a gel permeation chromatogram of the hyaluronic acid derivative of the present embodiment. The method for calculating areas A1 and A2 will be described in detail below with reference to FIG. 1.

(i) In Fig. 1, a gel permeation chromatogram of the hyaluronic acid derivative of the present embodiment and a gel permeation chromatogram of a 50 kDa polyacrylic acid as a standard substance are shown. First, an intersection point Ub is determined by: a perpendicular line drawn from the maximum point Kb of the refractive index intensity on the chromatogram of the 50 kDa polyacrylic acid as a standard substance to the baseline B; and the chromatogram of the hyaluronic acid derivative, and an intersection point Bb is determined by: a perpendicular line drawn from the maximum point Kb of the refractive index intensity to the baseline B; and the baseline B. The refractive index intensity at the start of the measurement is set to zero, and a line drawn horizontally therefrom is set as the baseline. For example, the refractive index intensity is adjusted such that the increase and decrease in the refractive index intensity falls within a range of ± 0.5 mV before the start of the measurement, and the increase and decrease in the refractive index intensity falls within 0.5 mV or less for five minutes. For example, the first point at which the increase amount of the refractive index intensity exceeds an amount equivalent to five times the noise value at the third time is set as the "starting point" on the chromatogram, and the elution time is set as zero minutes. For example, the point at which the refractive index intensity reaches 1/1,000 times the maximum refractive index intensity is set as the "end point" on the chromatogram, and when the refractive index intensity does not reach 1/1,000 times the maximum refractive index intensity, "Tlim" is set as the "end point". The term "Tlim" refers to an elution time at which the maximum refractive index intensity of a 2 kDa polyacrylic acid was measured.
   In the device, the refractive index intensity is calculated every 0.00167 minutes.
(ii) Next, the area A2 surrounded by: a curve from the intersection point Ub to the end point on the chromatogram of the hyaluronic acid derivative; a perpendicular line drawn from the maximum point Kb of the refractive index intensity to the baseline B (a straight line from the intersection point Ub to the intersection point Bb); and the baseline B is determined, and an the area A1 surrounded by: a curve from the starting point to the intersection point Ub on the chromatogram of the hyaluronic acid derivative; a perpendicular line drawn from the maximum point Kb of the refractive index intensity to the baseline B (a straight line from the intersection point Ub to the intersection point Bb); and the baseline B is determined.

Each area is calculated using an analysis application under the trade name of GPC workstation EcoSEC Elite-WS.

When each area value is calculated, peaks caused by the developing solvent used in gel permeation chromatography and pseudo-peaks due to baseline fluctuations caused by the column or device used are excluded.

The ratio A1/A2 of the areas A1 and A2 shown in Fig. 1 is 0.90 or more, preferably 1.00 or more, more preferably 1.10 or more, even more preferably 1.20 or more, even more preferably 1.30 or more, even more preferably 1.40 or more, 1.50 or more, even more preferably 1.60 or more, and particularly preferably 1.70 or more. When the ratio A1/A2 of the areas A1 and A2 is the above-mentioned lower limit or more, the hyaluronic acid derivative having a large particle size can be contained in a relatively large amount, and form a stable association state with a medicinal ingredient when formulated with the medicinal ingredient. Thus, the medicinal ingredient is allowed to be delivered stably and efficiently to immune cells (preferably DC, and more preferably cDC1), and an uptake of the medicinal ingredient into immune cells can be further improved, promoted or enhanced.

In contrast, the higher the area A1 than the area A2, the better the upper limit of the ratio A1/A2. Namely, since the particle size is increased toward the left side of the baseline (accompanying decrease in the elution time), and is decreased toward the right side thereof (accompanying increase in the elution time) in Fig. 1, the higher the ratio of large particles than the ratio of small particles, the better. Thus, the upper limit of the ratio A1/A2 is not particularly limited, but may be 7.0 or less, 6.0 or less, or 5.0 or less, for example.

Although the shape of the gel permeation chromatogram of the hyaluronic acid derivative of the present embodiment is not particularly limited, there may be at least two maximum points (peak-tops) of the refractive index intensity of the hyaluronic acid derivative. Although there are no particular limitations, there may be at least one maximum point (peak-top) of the refractive index intensity of the hyaluronic acid derivative on each of both sides (left and right) relative to the perpendicular line drawn from the maximum point Kb of the refractive index intensity on a chromatogram of a 50 kDa polyacrylic acid serving as a standard substance to the baseline B. At this time, the refractive index intensity at the maximum point (peak-top) of the refractive index intensity of the hyaluronic acid derivative on the left side relative to the perpendicular line drawn from the maximum point Kb of the refractive index intensity to the baseline B may be larger than the refractive index intensity at the maximum point (peak-top) of the refractive index intensity of the hyaluronic acid derivative on the right side relative to the perpendicular line from the maximum point Kb of the refractive index intensity to the baseline B. The refractive index intensity at the maximum point (peak-top) on the left side relative to the refractive index intensity at the maximum point (peak-top) on the right side is not particularly limited, but may be 1.0 time or more, 2.0 times or more, 3.0 times or more, 5.0 times or more, or 10.0 times or more. The upper limit thereof may be large, but may be, for example, 100 times or less, 50.0 times or less, 20.0 times or less, 10.0 times or less, 2.0 times or less, or 1.5 times or less.

Furthermore, the fact that the particle size distribution of the hyaluronic acid derivative of the present embodiment is controlled as mentioned above can be defined by the ratio Da/Db of the distances Da and Db shown below.

Fig. 2 is an example of a gel permeation chromatogram of the hyaluronic acid derivative of the present embodiment, and the gel permeation chromatogram of the hyaluronic acid derivative and the gel permeation chromatogram of a 50 kDa polyacrylic acid as a standard substance shown in Fig. 2 are the same as those shown in Fig. 1. However, Fig. 2 differs from Fig. 1 in that the gel permeation chromatogram of a 150 kDa polyacrylic acid as a standard substance is added thereto, and each intersection point and the like are defined. The method for calculating the distances Da and Db will be explained specifically with reference to Fig. 2.

(i) First, an intersection point Ba is determined by: a perpendicular line drawn from the maximum point Ka of the refractive index intensity on the chromatogram of a 150 kDa polyacrylic acid serving as a standard substance to the baseline B; and the baseline, and the length La between the maximum point Ka of the refractive index intensity and the intersection point Ba is determined.
(ii) Next, among two points at which the refractive index intensity on the chromatogram reaches La/20, one point at which the elution time is earlier is determined as the point R1, and the other point at which the elution time is later is determined as the point S1.
(iii) Next, an intersection point Bb is determined by: a perpendicular line drawn from the maximum point Kb of the refractive index intensity on a chromatogram of a 50 kDa polyacrylic acid serving as a standard substance to the baseline B and the baseline.
(iv) Next, an intersection point Ta is determined by: the straight line D1 that connects the point R1 and the point S1; and the perpendicular line drawn from the maximum point Ka of the refractive index intensity to the baseline B, and an intersection point Tb is determined by: the perpendicular line drawn from the maximum point Kb of the refractive index intensity to the baseline B; and the straight line D1.
(v) Finally, the distance Da is determined as a distance between the point R1 and the intersection point Ta, and the distance Db is determined as a distance between the intersection point Ta and the intersection point Tb.

The ratio Da/Db of the distances Da and Db shown in Fig. 2 is preferably more than 0.00 and 1.20 or less, more preferably 0.10 or more and 1.00 or less, even more preferably 0.20 or more and 0.8 or less, even more preferably 0.30 or more and 0.95 or less, even more preferably 0.40 or more and 0.94 or less, and particularly preferably 0.50 or more and 0.93 or less. When the ratio Da/Db of the distances Da and Db is within the above-mentioned range, the hyaluronic acid derivative having a particle size larger than that of a 150 kDa polyacrylic acid as a standard substance can be contained in a relatively large amount, and form a stable association state with a medicinal ingredient when formulated with the medicinal ingredient. Thus, the medicinal ingredient is allowed to be delivered stably and efficiently to immune cells (preferably DC, and more preferably cDC1), and an uptake of the medicinal ingredient into immune cells can be further improved, promoted or enhanced.

In the gel permeation chromatogram of the hyaluronic acid derivative of the present embodiment, the horizontal axis shows the elution time and the vertical axis shows the refractive index intensity obtained using a differential refractometer. The number of the maximum point of the refractive index is not limited. The gel permeation chromatogram of the hyaluronic acid derivative of the present embodiment preferably has a particle size distribution with one to five maximum points, and more preferably a particle size distribution with one to three maximum points. Furthermore, the minimum point may be present or absent.

Furthermore, the chromatogram expressed by the refractive index intensity obtained using a differential refractometer and the elution time may be asymmetrical or symmetrical in the gel permeation chromatogram of the hyaluronic acid derivative of the present embodiment.

In the hyaluronic acid derivative of the present embodiment, steryl groups in the hyaluronic acid derivative self-associate in water to allow a single molecule or plural molecules to associate, thereby forming a nano-sized hydrogel.

In the hyaluronic acid derivative, a steryl group may be bonded directly to a hyaluronic acid or may be bonded thereto via a linker.

The term "linker" used herein refers to an arbitrary peptide linker that can be introduced by genetic engineering or a synthetic compound linker, but a peptide linker is preferably used in the hyaluronic acid derivative. Although the length of the peptide linker is not particularly limited and may be appropriately determined by those skilled in the art depending on the purpose, the length is preferably two amino acids or more (the upper limit is not particularly limited, but is usually 30 amino acids or less, preferably 20 amino acids or less), and particularly preferably 15 amino acids. The length of the peptide linkers contained in the hyaluronic acid derivative may be identical to or different from each other.

### [Steryl group]

The term "steryl group" used in the present specification is not particularly limited as long as it is a group having a steroid skeleton. Specific examples of the steroid include cholesterol, cholestanol, campestanol, ergostanol, stigmastanol, coprostanol, stigmasterol, sitosterol, lanosterol, ergosterol, simiarenol, bile acid, testosterone, estradiol, progesterone, cortisol, cortisone, aldosterone, corticosterone, and deoxycortisterone. Examples of the steryl group include a cholesteryl group, a stigmasteryl group, a lanosteryl group, and an ergosteryl group. Among them, a cholesteryl group (particularly a cholest-5-en-3β-yl group) is preferable.

Although the weight-average molecular weight (absolute molecular weight) of the hyaluronic acid derivative is not particularly limited, the hyaluronic acid derivative preferably has a relatively large molecular weight from the viewpoint of increasing the number of steryl groups introduced per molecule of the hyaluronic acid derivative to form a complex with a medicinal ingredient, and increasing molecular entanglement to enhance the retention in blood. The weight-average molecular weight (absolute molecular weight) of such a hyaluronic acid derivative is preferably4,000 (4k) or more and 1,000,000 (1,000k) or less, more preferably 5k or more and 500k or less, even more preferably 6k or more and 500k or less, even more preferably 7k or more and 300k or less, even more preferably 7k or more and 100k or less, even more preferably 7k or more and 50k or less, even more preferably 7k or more and 25k or less, and particularly preferably 8k or more and 15k or less. Alternatively, the weight-average molecular weight (absolute molecular weight) of the hyaluronic acid derivative is more preferably 5k or more and 25k or less. When the weight-average molecular weight (absolute molecular weight) of the hyaluronic acid derivative is the above-mentioned lower limit or more, the molecular entanglement can be increased, thereby enhancing the retention in blood. In contrast, when the weight-average molecular weight (absolute molecular weight) of the hyaluronic acid derivative is the above-mentioned upper limit or less, the viscosity increase can be suppressed, thereby making it possible to dissolve a higher concentration of the hyaluronic acid derivative in a pharmaceutical composition. The weight-average molecular weight (absolute molecular weight) of the hyaluronic acid derivative can be generally adjusted using raw materials having the corresponding molecular weight.

The term "molecular weight (absolute molecular weight) of the hyaluronic acid derivative" used here refers to the weight-average molecular weight (absolute molecular weight) determined using size exclusion chromatography with a multi-angle static light scattering (SEC-MALS) detector.

Although the ratio Pt/Pr of retention time Pt at the maximum point (peak-top) of the refractive index intensity of the hyaluronic acid derivative relative to the retention time Pr (elution time Bb) at the maximum point (peak-top) of the refractive index intensity of a 50 kDa polyacrylic acid as a standard substance in the chromatograms obtained by gel permeation chromatography measurements is not particularly limited, the ratio Pt/Pr is preferably 0.5 or more and less than 1.0, more preferably 0.7 or more and less than 0.95, and even more preferably 0.75 or more and less than 0.92. When the ratio Pt/Pr is within the above-mentioned range, the hyaluronic acid derivative having a large particle size can be contained in a relatively large amount, and form a stable association state with a medicinal ingredient when formulated with the medicinal ingredient. Thus, the medicinal ingredient is allowed to be delivered stably and efficiently to immune cells (preferably DC, and more preferably cDC1), and an uptake of the medicinal ingredient into immune cells can be further improved, promoted or enhanced.

When there are plural maximum points (peak-tops) of the refractive index intensity of the hyaluronic acid derivative in the chromatogram obtained by the gel permeation chromatography measurement, the elution time at which the maximum point (peak-top) of the refractive index intensity is the largest refractive index intensity is adopted as the retention time Pt at the maximum point (peak-top) of the refractive index intensity of the hyaluronic acid derivative.

In the gel permeation chromatography measurement, a polyacrylic acid used as a standard substance is preferably a sodium polyacrylate, and more preferably ORDER No. PSS-Paa series (sodium polyacrylate) manufactured by Polymer Standards Service-USA.

Although the weight-average molecular weight (based on polyacrylic acid) of the hyaluronic acid derivative is not particularly limited, the weight-average molecular weight (based on polyacrylic acid) of the hyaluronic acid derivative is preferably 110,000 or more, more preferably 110,000 or more and less than 500,000, even more preferably 130,000 or more and less than 300,000, and most preferably 140,000 or more and less than 250,000. When the weight-average molecular weight (based on polyacrylic acid) of the hyaluronic acid derivative is within the above-mentioned range, the hyaluronic acid derivative having a large particle size can be contained in a relatively large amount, and form a stable association state with a medicinal ingredient when formulated with the medicinal ingredient. Thus, the medicinal ingredient is allowed to be delivered stably and efficiently to immune cells (preferably DC, and more preferably cDC1), and an uptake of the medicinal ingredient into immune cells can be further improved, promoted or enhanced.

In contrast, when the weight-average molecular weight (based on polyacrylic acid) of the hyaluronic acid derivative exceeds the above-mentioned upper limit, the viscosity increases, and there is a case in which the hyaluronic acid derivative is difficult to be used as a preparation.

The weight-average molecular weight (based on polyacrylic acid) of the hyaluronic acid derivative is calculated from the chromatogram obtained by a gel permeation chromatography measurement in accordance with the following formula: At3+Bt2+Ct+D, based on a calibration curve obtained using polyacrylic acids having a molecular weight of 2 kDa, 4 kDa, 8 kDa, 18 kDa, 40 kDa, or 150 kDa (PSS-Paa 2k (2 kDa), 4k (4 kDa), 8k (8 kDa), 18k (18 kDa), 40k (40 kDa), or 150k (150 kDa) (manufactured by PSS Polymer Standard service GmbH, sodium polyacrylates) as standard substances.

Preferable examples of the hyaluronic acid derivative include hyaluronic acid derivatives having at least one repeating unit of the following general formula (I) (hereinafter, may be referred to as "repeating unit (I)").

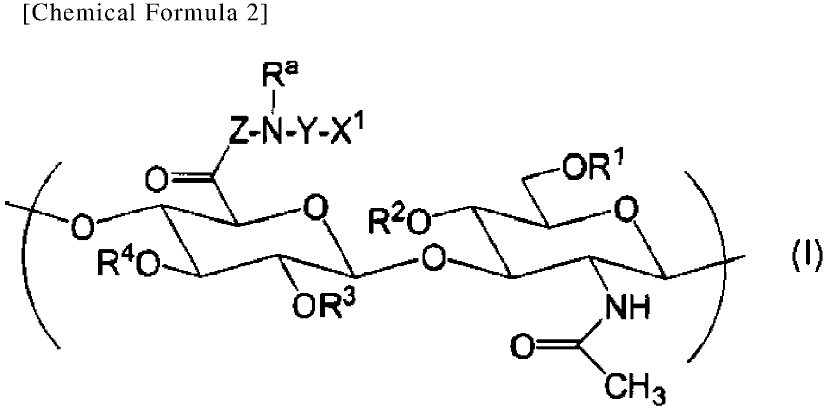

(In the formula, each of R¹, R², R³ and R⁴ is independently selected from the group consisting of a hydrogen atom, C₁₋₆ alkyls, a formyl and C₁₋₆ alkylcarbonyls;
Z is a direct bond or a peptide linker consisting of 2 to 30 arbitrary amino acid residues;
X¹ is a group selected from the group consisting of:
   -NR^{b}-R,
   -NR^{b}-COO-R,
   -NR^{b}-CO-R,
   -NR^{b}-CO-NR^{c}-R,
   -COO-R,
   -O-COO-R,
   -S-R,
   -CO-Y^{a}-S-R,
   -O-CO-Y^{b}-S-R,
   -NR^{b}-CO-Y^{b}-S-R, and
   -S-S-R;
   Each of R^{a}, R^{b} and R^{c} is independently selected from the group consisting of a hydrogen atom, C₁₋₂₀ alkyls, amino C₂₋₂₀ alkyls and hydroxy C₂₋₂₀ alkyls, in an alkyl moiety of which a group selected from the group consisting of -O- and -NR^{f}- may be inserted;
   R^{f} is selected from the group consisting of a hydrogen atom, C₁₋₁₂ alkyls, amino C₂₋₁₂ alkyls and hydroxy C₂₋₁₂ alkyls, in an alkyl moiety of which a group selected from the group consisting of -O- and -NH- may be inserted;
   R is a steryl group;
   Y is C₂₋₃₀ alkylene or (CH₂CH ₂O)ₘ-CH₂CH₂-, and a group selected from the group consisting of -O-, -NR^{g}- and -S-S- may be inserted in the alkylene;
   R^{g} is selected from the group consisting of a hydrogen atom, C₁₋₂₀ alkyls, amino C₂₋₂₀ alkyls and hydroxy C₂₋₂₀ alkyls, in an alkyl moiety of which a group selected from the group consisting of -O- and -NH- may be inserted;
   Y^{a} is a C₁₋₅ alkylene;
   Y^{b} is a C₂₋₈ alkylene or a C₂₋₈ alkenylene; and
   m is an integer of 1 or more and 100 or less.)

The hyaluronic acid derivative preferably contains at least one repeating unit of the following general formula (la) (hereinafter, may be referred to as "repeating unit (Ia)") as the repeating unit (I).

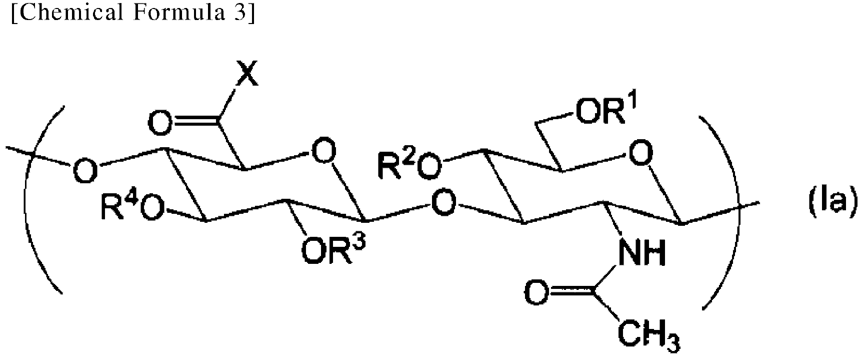

(In the formula, each of R¹, R², R³ and R⁴ is independently selected from the group consisting of a hydrogen atom, C₁₋₆ alkyls, a formyl and C₁₋₆ alkylcarbonyls;
X is a hydrophobic group of -NR^{a}-Y-NR^{b}-COO-R;
each of R^{a} and R^{b} is independently selected from the group consisting of a hydrogen atom and C₁₋₆ alkyls;
R is a steryl group;
Y is a C₂₋₃₀ alkylene, or (CH₂CH ₂O)ₘ-CH₂CH₂-; and
m is an integer of 1 or more and 100 or less.)

When at least two repeating units (I) or repeating units (Ia) are contained in the hyaluronic acid derivative, the repeating units may be identical to or different from each other.

The hyaluronic acid derivative may be modified at a position other than the repeating unit (I) or the repeating unit (Ia), and, for example, a hydroxy group may be converted to -O(C₁₋₆ alkyl), -O(formyl), -O(C₁₋₆ alkylcarbonyl), or the like, and a carboxy group may be converted to an amide or ester, or may form a salt.

### [Repeating unit (I)]

The group "-Z-N(R^{a})Y-X¹" in the general formula (I) is preferably selected from groups of the following formulae:
-NH-(CH₂)_{mz}-NH-R;
-NH-(CH₂)_{mz}-NH-COO-R;
-NH-(CH₂CH₂O)ₘ-CH₂CH₂-NH-COO-R;
-NH-(CH₂)_{mz}-COO-R;
-NH-(CH₂CH₂O)ₘ-CH₂CH₂-COO-R,
-NH-(CH₂)_{mz}-O-COO-R;
-NH-(CH₂CH₂O)ₘ-CH₂CH₂-O-COO-R,
-NH-(CH₂)_{mz}-S-R;
-NH-(CH₂CH₂O)ₘ-CH₂CH₂-S-R;
-NH-(CH₂)_{mz}-O-CO-CH(R⁸)-CH₂-S-R;
-NH-(CH₂)_{mz}-NHCO-CH(R⁸)-CH₂-S-R;
-NH-(CH₂CH₂O)ₘ-CH₂CH₂-NHCO-CH(R⁸)-CH₂-S-R;
-NH-(CH₂CH₂0o)ₘ-CH₂CH₂-O-CO-CH(R⁸)-CH²-S-R;
-NH-(CH²)_{mz}-S-S-R; and
-Z-NR ^{a} -Y-NR^{b}-COO-R,
(in which mz is an integer of 2 to 30, R⁸ is a hydrogen atom or a methyl group, and R and m are as have been already defined in the present specification), and is more preferably selected from groups of the following formulae:
   -NH-(CH₂)_{mz}-NH-COO-R;
   -NH-(CH₂CH₂O)ₘ-CH₂CH₂-NH-COO-R; and
   -NH-(CH₂)_{mz}-S-S-R
   (in which mz, R, and m are as have been already defined in the present specification).

### (Z)

In the general formula (I), Z is preferably a direct bond.

In another aspect, when Z is a peptide linker, X¹ is preferably -NR^{b}-COO-R.

In another aspect, Z may be a peptide linker of -NH-[CH(-Z^{a})-CONH]ₙ₋₁-CH(-Z^{a})-CO-, wherein n is an integer of 2 to 30, Z^{a} is each independently a substituent in an α-amino acid of H₂N-CH(-Z^{a})-COOH. The peptide linker is bonded to a carboxy group of a glucuronic acid moiety at the N-terminus and to a group of -N(-R^{a})-Y-X¹ at the C-terminus. Examples of amino acids available as amino acid residues in the peptide linker include: α-amino acids such as natural (L-type) amino acids such as alanine, arginine, asparagine (Asn), aspartic acid, cysteine, glutamine, glutamic acid, glycine (Gly), histidine, isoleucine, leucine (Leu), lysine, methionine, phenylalanine (Phe), proline, serine, threonine, tryptophan, tyrosine, and valine; and D forms thereof, and all α-amino acids including synthetic amino acids may be used. Namely, examples of Z^{a} include - CH₃, H₂NC(NH)NH(CH₂)₃-, and H₂NCOCH₂-. In addition, n of Z may be identical to or different from each other. Although n is an integer of 2 to 30, n is preferably 2 to 10, and more preferably 2 to 4. Preferable examples of the peptide linker include -Gly-Phe-Leu-Gly-, -Asn-Phe-Phe-, -Phe-Phe-, and -Phe-Gly-.

### (Y)

In the general formula (I), Y is preferably a group selected from the group consisting of -(CH₂)ₙ₁- and (CH₂CH₂O)ₘ₁-CH₂CH₂- (in which n1 is an integer of 2 to 20, preferably an integer of 2 to 15, more preferably an integer of 2 to 12, and even more preferably an integer of 2 to 6, and m1 is an integer of 1 to 4). Specifically, Y is preferably -(CH₂)₂-, -(CH₂)₆-, -(CH₂)₈-, -(CH₂)₁₂-, or -(CH₂CH₂O)₂-CH₂CH₂-. In addition, Y is preferably a group selected from the group consisting of -(CH₂)₂-, -(CH₂)₆-, -(CH₂)₈- and -(CH₂)₁₂-, and more preferably -(CH₂)₆-, from the viewpoint of achieving high solubility in pure water or under a low salt concentration while exhibiting a high precipitate forming ability under a physiological saline concentration.

Y may be, for example, -CH₂CH₂O-CH₂CH₂-S-S-CH₂CH₂O-CH₂CH₂-, - (CH₂CH₂O)₂-CH₂CH₂-S-S-CH₂CH₂O-CH₂CH₂-, -CH₂CH₂O-CH₂CH₂-S-S-(CH₂CH₂O)₂-CH₂CH₂-, -(CH₂CH₂O)₂-CH₂CH₂-S-S-(CH₂CH₂O)₂-CH₂CH₂- or the like.

### (Y^{a})

Y^{a} is preferably -CH₂- or -CH₂-CH₂-.

### (Y^{b})

Y^{b} is preferably -CH₂-CH₂-, -CH(CH₃)CH₂-, 2-butene-1,4-diyl, hepta-2,4-diene-1,6-diyl or octa-2,4,6-triene-1,8-diyl, and more preferably -CH₂-CH₂- or -CH(CH₃)CH₂-.

Specific examples of the group of "-Z-N(R^{a})Y-X¹" include -NH-(CH₂)₂-NH-CO-cholesteryl, -NH-(CH₂)₄-NH-(CH₂)₃-NH-(CH₂)₃-NH-COO-cholesteryl, -NH-(CH₂)₃-NH-(CH₂)₄-NH-(CH₂)₃-NH-COO-cholesteryl, -NH-(CH₂)₄-NH-(CH₂)₃-NH-COO-cholesteryl, -NH-(CH₂)₄-N(-(CH₂)₃-NH₂)-COO-cholesteryl, -NH-(CH₂)₃-NH-(CH₂)₄-N(-(CH₂)₃-NH₂)-COO-cholesteryl, -NH-(CH₂)₃-NH-(CH₂)₄-N(-(CH₂)₃-NH-(CH₂)₃-NH₂)-COO-cholesteryl, -NH-(CH₂)₃-NH-(CH₂)₄-N(-(CH₂)₃-NH₂)-CO-NH-cholesteryl, -NH-(CH₂)₃-NH-(CH₂)₄-N(-(CH₂)₃-NH₂)-CO-cholesteryl, and -NH-(CH₂)₃-NH-(CH₂)₄-N(-(CH₂)₃-NH₂)-cholesteryl. In the preferable group of "-Z-N(R^{a})Y-X¹", R^{a}, R^{b} and R^{c} are hydrogen atoms, Y is a linear C₂₋₃₀ alkylene or (CH₂CH₂O)ₘ-CH₂CH₂-, Y^{a} is a linear C₁₋₅ alkylene, or Y^{b} is a linear C₂₋₈ alkylene or linear C₂₋₈ alkenylene.

### [Repeating unit (la)]

In the general formula (Ia), X is preferably -NH-(CH₂)₂-NH-COO-cholesteryl, - NH-(CH₂)₆-NH-COO-cholesteryl, -NH-(CH₂)₁₂-NH-COO-cholesteryl or -NH-(CH₂ CH₂O)₂- CH₂CH₂-NH-COO-cholesteryl, more preferably -NH-(CH₂)₂-NH-COO-cholesteryl, -NH-(CH₂)₆-NH-COO-cholesteryl or -NH-(CH₂CH₂O)₂-CH₂CH₂-NH-COO-cholesteryl, and even more preferably -NH-(CH₂)₆-NH-COO-cholesteryl.

The hyaluronic acid derivative may further contain a repeating unit of the general formula (II) (hereinafter, may be referred to as "repeating unit (II)") in addition to the repeating unit (I).

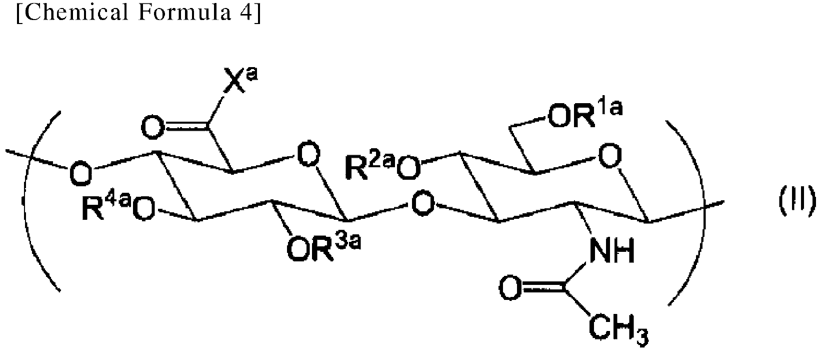

(In the formula, each of R^{1a}, R^{2a}, R^{3a} and R^{4a} is independently selected from the group consisting of a hydrogen atom, C₁₋₆ alkyls, a formyl and C₁₋₆ alkylcarbonyls; and
X^{a} is selected from the group consisting of a hydroxy and -O-Q⁺; wherein Q⁺ is a counter-cation.)

When at least two repeating units (ii) are contained in the hyaluronic acid derivative, the repeating units may be identical to or different from each other.

In another aspect, the hyaluronic acid derivative may be a hyaluronic acid derivative consisting essentially of the repeating unit (I), the repeating unit (Ia) and the repeating unit (II).

### [Repeating unit (II)]

In the general formula (II), Q⁺ is not particularly limited as long as Q⁺ is a counter-cation that forms a salt with a carboxy group in water. When Q⁺ has a valence of two or more, Q⁺ forms a salt with plural carboxy groups depending on the valence.

Examples of the counter-cation include: metal ions such as lithium ion, sodium ion, rubidium ion, cesium ion, magnesium ion, and calcium ion; and ammonium ions of formula: N⁺R^{j}R^{k}R^{l}R^{m} (in the formula, each of R^{j}, R^{k}, R^{l} and R^{m} is independently selected from the group consisting of a hydrogen atom and C₁₋₆ alkyls).

Among them, Q⁺ is preferably a sodium ion, potassium ion, or tetraalkylammonium ion (such as a tetra n-butylammonium ion).

In the formula, Rⁱ, R^{k}, R^{l} and R^{m} are preferably the same groups selected from the group consisting of C₁₋₆ alkyls, and more preferably n-butyl groups.

All of R¹, R², R³, R⁴, R^{1a}, R^{2a}, R^{3a} and R^{4a} are preferably hydrogen atoms. It is preferable that both of R^{a} and R^{b} be hydrogen atoms.

Among them, the hyaluronic acid derivative is preferably a hyaluronic acid derivative consisting essentially of the repeating unit (I) and the repeating unit (II). In the hyaluronic acid derivative, for example, 80% or more, preferably 90% or more, and more preferably 95% or more of the disaccharide repeating units formed from D-glucuronic acid and N-acetyl-D-glucosamine contained in the derivative are composed of the repeating unit (I) and the repeating unit (II). The hyaluronic acid derivative may consist of the repeating unit (I) and the repeating unit (II), or may consist of the repeating unit (I).

In the hyaluronic acid derivative of the present embodiment, the introduction ratio of the steryl group relative to the disaccharide repeating unit that constitutes the hyaluronic acid derivative (hereinafter, may be referred to as "introduction ratio of the steryl group") is preferably 15% or more and 60% or less, more preferably 20% or more and 60% or less, even more preferably 30% or more and 60% or less, even more preferably 30% or more and 55% or less, even more preferably 35% or more and 50% or less, and particularly preferably 35% or more and 45% or less.

When the introduction ratio of the steryl group is the above-mentioned lower limit or more, a stable hydrogel can be maintained without precipitating in vivo. In contrast, when the introduction ratio of the steryl group is the above-mentioned upper limit or less, the average particle size of the hydrogel can fall within the above-mentioned range.

The introduction ratio of the steryl group can be measured by a ¹H-NMR measurement. Namely, the integral value of the peak derived from steryl groups of the hyaluronic acid derivative in the ¹H-NMR spectrum of the hyaluronic acid derivative component, and the integral value of the peak derived from acetyl groups of N-acetyl-D-glucosamine contained in the hyaluronic acid derivative (COCH₃, 1.6 ppm or more and 2.0 ppm or less, 3H) are used to calculate the introduction ratio of the steryl group based on the following formula. In the formula, n_{H} indicates the number of hydrogen atoms corresponding to the peak.

The ¹H-NMR measurement may be carried out, for example, using a 0.02N DCl DMSO-d₆/D₂O mixture liquid (2N DCl D₂O: DMSO-d₆=1:99) as a measurement solvent at a measurement temperature of 85°C.

Since the peak (5H) derived from cholesteryl groups overlaps the peak around 1.6 to 2.0 ppm, which includes the peak derived from acetyl groups of glucosamine, a value calculated by subtracting a 5/3 time integral value of the peak (0.7 ppm) derived from the cholesteryl group methyl from the integral value of the peak around 1.6 to 2.0 ppm (that is, integral value (1.6 to 2.0 ppm) - integral value (0.7 ppm)×5/3) can be used as an integral value of acetyl groups derived from a hyaluronic acid to calculate the introduction ratio. [introduction ratio of the steryl group](%) = [(the integral value of the peak derived from steryl groups×3/nH)/(the integral value of the peak derived from acetyl groups of N-acetyl-D-glucosamine)] ×100

### <Method for producing hyaluronic acid derivative>

Hyaluronic acid derivatives can be obtained, for example, by converting a carboxyl group of a glucuronic acid into an amide to introduce a steryl group. Furthermore, the introduction ratio of the steryl group can be controlled by adjusting the addition amount of a compound having a steryl group to be reacted with a hyaluronic acid as a raw material or a derivative thereof.

Specifical examples of the method of converting a carboxyl group of a glucuronic acid into an amide to introduce a steryl group include a method in which a hyaluronic acid as a raw material or a derivative thereof, preferably a hyaluronic acid consisting of the repeating unit (II) or a derivative thereof is ion-exchanged to a tetraalkylammonium salt (such as a tetrabutylammonium (TBA) salt), followed by reacting the resultant hyaluronic acid salt in a solvent in the presence of a suitable condensing agent with an amine in which a steryl group (particularly, a cholesteryl group) is introduced, the amine being of the formula: "HNR^{a}-Y-NR^{b}-R, NHR^{a}-Y-NR^{b}-COO-R, HNR^{a}-Y-NR^{b}-COO-R, HNR^{a}-Y-NR^{b}-CO-R, HNR^{a}-Y-NR^{b}-CO-NR^{c}-R, HNR^{a}-Y-COO-R, HNR^{a}-Y-O-COO-R, HNR^{a}-Y-S-R, HNR^{a}-Y-CO-Y^{a}-S-R, HNR^{a}-Y-O-CO-Y^{b}-S-R, HNR^{a}-Y-NR^{b}-CO-Y^{b}-S-R, HNR^{a}-Y-S-S-R, or Z-NR^{a}-Y-NR^{b}-COO-R (in the formulae, R^{a}, R^{b}, R^{c}, Y, Y^{a}, y^{b}, Z and R are as have been already defined in the present specification)".

The condensing agent available in the above-mentioned reaction is not particularly limited, and examples thereof include 4-(4,6-dimethoxy-1,3,5-triazine)-4-methylmorpholium (DMT-MM), N,N'-carbonyldiimidazole (CDI), N,N'-dicyclohexylcarbodiimide (DCC), N-ethoxycarbonyl-2-ethoxy-1,2-dihydroquinoline (EEDQ), 2-benzotriazole-1,1,3,3-tetramethyluronium tetrafluoroborate (TBTU), 3,4-dihydro-3-hydroxy-4-oxo-1,2,3-benzotriazine (HODhbt), benzotriazole-1-oxy-tris-pyrrolidino-phosphonium hexafluorophosphate (PyBOP), benzotriazol-1-yl-oxy-tris(dimethylamino)phosphonium hexafluorophosphate (BOP), 1-ethyl-3-(3-dimethylaminopropyl) carbodiimide (EDC), and N-hydroxysuccinimide (NHS).

Although there are no particular limitations, DMT-MM is preferable in terms that the reaction proceeds with high efficiency even in a mixed solvent of water and an organic solvent. Furthermore, the use of DMT-MM as a condensing agent makes it possible to highly selectively form an amide bond between an amino group and a carboxy group while suppressing the formation of an ester bond in a system where a large number of hydroxy groups coexist. The use of the condensing agent makes it possible to prevent an alcohol as a solvent from reacting with a carboxy group of a hyaluronic acid moiety, or prevent a carboxyl group and a hydroxy group, which are simultaneously present in a hyaluronic acid moiety, from forming undesired crosslinks due to bonding thereof in a molecule or between molecules, for example.

Examples of a solvent available in the steryl group-introduction reaction include water, DMSO, methanol, ethanol, propanol, butanol, isopropanol, polyhydric alcohol, acetonitrile, DMF, THF, dichloromethane, chloroform, hexane, diethyl ether, ethyl acetate, and mixtures thereof.

The polyhydric alcohol may be a dihydric alcohol or a trihydric alcohol.

Examples of the dihydric alcohol include ethylene glycol, diethylene glycol, propylene glycol, dipropylene glycol, neopentyl glycol, 1,4-butanediol, and 1,6-hexanediol.

Examples of the trihydric alcohol include glycerin and trimethylolpropane.

Alternatively, a hyaluronic acid as a raw material or a derivative thereof may be ion-exchanged to a tetraalkylammonium salt (such as tetrabutylammonium (TBA) salt), followed by reacting the resultant hyaluronic acid salt and a spacer moiety in a solvent in the presence of an appropriate condensing agent (at this time, protection and deprotection reactions may be carried out, as needed) to convert a carboxy group (-COOH) of the hyaluronic acid as a raw material or the derivative thereof, and then reacting the resultant with an appropriate reagent. Examples of the combination of a group derived from a carboxy group and a reaction reagent are shown below.
-CONR^{a}-Y-NR^{b}H + Hal-R;
-CONR^{a}-Y-NR^{b}H + Hal-COOR;
-CONR^{a}-Y-NR^{b}H + HOCO-R;
-CONR^{a}-Y-NR^{b}H + Hal-CO-R;
-CONR^{a}-Y-NR^{b}-COOH + HNR^{c}-R;
-CONR^{a}-Y-NR^{b}-CO-NR^{c}H + Hal-R;
-CONR^{a}-Y-NR^{h}H + HOCO-NR^{c}-R;
-CONR^{a}-Y-NR^{b}H + Hal-CO-NR^{c}-R;
-CONR^{a}-Y-COOH + HO-R;
-CONR^{a}-Y-OH + Hal-COO-R;
-CONR^{a}-Y- O COOH + HO-R;
-CONR^{a}-Y- O COOH + Hal-R;
-CONR^{a}-Y- O CO-Hal + HO-R;
-CONR^{a}-Y-SH + Hal-R;
-CONR^{a}-Y-Hal + HS-R;
-CONR^{a}-Y-CO-Y^{a}-Hal + HS-R;
-CONR^{a}-Y-CO-Y^{a}-SH + Hal-R;
-CONR^{a}-Y-O-CO-CH=CH₂ + HS-R;
-CONR^{a}- Y-NR^{b}-CO-CH(CH₃)=CH₂ + HS-R;
-CONR^{a}-Y-SH + HS-R;
-COZ-OH + HNR^{a}-Y-NR^{b}-COO-R; and
-COZ-NR^{a}-Y-NR^{b}H + Hal-COO-R,
(in the formulae, R^{a}, R^{b}, R^{c}, Y, Y^{a}, Y^{b}, and Z are as have been already defined in the present specification, and Hal is a halogen atom selected from the group consisting of a fluorine atom, a chlorine atom, a bromine atom, and an iodine atom).

Examples of the reaction mode include dehydrohalogenation reaction, condensation reaction, dehydration reaction, nucleophilic addition reaction such as Michael addition, and oxidative disulfide formation reaction. These reactions are well-known reactions and may be selected as appropriate by those skilled in the art, and preferable reaction conditions thereof may be found to carry out the reaction. When a converter or reactant has a carboxyl group, it may be made into an N-hydroxysuccinimide (hereinafter also referred to as "NHS") ester to be reacted.

Additional examples thereof include a method in which 2-aminoethyl 2-pyridyldisulfide is reacted with a carboxy group of a hyaluronic acid as a raw material or a derivative thereof to prepare a hyaluronic acid derivative having an introduced spacer having a mercapto group modified with a leaving group at the end thereof, followed by subjecting the hyaluronic acid derivative to a nucleophilic substitution reaction with thiocholesterol to form a disulfide bond.

Additional examples thereof include a method in which a part of a spacer is introduced into a carboxy group of a hyaluronic acid or a derivative thereof and a part of a spacer is introduced into a steryl group, followed by reacting them. Although some of the specific examples are mentioned above, in the case where -S-S- is inserted in Y, a hyaluronic acid derivative in which a spacer having a mercapto group at the end thereof is introduced into a carboxy group of a hyaluronic acid is prepared, and a steryl group into which a spacer having a mercapto group at the end thereof is introduced is prepared, followed by reacting them oxidatively to form a disulfide bond. At this time, one mercapto group may be reacted with 2-mercaptopyridine to form a disulfide, followed by substituting with the other mercapto group.

After the hyaluronic acid derivative is prepared, another substituent may be further introduced. For example, 0.1% by mol or more and 99.5% by mol or less, preferably 40% by mol or more and 65% by mol or less of carboxy groups in a hyaluronic acid derivative consisting essentially of the repeating units (I) and the repeating units (II) are converted into -CO-X^{Z}, [where X^{Z} is selected from the group consisting of the following groups:
-NH-(CH₂)ₚ₁-O-CO-C(R¹⁷)=CH₂;
-NH-(CH₂)ₚ₁-O-CO-CH(R¹⁷)-CH₂- S -CH₂-CH(OH)-CH(OH)-CH₂-SH;
-NH-(CH₂)ₚ₁-SH;
-NH-(CH₂)ₚ₁-NH-CO-C(R¹⁷)=CH₂;
-NH-(CH₂)ₚ₁-NH-C(=NH)-(CH₂)₃-SH;
-NH-(CH₂)ₚ₁-NH-CO-(CH₂)ᵣ-SH;
-NH-(CH₂)ₚ₁-NH-CO-CH(R¹⁷)-CH₂- S -CH₂-CH(OH)-CH(OH)-CH₂-SH;
-NH-(CH₂)ₚ₁-NH-CO-CH(NH₂)-CH₂-SH;
-NH-(CH₂)ₚ₁-NH-CO-CH(NH₂)-(CH₂)₂-SH;
-NH-NH-CO-(CH₂)₄-CO-NH-NH-C(=NH)-(CH₂)₃-SH;
-NH-(CH₂-CH₂-O)_{q}-CH₂-CH₂-O-CO-C(R¹⁷)=CH₂;
-NH-(CH₂-CH₂-O)_{q}-CH₂-CH₂-O-CO-CH(R¹⁷)-CH₂-S-CH₂-CH(OH)-CH(OH)-CH₂-SH;
-NH-(CH₂-CH₂-O)_{q}-CH₂-CH₂-SH;
-NH-(CH₂-CH₂-O)_{q}-CH₂-CH₂-NH-CO-C(R¹⁷)=CH₂;
-NH-(CH₂-CH₂-O)_{q}-CH₂-CH₂-NH-C(=NH)-(CH₂)₃-SH;
-NH-(CH₂-CH₂-O)_{q}-CH₂-CH₂-NH-CO-(CH₂)ᵣ-SH;
-NH-(CH₂-CH₂-O)_{q}-CH₂-CH₂-NH-CO-CH(R¹⁷)-CH₂-S-CH₂-CH(OH)-CH(OH)-CH₂-SH;
-NH-(CH₂-CH₂-O)_{q}-CH₂-CH₂-NH-CO-CH(NH₂)-CH₂-SH;
-NH-(CH₂-CH₂-O)_{q}-CH₂-CH₂-NH-CO-CH(NH₂)-(CH₂)₂-SH;
-NH-CH(CO₂H)-(CH₂)-SH;
-NH-CH(CO₂H)-(CH₂)₂-SH; and
-NH-CH(CO₂H)-(CH₂)₂-CONH-CH(CONH-CH₂-CO₂H)-CH₂-SH,
(in the formulae, R¹⁷ is a hydrogen atom or a C₁₋₆ alkyl group, p1 is an integer of 2 to 10, q is an integer of 1 to 100, and r is an integer of 1 to 3), to cause crosslink formation chemically in a molecule or between molecules including another molecule to be gelated.

In the case where the particle size is controlled within a specific range by dialysis, a hyaluronic acid derivative having a particle size distribution satisfying the area ratio A1/A2 of 0.9 or more can be obtained by carrying out dialysis using a dialysis membrane with a MWCO (molecular weight cutoff) of 300 kDa one to nine times, preferably three to nine times, and more preferably six to nine times. The arbitrary number of times of dialysis may be appropriately determined by those skilled in the art, as long as the area ratio A1/A2 is 0.9 or more. In some cases, fractionation may be carried out by dialysis nine or more times.

At this time, the method for obtaining a hyaluronic acid derivative having a particle size distribution satisfying the area ratio A1/A2 of 0.9 or more is not limited to dialysis only. For example, a hyaluronic acid derivative having a particle size distribution satisfying the area ratio A1/A2 of 0.9 or more may be obtained by removing hyaluronic acid derivatives having a particle size distribution equal to or lower than that of a polyacrylic acid (50 kDa, ORDER No. PSS-Paa 50k manufactured by Polymer Standards Service-USA) as a standard substance by carrying out a centrifugal filtration using an ultrafiltration membrane or microfiltration membrane, preparative purification HPLC, preparative purification GPC, separation by ultracentrifugation, separation by ion exchange resin, separation by membrane distillation, membrane separation by organic or inorganic membrane, separation by an adsorption/desorption method using activated carbon, zeolite, or MOF (Metal Organic Frameworks), separation by TFF (Tangential Flow Filtration), liquid feeding using a filter, pressure or vacuum filtration separation, precipitation separation using salt deposition, or the like, separation using a hyaluronic acid receptor, or the like.

The obtained hyaluronic acid derivative may be dried. Examples of the drying method include ventilation drying, drying in a thermostatic chamber, reduced pressure drying, hot air circulation drying, and freeze drying. Among them, freeze drying is preferable. When freeze drying is carried out, the hyaluronic acid derivative preferably further contains a cryoprotectant from the viewpoint of more effectively suppressing an increase in the particle size of fine particles formed by the hyaluronic acid derivative.

The cryoprotectant is not particularly limited as long as it is known as a "cryoprotectant" or "lyoprotectant", and examples thereof include disaccharides, sorbitol, dextran, polyethylene glycol, propylene glycol, glycerin, glycerol, polyvinylpyrrolidone and dimethyl sulfoxide.

Disaccharides are not particularly limited and examples thereof include sucrose, lactulose, lactose, maltose, trehalose, cellobiose, kojibiose, nigerose, isomaltose, isotrehalose, neotrehalose, sophorose, laminaribiose, gentiobiose, turanose, maltulose, palatinose, gentiobiulose, mannobiose, melibiose, melibiulose, neolactose, galactosucrose, silabiose, neohesperidose, rutinose, rutinulose, vicianose, xylobiose, and primeverose. Among them, sucrose, trehalose, maltose, or lactose is preferable, because they are widely used as cryoprotectants. Furthermore, sucrose is more preferable from the viewpoint of the use record thereof as a pharmaceutical additive and from the viewpoint of more effectively suppressing an increase in particle size of fine particles formed by the hyaluronic acid derivative during freeze drying.

The cryoprotectant may be added in a solid state or in a state in which the cryoprotectant is dissolved in a solvent such as water.

Although the addition amount of the cryoprotectant is not particularly limited, the addition amount thereof relative to 100 parts by mass of the hyaluronic acid derivative is preferably 20 parts by mass or more. When the addition amount of the cryoprotectant is the above-mentioned lower limit or more, a more sufficient effect of suppressing an increase in the particle size can be exhibited. On the other hand, the upper limit of the addition amount of the cryoprotectant is not particularly limited, but may be, for example, 100,000 parts by mass.

A device used in freeze drying is not particularly limited, and for example, a commercially available freeze-dryer may be used. Among them, a freeze dryer that can monitor the degree of vacuum inside the device during freeze-drying is preferable from the viewpoint of controlling the degree of vacuum, and a shelf-type freeze dryer is preferable from the viewpoint of controlling the product temperature.

A hyaluronic acid derivative according to the second embodiment of the present invention is a hyaluronic acid derivative into which a steryl group is introduced, in which the ratio Pt/Pr of retention time Pt at the maximum point (peak-top) of the refractive index intensity of the hyaluronic acid derivative to the retention time Pr at the maximum point (peak-top) of the refractive index intensity of a 50 kDa polyacrylic acid as a standard substance on chromatograms obtained by gel permeation chromatography measurements is 0.5 or more and less than 1.0, preferably 0.7 or more and less than 0.95, and most preferably 0.75 or more and less than 0.92. When the ratio Pt/Pr is within the above-mentioned range, the hyaluronic acid derivative having a large particle size can be contained in a relatively large amount, and form a stable association state with a medicinal ingredient when formulated with the medicinal ingredient. Thus, the medicinal ingredient is allowed to be delivered stably and efficiently to immune cells (preferably DC, and more preferably cDC1), and an uptake of the medicinal ingredient into immune cells can be further improved, promoted or enhanced.

In the gel permeation chromatography measurement, a polyacrylic acid used as a standard substance is preferably a sodium polyacrylate, and more preferably ORDER No. PSS-Paa 50k (sodium polyacrylate) manufactured by Polymer Standards Service-USA.

The measurement by the gel permeation chromatography may be carried out by the following method, for example.

1 mg/mL of an aqueous solution of a hyaluronic acid derivative and 2 mg/mL of an aqueous solution of a polyacrylic acid as a standard substance are prepared, and the measurement by a gel permeation chromatograph is carried out under the following conditions.

### (Measurement conditions)

Device: High-speed GPC (gel permeation chromatography) device
Column: G4000SWXL (manufactured by TOSOH CORPORATION, particle size 8 µm, inner diameter 7.8 mm, length 30 cm, product number:8542)
Eluent: 10 mM phosphate buffer (pH 7.4)
Flow rate: 1 mL/min
Injection amount: 50 µL
Detector: RI (differential refractive index detector)
Temperature: 30°C

In addition, the explanation relating to the same constitution as the hyaluronic acid derivative according to the first embodiment will be omitted.

A hyaluronic acid derivative according to the third embodiment of the present invention is a hyaluronic acid derivative into which a steryl group is introduced, in which the weight-average molecular weight (based on polyacrylic acid) of the hyaluronic acid derivative is 110,000 or more and less than 500,000, preferably 130,000 or more and less than 300,000, and even more preferably 140,000 or more and less than 250,000. When the weight-average molecular weight (based on polyacrylic acid) of the hyaluronic acid derivative is within the above-mentioned range, the hyaluronic acid derivative having a large particle size can be contained in a relatively large amount, and form a stable association state with a medicinal ingredient when formulated with the medicinal ingredient. Thus, the medicinal ingredient is allowed to be delivered stably and efficiently to immune cells (preferably DC, and more preferably cDC1), and an uptake of the medicinal ingredient into immune cells can be further improved, promoted or enhanced.

In contrast, when the weight-average molecular weight (based on polyacrylic acid) of the hyaluronic acid derivative exceeds the above-mentioned upper limit, there is a case in which the viscosity increases, thereby making it difficult to use the hyaluronic acid derivative as a preparation.

The weight-average molecular weight (based on polyacrylic acid) of the hyaluronic acid derivative is calculated from the chromatogram obtained by a gel permeation chromatography measurement in accordance with the following formula: At3+Bt2+Ct+D, based on a calibration curve obtained using polyacrylic acids having a molecular weight of 2 kDa, 4 kDa, 8 kDa, 18 kDa, 40 kDa, or 150 kDa, as standard substances.

The polyacrylic acids used as standard substances are preferably sodium polyacrylates, and specifically preferably ORDER No. PSS-Pa series (sodium polyacrylates) manufactured by Polymer Standards Service-USA.

The measurement by the gel permeation chromatography may be carried out by the method described in the above-mentioned first or second embodiment. In addition, the explanation relating to the same constitution as the hyaluronic acid derivative according to the first embodiment and/or the second embodiment will be omitted.

### <<Pharmaceutical composition>>

The hyaluronic acid derivatives according to the first to third embodiments may be formulated with a medicinal ingredient to be used as a pharmaceutical composition.

Namely, a pharmaceutical composition of the present embodiment contains the above-mentioned hyaluronic acid derivative and a medicinal ingredient.

In the pharmaceutical composition of the present embodiment, the hyaluronic acid derivative forms a complex with the medicinal ingredient (hereinafter, may be referred to as "complex of the medicinal ingredient and the hyaluronic acid derivative"). Specifically, it is assumed that a steryl group in the hyaluronic acid derivative and a hydrophobic moiety of the medicinal ingredient form a complex due to hydrophobic interaction, thereby forming a core-shell type spherical structure in which the hydrophobic moieties of the medicinal ingredient, the steryl group, and the like are present in the center thereof, whilst a hydrophilic moiety such as a moiety derived from a hyaluronic acid in the hyaluronic acid derivative is present at the outer edge thereof. Namely, it is assumed that a structure in which the medicinal ingredient is encapsulated or enclosed in the hyaluronic acid derivative is formed.

In the pharmaceutical composition of the present embodiment, the average particle size of the spherical structure containing the complex of the medicinal ingredient and the hyaluronic acid derivative may be 20 nm or more and 100 nm or less, 20 nm or more and 95 nm or less, or 20 nm or more and 90 nm or less. The term "average particle size" used here refers to a value expressed as a z average. When the average particle size is within the above-mentioned range, the structure can be stable in vivo and can more easily pass through lymph nodes. The average particle size may be measured by, for example, DLS (Dynamic Light Scattering), a nanotracking particle measuring device, size exclusion chromatography, high-performance liquid chromatography, electron microscopy method, or the like. More specifically, for example, dilution is carried out with 10 mM phosphate buffer containing 10 w/v% sucrose such that the concentration of the hyaluronic acid derivative becomes 1 mg/mL to carry out measurement using a DLS device.

In the pharmaceutical composition of the present embodiment, the weight-average molecular weight Mw (based on polyacrylic acid) of the hyaluronic acid derivative or the complex of the medicinal ingredient and the hyaluronic acid derivative is preferably 110,000 or more. The weight-average molecular weight Mw (based on polyacrylic acid) may be calculated by creating a calibration curve using plural polyacrylic acid standard substances having different molecular weights. The polyacrylic acid standard substances used are preferably sodium polyacrylates, and more preferably ORDER No. PSS-Paa series (sodium polyacrylates) manufactured by Polymer Standards Service-USA.

When the weight-average molecular weight Mw is within the above-mentioned range, it is assumed that a larger amount of associated products having a size suitable to activate co-stimulatory molecules is present, the use of the pharmaceutical composition of the present embodiment makes it possible to stably and efficiently deliver the medicinal ingredient such as a peptide to immune cells (especially cDC1 or macrophages), furthermore improve, promote, or enhance the uptake of the medicinal ingredient into immune cells, and simultaneously improve, promote, maintain or enhance the activation of the immune cells (especially, expression of co-stimulatory molecules CD80 and CD86 on cDC1).

The weight-average molecular weight Mw may be measured, for example, by DLS (Dynamic Light Scattering), size exclusion chromatography, an electron microscopy method, or the like. More specifically, for example, dilution is carried out such that the concentration of the hyaluronic acid derivative becomes 1 mg/mL to carry out the measurement using a size exclusion chromatography device.

In the pharmaceutical composition of the present embodiment, the peak top time in the particle size distribution of the hyaluronic acid derivative or the complex of the medicinal ingredient and the hyaluronic acid derivative by size exclusion chromatography is preferably smaller than the peak top time of the polyacrylic acid (50 kDa) as a standard substance. When the peak top time is within the above-mentioned range, it is assumed that a larger amount of associated products having a size suitable to activate co-stimulatory molecules is present, the use of the pharmaceutical composition of the present embodiment makes it possible to stably and efficiently deliver the medicinal ingredient such as a peptide to immune cells (especially cDC1 or macrophages), furthermore improve, promote, or enhance the uptake of the medicinal ingredient into immune cells, and simultaneously improve, promote, maintain or enhance the activation of the immune cells (especially, expression of co-stimulatory molecules CD80 and CD86 on cDC1). The peak-top time can be measured, for example, by size exclusion chromatography. More specifically, for example, dilution is carried out such that the concentration of the hyaluronic acid derivative becomes 1 mg/mL to carry out measurement using a size exclusion chromatography device.

Although the amount of the hyaluronic acid derivative in the pharmaceutical composition of the present embodiment is not particularly limited, the amount relative to 100 parts by mass of the pharmaceutical composition is preferably 0.01 parts by mass or more and 50.00 parts by mass or less, more preferably 0.10 parts by mass or more and 25.00 parts by mass or less, and even more preferably 0.20 parts by mass and 10.00 parts by mass or less.

Next, constituent components of the present embodiment will be explained below in detail.

### <Medicinal ingredient>

Although the medicinal ingredient is not particularly limited, examples thereof include antigens (such as cancer antigens, antigens derived from infectious diseases, and autoantigens in immune diseases), pharmaceutically active peptides or proteins, nucleic acids, low-molecular compounds, and middle-molecular compounds. Among them, antigens are preferable, at least one selected from the group consisting of cancer antigens, antigens derived from infectious diseases, and autoantigens in immune diseases is more preferable, and cancer antigens or antigens derived from infectious diseases are even more preferable.

Namely, the pharmaceutical composition of the present embodiment is preferably a pharmaceutical composition to be used to prevent or treat at least one disease selected from the group consisting of cancers, infectious diseases and immune diseases, and more preferably a pharmaceutical composition to be used to prevent or treat a cancer or an infectious disease. When the application disease is cancer, infectious disease or immune disease, the pharmaceutical composition of the present embodiment may also be referred to as a vaccine composition.

### [Antigens]

### (Cancer antigens)

Cancer antigens are antigens that are often expressed by cancer cells, and in some cases are expressed only by cancer cells. Cancer antigens can be expressed inside cancer cells or on the surface of cancer cells.

Although there are no limitations on antigenic proteins available in the pharmaceutical composition of the present embodiment, examples thereof include ERK1, ERK2, WT1, MART-1/Melan-A, gp100, adenosine deaminase-binding protein (ADAbp), FAP, cyclophilin b, colorectal-related antigen (CRC)-C017-1A/GA733, carcinoembryonic antigen (CEA), CAP-1, CAP-2, etv6, AML1, prostate specific antigen (PSA), PSA-1, PSA-2, PSA-3, prostate specific membrane antigen (PSMA), T cell receptor/CD3-zeta chain, CD20, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5, GAGE-1, GAGE-2, GAGE-3, GAGE-4, GAGE-5, GAGE-6, GAGE-7, GAGE-8, GAGE-9, BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin, γ-catenin, p120 ctn, gp 100 Pmel 117, PRAME, NY-ESO-1, cdc 27, adenomatous polyposis protein (APC), fodrin, connexin 37, Ig idiotype, p15, gp75, GM2 ganglioside, GD2 ganglioside, human papillomavirus protein, Smad family of tumor antigens, lmp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1, CT-7, CD 20, and c-erbB-2.

As the above-mentioned antigenic proteins, the entire sequence thereof may be used, or a partially deleted sequence may be used.

The antigenic peptide available in the pharmaceutical composition of the present embodiment is an antigenic peptide containing at least one epitope selected from the group consisting of a CD8-positive cytotoxic T cell recognition epitope and a CD4-positive helper T cell recognition epitope among the antigenic protein sequences. In one embodiment, the antigenic peptide is preferably an antigenic peptide containing at least two epitopes from the viewpoint of being loaded on MHC class I molecules or MHC class II molecules after being decomposed in antigen-presenting cells. Specific examples of the above-mentioned antigenic peptide include an antigenic peptide containing an epitope of an antigenic protein of tumor cells.

In one embodiment, the antigenic peptide has, for example, 8 to 120 amino acids, preferably 8 to 80 amino acids, more preferably 15 to 80 amino acids, even more preferably 16 to 80 amino acids, even more preferably 23 to 80 amino acids, and even more preferably 23 to 60 amino acids, and particularly preferably 23 to 50 amino acids.

In one embodiment, the above-mentioned antigenic peptide is an antigenic peptide having at least one CD8-positive cytotoxic T cell recognition epitope and at least one CD4-positive helper T cell recognition epitope, from the viewpoint of inducing activation of cytotoxic T cells (CTL) by helper T cells.

In one embodiment, when at least two epitopes are included, an amino acid linker may be placed between the epitopes. The linker has, for example, two to ten amino acids, preferably four to ten amino acids, and more preferably four to eight amino acids.

Examples of the amino acids used in the linker include glycine (G), tyrosine (Y), leucine (L), and tryptophan (W). The amino acids are preferably tyrosine (Y), leucine (L), and tryptophan (W). Specific examples of the amino acid linker include a linker (4Y) consisting of four consecutive tyrosines (Y), a linker (4L) consisting of four consecutive leucines (L), a linker (4W) consisting of four consecutive tryptophans (W), a linker (6G) consisting of six consecutive glycines (G), a linker (6Y) consisting of six consecutive tyrosines (Y), a linker (6L) consisting of six consecutive leucines (L), a linker (6W) consisting of six consecutive tryptophans (W), a linker (8Y) consisting of eight consecutive tyrosinea (Y), a linker (8L) consisting of six consecutive leucines (L), and a linker (8W) consisting of eight consecutive tryptophans (W), and the amino acid linker is preferably 6Y, 6L or 6W.

Cancer antigens may be tumor-associated antigens, cancer testis antigens, viral antigens, or tumor-specific antigens (including neoantigens). One of the cancer antigens may be used alone or at least two thereof may be used in combination.

### (Antigens derived from infectious diseases)

There are no particular limitations on antigens derived from infectious diseases, as long as the antigens are infectious pathogens or antigens derived from infectious pathogens. Examples of the infectious pathogens include viruses, bacteria, fungi, and nematodes. The antigens derived from infectious pathogens may be antigenic proteins or antigenic peptides.

The diseases caused by the above-mentioned infectious pathogens are not particularly limited, and examples thereof include: viral diseases such as diseases caused by infection with viruses such as adenoviruses, herpesviruses (such as HSV-I, HSV-II, CMV, and VZV), poxviruses (such as orthopoxviruses such as variola, vaccinia, and molluscum contagiosum virus), picornaviruses (such as rhinoviruses and enteroviruses), orthomyxoviruses (such as influenza viruses), paramyxoviruses (such as parainfluenza viruses, mumps viruses, measles viruses, and respiratory syncytial virus (RSV)), coronaviruses (such as SARS coronavirus (SARS-CoV), MERS coronavirus (MERS-CoV), and SARS-CoV-2), papovaviruses (such as papillomaviruses which cause genital warts, common warts or plantar warts), hepadnaviruses (such as hepatitis B virus), flaviviruses (such as hepatitis C virus and dengue virus), retroviruses (such as lentiviruses such as HIV); bacterial diseases such as diseases caused by infection with bacteria such as Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campylobacter, Vibrio, Serratia, Providencia, Chromobacterium, Brucella, Yersinia, Haemophilus, or Bordetella; fungal diseases such as chlamydia, candidiasis, aspergillosis, histoplasmosis and cryptococcal meningitis; malaria, Pneumocystis carinii pneumonia, leishmaniasis, cryptosporidiosis, toxoplasmosis, and trypanosome infection.

There are no particular limitations on the structure of the antigen available in the pharmaceutical composition of the present embodiment as long as the structure is at least a part of various components constituting a pathogen, and examples thereof include live vaccines, inactivated whole particles, a part thereof, protein subunits, proteins, and peptides. Among them, protein subunits, proteins, or peptides are preferable from the viewpoint of conjugation with hyaluronic acid derivatives.

The above-mentioned influenza virus is an RNA envelope virus belonging to Orthomyxoviridae, and having a particle size of about 100 nm in diameter, and is classified into types A, B and C based on the antigenicity of the internal protein. The influenza virus is composed of a core of ribonucleic acid (RNA) associated with an internal nucleocapsid surrounded by a virus envelope having a lipid bilayer structure or nucleic protein, and an external glycoprotein. The inner layer of the virus envelope is mainly formed of a matrix protein, and the outer layer is mostly formed of a lipid substance derived from the host. RNA of the influenza virus has a multipartite structure. Pandemic influenza is caused by an influenza A virus, and the influenza A virus has two envelope glycoproteins, namely hemagglutinin and neuraminidase, the hemagglutinin being classified into 16 subtypes and the neuraminidase being 9 subtypes depending on the antigenicity.

As the antigens derived from infectious diseases, antigens derived from influenza A virus or influenza B virus are preferably used. The subtype of the influenza A or B virus is not particularly limited, and may be a subtype that has been already isolated, or a subtype that will be isolated in future.

As the antigens derived from influenza viruses are not particularly limited as long as the antigens are at least a part of various components constituting the influenza viruses, and examples thereof include: viral whole particles obtained by inactivating purified viral particles with an organic solvent, a surfactant or another reagent; and viral subunits formed by removing impurities from the viral whole particles to purify hemagglutinin and /or neuraminidase. From the viewpoint of immunogenicity, hemagglutinin subunit or a viral whole particle is preferable. The viral whole particle is preferably inactivated with formalin or the like. Furthermore, a hemagglutinin subunit (split), which has few impurities and requires an adjuvant such as an immunostimulant, is particularly effective.

The method for preparing the aforementioned influenza virus antigen is not limited, and any known method may be used without restriction. Examples thereof include a method including: infecting a chicken egg or the like with a viral strain that is isolated from an animal or a patient infected with influenza; culturing the viral strain by an ordinary method; and preparing an antigen from the purified undiluted viral culture. Also an antigen derived from a virus prepared in cultured cells by genetic engineering may be used.

### (Antigens in immune diseases)

Antigens in immune diseases are not particularly limited as long as the antigens contain epitopes of target proteins of immune diseases. Immune diseases are not particularly limited, and examples thereof include psoriasis vulgaris, ankylosing spondylitis, rheumatoid arthritis, psoriatic arthritis, axial spondyloarthritis, Crohn's disease, ulcerative colitis, bronchial asthma, chronic urticaria, hay fever, and atopic dermatitis.

Target proteins are not particularly limited, and examples thereof include IL-17A, DPP4, S100A9, PCSK9, IL-23, IgE, TNFα, IL-12/23 p40, IL-6, α4β7 integrin, IL-4/13, IL-5, BLyS and IL-13. Reference Document 2 (International Patent Application, Publication No. 2017/164409) describes a peptide derived from IL-17A.

The peptide may have not only an epitope of the target protein, but also an epitope of a protein other than the target protein. The peptide may be a B cell epitope or a T cell epitope.

The peptide is a sequence that has an epitope and may have a cyclic structure. The peptide may have plural cyclic structures in the molecule thereof.

**In** addition, the peptide may be conjugated to a protein.

Treatment effects are expected by producing an antibody against an administered protein or peptide in vivo.

### [Pharmaceutically active peptide or protein]

The term "pharmaceutically active peptide or protein" means one that has a positive or beneficial effect on a condition or disease state of a subject when administered to the subject in a therapeutically effective amount. The preferable pharmaceutically active peptide or protein has curative or palliative properties and may be administered to ameliorate, relieve, alleviate, or reverse one of diseases or disorders or plural symptoms, or delay the onset of symptoms, or reduce the severity of symptoms. Since the pharmaceutically active peptide or protein may also have prophylactic properties, the pharmaceutically active peptide or protein may be used to delay the onset of disease or reduce the severity of such a disease or pathological condition. The term "pharmaceutically active peptide or protein" encompasses a full-length protein or polypeptide and may also refer to a pharmaceutically active fragment thereof. The term also encompasses pharmaceutically active analogs of peptides or proteins.

Although the pharmaceutically active protein is not limited to the following examples, the examples include cytokines such as immunoactive compounds and immune system proteins (such as interleukins, colony stimulating factor (CSF), granulocyte-colony stimulating factor (G-CSF), granulocyte macrophage-colony stimulating factor (GM-CSF), erythropoietin, tumor necrosis factor (TNF), interferon, integrin, addressin, seletin, homing receptor, T cell receptor, immunoglobulin, antibodies, hormones (such as insulin, thyroid hormone, catecholamine, gonadotropin, stimulating hormones, prolactin, oxytocin, dopamine, bovine somatotropin, and leptin), growth hormones (such as human growth hormone), growth factors (such as epidermal growth factor, nerve growth factor, and insulin-like growth factor), growth factor receptors, enzymes (such as tissue plasminogen activator, streptokinase, cholesterol biosynthetic or degrading enzymes, steroidogenic enzymes, kinases, phosphodiesterases, methylases, demethylases, dehydrogenases, cellulases, proteases, lipase, phospholipase, aromatase, cytochrome, adenylate cyclase or guanylaste cyclase, and neuramidase), receptors (such as steroid hormone receptors, and peptide receptors), binding proteins (such as growth hormone binding protein or growth factor binding proteins), transcription factors and translation factors, tumor growth suppressor proteins (such as proteins that inhibit angiogenesis), structural proteins (such as collagen, fibroin, fibrinogen, elastin, tubulin, actin, and myosin), and blood proteins (such as thrombin, serum albumin, factor VII, factor VIII, insulin, factor IX, factor X, tissue plasminogen activator, protein C, von Willebrand factor, antithrombin III, glucocerebrosidase, erythropoietin, modified factor VIII, and anticoagulant factor).

In one embodiment, the pharmaceutically active protein is a cytokine that participates in the regulation of lymphocyte homeostasis, preferably a cytokine that participates in one or more selected from the group consisting of development, priming, expansion, differentiation and survival of T cells and induces or enhances them.

In one embodiment, the cytokine is an interleukin.

In one embodiment, the pharmaceutically active protein is one or more interleukins selected from the group consisting of IL-2, IL-7, IL-12, IL-15 and IL-21.

In one embodiment, the pharmaceutically active peptide may have a cyclic structure. The peptide may have plural cyclic structures in the molecule thereof.

### [Nucleic acids]

Examples of nucleic acids include DNA, RNA, antisense nucleic acids, decoy nucleic acids, ribozymes, small interfering RNA, and nucleic acid aptamers. Furthermore, when the antigen is a peptide or protein, nucleic acids (such as DNA or mRNA) encoding the antigenic peptide or protein are also preferably used.

### [Low-molecular compounds]

Examples of low-molecular compounds include anticancer agents (such as alkylating agents, antimetabolites, and alkaloids), immunosuppressants, anti-inflammatory agents (such as steroidal agents and nonsteroidal anti-inflammatory agents), antirheumatic agents, and antibacterial agents (such as β-lactam antibiotics, aminoglycoside antibiotics, macrolide antibiotics, tetracycline antibiotics, new quinolone antibiotics, and sulfa agents).

Furthermore, highly hydrophobic, namely, sparingly water-soluble ones may also be preferably used as medicinal ingredients, because the interaction with a steryl group of the above-mentioned hyaluronic acid derivative can be sufficiently exhibited. The term "sparingly water-soluble" is defined in the 17th edition of the Japanese Pharmacopoeia as requiring 30 mL or more of water to dissolve 1 g of solute.

Examples of sparingly water-soluble solid medicinal ingredients include pharmaceutical medicinal components disclosed in "Japanese Pharmacopoeia", "Japanese Pharmaceutical Codex", "USP (United States Pharmacopoeia)", "NF (National Formulary)" and "EP (European Pharmacopoeia)", such as antipyretic analgesics, nervous system drugs, sedative hypnotic drugs, muscle relaxants, hypotensive agent and antihistamines such as acetaminophen, ibuprofen, benzoic acid, ethenzamide, caffeine, camphor, quinine, calcium gluconate, dimercaprol, sulfamine, theophylline, theobromine, riboflavin, mephenesin, phenobarbital, aminophylline, thioacetazone, quercetin, rutin, salicylic acid, theophylline sodium, pyrabital, quinine hydrochloride, irgapyrin, digitoxin, griseofulvin, and phenacetin; antibiotics such as acetylspiramycin, ampicillin, erythromycin, kitasamycin, chloramphenicol, triacetyloleandomycin, nystatin, and colistin sulfate; steroid hormones such as methyltestosterone, methylandrostenediol, progesterone, estradiol benzoate, ethinylestradiol, deoxycorticosterone acetate, cortisone acetate, hydrocortisone, hydrocortisone acetate, and prednisolone; non-steroidal estrogen hormones such as dienestrol, hexestrol, diethylstilbesterol, diethylstilbestrol dipropionate, and chlorotrianisene; and other fat-soluble vitamins. One selected from these medicinal ingredients may be used or a combination of at least two thereof may be used.

The medicinal ingredient may also be a sparingly water-soluble oil or liquid. Examples of the sparingly water-soluble oil or liquid medicinal ingredient include pharmaceutical medicinal components disclosed in "Japanese Pharmacopoeia", "Japanese Pharmaceutical Codex", "USP", "NF" and "EP", such as vitamins such as teprenone, indometacin farnesil, nienatetrenone, phytonadione, vitamin A oil, fenipentol, vitamin D and vitamin E, higher unsaturated fatty acids such as DHA (docosahexaenoic acid), EPA (eicosapentaenoic acid) and cod liver oil, coenzyme Q substances, and oil-soluble flavorings such as orange oil, lemon oil and peppermint oil. Although vitamin E has various homologues and derivatives, there are no particular limitations thereon as long as it is liquid at normal temperatures, and examples thereof include dl-α-tocopherol, dl-α-tocopherol acetate, d-α-tocopherol, and d-α-tocopherol acetate. One selected from these medicinal ingredients may be used or a combination of at least two thereof may be used.

The medicinal ingredient may also be a sparingly water-soluble semisolid substance. Examples of the sparingly water-soluble semisolid medicinal ingredient include traditional Chinese medicines and crude drug extracts such as ziryuu, glycyrrhiza, cassia bark, Chinese peony, moutan bark, Japanese valerian, zanthoxylum, ginger, citrus unshiu, ephedra, nantenzitsu, oohi, polygala root, platycodon root, plantago seed, plantago herb, lycoris radiata bulb, senega, fritillaria bulb, fennel, phellodendron bark, coptis rhizome, curcuma zedoaria, chamomile, gentian, oriental bezoar, animal bile, syazin, ginger, atractylodes lancea rhizome, clove, citrus unshiu, atractylodes rhizome, panax rhizome, ginseng, kakkon-to, keishi-to, kousosan, saiko-keishi-to, sho-saiko-to, sho-seiryu-to, bakumondo-to, hange-koboku-to, and mao-to; as well as oyster meat extract, propolis and propolis extract, and coenzyme Q substances. One selected from these medicinal ingredients may be used or a combination of at least two thereof may be used.

Although the amount of the medicinal ingredient in the pharmaceutical composition of the present embodiment depends on the structure of the medicinal ingredient, the amount of the medicinal ingredient relative to the mass of the hyaluronic acid derivative may be 0.001% by mass or more and 10,000% by mass or less, preferably 0.1% by mass or more and 1000% by mass or less, more preferably 1.0% by mass or more and 100.0% by mass or less, even more preferably 1.5% by mass or more and 50.0% by mass or less, particularly preferably 3.0% by mass or more and 30.0% by mass or less, and most preferably 5.0% by mass or more and 20.0% by mass or less.

Alternatively, the amount of the medicinal ingredient in the pharmaceutical composition of the present embodiment relative to the mass of the hyaluronic acid derivative is more preferably 0.1% by mass or more and 100.0% by mass or less, even more preferably 0.1% by mass or more and 50.0% by mass or less, even more preferably 0.1% by mass or more and 30.0% by mass or less, even more preferably 0.5% by mass or more and 30.0% by mass or less, even more preferably 1.0% by mass or more and 30.0% by mass or less, even more preferably 1.0% by mass or more and 25.0% by mass or less, even more preferably 1.0% by mass or more and 20.0% by mass or less, even more preferably 1.0% by mass or more and 15.0% by mass or less, and particularly preferably 1.0% by mass or more and 10.0% by mass or less.

Alternatively, the amount of the medicinal ingredient in the pharmaceutical composition of the present embodiment relative to 100% by mass of the pharmaceutical composition is preferably 0.0001 parts by mass or more and 1.00 part by mass or less, more preferably 0.001 parts by mass or more and 0.100 parts by mass or less, and even more preferably 0.002 parts by mass or more and 0.500 parts by mass or less.

Alternatively, the amount of the medicinal ingredient in the pharmaceutical composition of the present embodiment relative to 100% by mass of the pharmaceutical composition is preferably 0.001 parts by mass or more and 1.00 part by mass or less, more preferably 0.001 parts by mass or more and 0.500 parts by mass or less, even more preferably 0.001 parts by mass or more and 0.200 parts by mass or less, and even more preferably 0.005 parts by mass or more and 0.100 parts by mass or less.

When the amount of the medicinal ingredient is the above-mentioned lower limit or more, it is possible to activate immune cells more effectively. In contrast, when the amount is the above-mentioned upper limit or less, it is possible to encapsulate the medicinal ingredient in the hyaluronic acid derivative component, thereby making the structure further stable.

### <Adjuvant>

When the pharmaceutical composition of the present embodiment is a vaccine composition, the pharmaceutical composition may further contain an adjuvant in addition to the above-mentioned medicinal ingredient and the above-mentioned hyaluronic acid derivative. Thereby, immunity can be induced more effectively. Here, the induced immunity may be either humoral immunity or cell-mediated immunity. Namely, the pharmaceutical composition of the present embodiment is preferably a pharmaceutical composition containing the hyaluronic acid derivative and an antigen (preferably a cancer antigen or an antigen derived from an infectious disease) as the medicinal ingredient, and further an adjuvant.

The term "humoral immunity" generally refers to an immune mechanism in which B cells and antibodies play the main role. Cytokines produced by helper T cells (Th2 cells) stimulate B cells, which differentiate into plasma cells and produce a large amount of antibodies, which circulate in body fluids and spread throughout the body. In addition, some of the stimulated B cells become memory B cells that remember information about the antigens, thereby making it possible, upon reinfection, to produce a large amount of antibodies having a higher affinity against the antigens faster than the initial response.

On the other hand, the term "cell-mediated immunity" refers to an immune mechanism in which cells are the main effectors in eliminating foreign substances such as pathogens, cells infected with virus, or cancer cells. This is an elimination mechanism by immunocompetent cells such as macrophages, cytotoxic T cells (such as CTL and killer T cells), and natural killer cells (NK cells).

Although the adjuvant is not particularly limited as long as it is commonly used in vaccines, examples thereof include aluminum salts, squalene, and ligands of innate immunoreceptors.

The term "ligand" used herein refers to a substance that specifically binds to a receptor, and particularly a substance that specifically binds to a receptor and exhibits various physiological effects can be used. Such a substance may also be referred to as "agonist."

Examples of the innate immunoreceptor include toll-like receptor (TLR), RIG-I-like receptor (RLR), NOD-like receptor (NLR), and C-type lectin receptor (CLR).

As the TLR ligand, for example, one that interacts with at least one TLR selected from the group consisting of TLR-2, TLR-3, TLR-4, TLR-5, TLR-6, TLR-7, TLR-8, and TLR-9 may be appropriately used.

Examples of the TLR-2 ligand include Pam3CSK4.

Examples of the TLR-3 ligand include poly ICLC, and polyinosine: polycytidylic acid (poly I:C).

Examples of the TLR-4 ligand include R-type lipopolysaccharide, S-type lipopolysaccharide, Paclitaxel, lipid A, and monophosphoryl lipid A

Examples of the TLR-5 ligand include flagellin.

Examples of the TLR-2 and TLR-6 ligands include MALP-2.

Examples of the TLR-7 and TLR-8 ligands include resiquimod (R848), imiquimod (R837), gardiquimod, and loxoribine.

Examples of the TLR-9 ligand include CpG oligodeoxynucleotides.

Among them, the anionic compounds are preferable, and CpG oligodeoxynucleotides are more preferable as the adjuvants, since antigen-presentation effects can be further improved.

Examples of the CpG oligodeoxynucleotides include CpG-ODN 1826 and CpG-K3.

### <Other additives>

The pharmaceutical composition of the present embodiment can be administered alone or together with a pharmacologically acceptable carrier by a conventional method. When used in combination with a pharmacologically acceptable carrier, for example, the above-mentioned hyaluronic acid derivative and the above-mentioned medicinal ingredient, and if necessary an adjuvant, and water or other physiologically acceptable liquid (such as physiological saline, aqueous ethanol, or phosphate-buffered saline (PBS)) may be mixed, or physiologically acceptable buffers, excipients, vehicles, preservatives, stabilizers, binders, freeze-drying aids or the like may also be included.

Examples of the buffer include Tris, sodium phosphate, potassium phosphate, histidine, and citric acid.

Examples of the preservative include benzalkonium chloride, methyl paraoxybenzoate, propyl paraoxybenzoate, chlorobutanol, sorbic acid, and alkylpolyaminoethylglycine.

Examples of the stabilizer include sodium edetate hydrate, polyvinylpyrrolidone (povidone), and polysorbate 80.

The pharmaceutical composition of the present embodiment may be formulated. The formulation may be in a solid, semi-solid or liquid form.

In the case of the solid form, examples thereof include powder, granule, pill, pellet, tablet, and capsule. Among them, the solid form is preferably a freeze-dried powder.

In the case of the semi-solid form, examples thereof include gel.

In the case of the liquid form, examples thereof include suspension in which powders are diluted with or suspended in water or a buffer such as phosphate-buffered saline (PBS).

### <Method for producing a pharmaceutical composition>

Although the pharmaceutical composition of the present embodiment may be prepared by appropriately mixing the hyaluronic acid derivative and the medicinal ingredient, the pharmaceutical composition is preferably prepared by the following method.

The method for producing a pharmaceutical composition of the present embodiment is a method for producing a pharmaceutical composition containing the hyaluronic acid derivative and the medicinal ingredient, the method including the following steps:
a preparation step in which the medicinal ingredient is dissolved in an organic solvent or an organic solvent containing water to prepare an oil phase containing the medicinal ingredient; and
a mixing step in which the oil phase and an aqueous phase containing the hyaluronic acid derivative are mixed such that the mixing ratio of the oil phase and the aqueous phase, based on volume, becomes 20:100 to 0.01:100.

It was conventionally difficult to maintain the particle size of the hyaluronic acid derivative since the interaction between steryl groups was inhibited by the oil phase depending on the ratio of the oil phase and the aqueous phase, thereby causing disintegration of particles or aggregation of some particles. Therefore, only complexes of hyaluronic acid derivatives containing disintegrated particles had relatively small sizes or partially aggregated particles with medicinal ingredients were contained in a pharmaceutical composition. Furthermore, since the pharmaceutical composition was prepared using a dialysis method, it was difficult to control the weight ratio of the hyaluronic acid derivative and the medicinal ingredient, therefore the weight ratio was left to nature. Furthermore, a concentration step was necessary to achieve the target concentration of the medicinal ingredient.

In contrast, the method for producing a pharmaceutical composition of the present embodiment does not use the dialysis method, and, in particular, mixing of the above-mentioned components such that the mixing ratio of the oil phase and the aqueous phase is within the above-mentioned range makes it possible to form a complex of the hyaluronic acid derivative and the medicinal ingredient while maintaining the particle size distribution of the hyaluronic acid derivative before preparing the pharmaceutical composition and to control the weight ratio of the hyaluronic acid derivative and the medicinal ingredient to a constant level. Thus, the complex of the medicinal ingredient and the hyaluronic acid derivative can be obtained with a stable structure. As a result, a pharmaceutical composition that has excellent delivery properties to immune cells in lymph nodes and an improved activation ability against the immune cells can be obtained.

Next, each step of the method for producing the pharmaceutical composition of the present embodiment will be explained in detail below.

### [Preparation step]

In the preparation step, the medicinal ingredient is dissolved in an organic solvent or an organic solvent containing water to prepare an oil phase containing the medicinal ingredient.

A sparingly water-soluble medicinal ingredient is preferably used, since the medicinal ingredient is dissolved in an organic solvent or organic solvent-containing water. Specific examples of the medicinal ingredient include those exemplified as the "medicinal ingredient" above.

Examples of an organic solvent in which the medicinal ingredient is dissolved include known ones that are generally used to prepare pharmaceutical compositions, and specific examples thereof include dimethyl sulfoxide (DMSO), methanol, ethanol, tetrahydrofuran, acetone, acetonitrile, ethyl acetate, 1-propanol, 2-propanol, 1-butanol, 2-butanol, 1-pentanol, methyl ethyl ketone, heptane, isobutyl acetate, isopropyl acetate, methyl acetate, methyl isobutyl ketone, and dimethylformamide.

### [Mixing step]

In the mixing step, the oil phase obtained in the preparation step and an aqueous phase containing the hyaluronic acid derivative are mixed such that the mixing ratio of the oil phase and the aqueous phase, based on volume, becomes 20:100 to 0.01:100.

The hyaluronic acid derivative is used after being dissolved or dispersed in water or the like in advance to a desired concentration. At this time, the pH of the aqueous phase containing the hyaluronic acid derivative is preferably 6.00 or more, and more preferably 6.00 or more and 11.00 or less. When the pH is the above-mentioned lower limit or more, a complex of the hyaluronic acid derivative with the medicinal ingredient can be formed while more effectively suppressing aggregation of the hyaluronic acid derivative, thereby obtaining the complex of the medicinal ingredient and the hyaluronic acid derivative in a more stable structure. When the pH is the upper limit or less, a drug can be made into a complex while maintaining the dispersion stability of the hyaluronic acid derivative. Furthermore, since the charge of the drug tends to be negative, the hydrophobic moiety of the hyaluronic acid derivative and the drug tend to interact easily. In contrast, when the pH is the above-mentioned upper limit or less, decomposition of the main chain of the hyaluronic acid derivative can be further suppressed.

In the mixing step, the hydrophobic interaction between a steryl group of the hyaluronic acid derivative and the medicinal ingredient allows the complex of the medicinal ingredient and the hyaluronic acid derivative to have a structure in which the steryl group and the medicinal ingredient are present inside, while the hydrophilic moiety derived from a hyaluronic acid is present at the outer edge. The complex can be formed not only through the above-mentioned hydrophobic interaction but also through an interaction such as an electrostatic interaction or hydrogen bond depending on the structure of the medicinal ingredient.

In the mixing step, the mixing ratio of the oil phase and the aqueous phase, based on volume, is 20:100 to 0.01:100, preferably 10:100 to 0.05:100, more preferably 5:100 to 0.1:100, and even more preferably 2.5:100 to 0.5:100. When the mixing ratio of the oil phase and the aqueous phase is within the above-mentioned range, a sufficient amount of the medicinal ingredient can be encapsulated in the hyaluronic acid derivative to form a complex of the medicinal ingredient and the hyaluronic acid derivative, and disassociation of particles of the hyaluronic acid derivative due to inhibition of hydrophobic interaction between steryl groups by the oil phase can be effectively suppressed. Not only the deterioration of the hydrophobic interaction, but also the deterioration of an interaction that can contribute to structural stability, such as, electrostatic interaction or hydrogen bond can be effectively suppressed.

In the mixing step, the concentration of the medicinal ingredient that is dissolved in the oil phase is not particularly limited, but the concentration relative to 100 parts by mass of the oil phase is preferably 0.1 parts by mass or more and 50 parts by mass or less, more preferably 0.2 parts by mass or more and 25 parts by mass or less, and even more preferably 0.5 parts by mass or more and 10 parts by mass or less.

In the mixing step, the concentration of the hyaluronic acid derivative that is dissolved in the aqueous phase is not particularly limited, but the concentration relative to 100 parts by mass of the aqueous phase is preferably 0.01 parts by mass or more and 10 parts by mass or less, more preferably 0.1 parts by mass or more and 10 parts by mass or less, and even more preferably 0.5 parts by mass or more and 5 parts by mass or less.

In the mixing step, the temperature is preferably set to 20°C or more and 65°C or less, more preferably 23°C or more and 55°C or less, and even more preferably 25°C or more and 40°C or less, from the viewpoint of slightly exposing the hydrophobic moiety of the medicinal ingredient to make it easier to interact with the hyaluronic acid derivative. Alternatively, the optimum temperature may be appropriately set depending on the structure and properties of the medicinal ingredient.

Although the method is not particularly limited in the mixing step, the particle size of the hyaluronic acid derivative can be maintained through a mixing step in which an oil phase containing the medicinal ingredient is added to an aqueous phase containing the hyaluronic acid derivative at an arbitrary ratio, or an aqueous phase containing the hyaluronic acid derivative is added to an oil phase containing the medicinal ingredient at an arbitrary ratio such that the final mixing ratio of the oil phase and the aqueous phase based on volume is 20:100 to 0.01:100, for example.

The mixing step is not limited to a batch method, and the oil phase and the aqueous phase may be mixed by a flow method (continuous method). At this time, the particle size of the hyaluronic acid derivative can be maintained by setting the mixing ratio of the oil phase and the water phase to a volume ratio of 20:100 to 0.01:100.

Although the material and shape of a reactor in the flow method are not particularly limited as long as the material and shape are applicable to the production method of the present invention, the inner diameters of pipes through which the oil phase and the aqueous phase pass may be different from each other, for example.

The material and shape of the tube in the flow method are not particularly limited as long as the material and shape are applicable to the method for producing the pharmaceutical composition of the present embodiment, and examples thereof include Teflon (registered trademark) tubes, stainless steel tubes, glass tubes, and plastic tubes.

Although the time is not particularly limited in the mixing step, the time may be, for example, 30 minutes or more and 90 hours or less, or one hour or more and 80 hours or less.

The mixing step may include a step of removing an organic solvent in which the medicinal ingredient is dissolved by carrying out a tangential flow filtration (TFF) process, a centrifugal filtration process, or plural TFF processes with ultrafiltration/diafiltration (UFDF) membranes, or the like, after the medicinal ingredient is complexed with the hyaluronic acid derivative.

### [Sterilization step]

After the mixing step, a sterilization step may be carried out in which the solution containing the obtained complex of the medicinal ingredient and the hyaluronic acid derivative is sterilized to obtain a sterile preparation. Examples of the method for sterilizing the preparation include filtration sterilization using a sterilization filter, gas sterilization, γ-ray sterilization, and electron beam sterilization. The filtration sterilization using a sterilizing filter is most preferable.

### [Drying step]

After the mixing step, a drying step may be carried out in which the obtained solution containing the complex of the medicinal ingredient and the hyaluronic acid derivative is dried to obtain a dried product. Examples of the drying method include the same methods as those exemplified in the above "method for producing hyaluronic acid derivative."

### <Administration method>

Subjects to which the pharmaceutical composition of the present embodiment is administered include animals classified as mammals including humans (such as monkeys, marmosets, mice, rats, cows, horses, cats, dogs, pigs, sheep, goats, and rabbits).

Examples of the administration route include intrathecal injection, intraarterial injection, intravenous injection, and subcutaneous injection, as well as intranasal, transbronchial, transpulmonary, intramuscular, percutaneous, and oral administrations, which can be carried out by methods known to those skilled in the art. Among them, subcutaneous injection or intramuscular injection is preferable, when the pharmaceutical composition of the present is a vaccine composition.

When the pharmaceutical composition of the present embodiment is administered parenterally, although the dosage thereof may be appropriately determined taking into consideration the type of subject (such as age and sex), the amount of the medicinal ingredient (preferably antigen) per dose for humans (assuming that the body weight thereof is 60 kg) may be generally 0.01 µg or more and 5 mg or less, 0.1 µg or more and 500 µg or less, or 1 µg or more and 100 µg or less, for example.

The frequency of administration may be a single administration of the above-mentioned dose, or plural administrations of the above-mentioned dose carried out once or plural times every week, two weeks, three weeks, four weeks, month, two months, three months, six months, or the like. Alternatively, the administration may be carried out at two or more locations in one administration.

### <<Other embodiments>>

In one embodiment, the present invention provides a method for preventing or treating at least one disease selected from the group consisting of cancers, infectious diseases and immune diseases, the method including: administering an effective amount of the above-mentioned pharmaceutical composition to a patient or diseased animal.

Examples of the infectious diseases include those exemplified in "antigens derived from infectious diseases" under "antigens" above.

Furthermore, the term "effective amount" as used herein includes an amount effective for prevention or treatment, that is, an amount suitable to prevent or treat the onset of the above-mentioned diseases.

In one embodiment, the present invention provides a composition for preventing or treating at least one disease selected from the group consisting of cancers, infectious diseases and immune diseases, the composition containing the above-mentioned complex of the medicinal ingredient and the hyaluronic acid derivative.

In one embodiment, the present invention provides the use of the above-mentioned complex of the medicinal ingredient and the hyaluronic acid derivative to prepare a pharmaceutical composition.

The pharmaceutical composition is preferably an infectious disease vaccine, a cancer vaccine, or a pharmaceutical composition for immune diseases.

In one embodiment, the present invention further provides a pharmaceutical composition containing: a composition including a hyaluronic acid derivative and an antigen; and a lymphocyte expressing an immunoreceptor against the antigen. The use of the composition containing a hyaluronic acid derivative and an antigen in combination with the antigen-specific lymphocyte makes it possible to exhibit antitumor effects more strongly, more efficiently, more sustainably, and/or over a wider range.

A pharmaceutical composition containing a lymphocyte expressing an immunoreceptor against the antigen is provided to be administered together with the above-mentioned composition containing the hyaluronic acid derivative and the antigen.

A pharmaceutical composition characterized by being composed of a combination of the composition containing the hyaluronic acid derivative and the antigen with an antigen-specific lymphocyte may also be in the form of a kit. Examples of the pharmaceutical composition include a cancer treatment kit including: the composition containing the hyaluronic acid derivative and the antigen; and the antigen-specific lymphocyte.

In a preferable embodiment of the present invention, the composition containing the hyaluronic acid derivative and the antigen is preferably administered before administering the antigen-specific lymphocyte. In a preferable embodiment of the present invention, when a procedure in which the composition containing the hyaluronic acid derivative and the antigen is administered once, followed by injecting the antigen-specific lymphocyte once is considered as one administration cycle, the composition containing the hyaluronic acid derivative and the antigen is preferably administered at least once after one or two administration cycles are carried out.

In a preferred embodiment of the present invention, the interval between the first administration and the second administration of the composition containing the hyaluronic acid derivative and the antigen is, for example, 1 day or more, 2 days or more, 3 days or more, 4 days or more, 5 days or more, 6 days or more, 7 days or more, 8 days or more, 9 days or more, 10 days or more, 11 days or more, 12 days or more, 13 days or more, or 14 days or more. In addition, the interval may be, for example, 28 days or less, 24 days or less, 21 days or less, 17 days or less, 14 days or less, 13 days or less, 12 days or less, 11 days or less, 10 days or less, 9 days or less, 8 days or less, 7 days or less, 6 days or less, 5 days or less, 4 days or less, 3 days or less, or 2 days or less.

In one embodiment of the present invention, the composition containing the hyaluronic acid derivative and the antigen may be administered in combination with one or more antibodies used in cancer treatment.

The antibodies are, for example, one or more antibodies that inhibit immunosuppressive signals by a tumor or activate co-stimulatory signals of immune cells, preferably an antibody that inhibits immunosuppressive signals by a tumor or an antibody that activates co-stimulatory signals of immune cells. Specifically, the antibodies are one or more antibodies selected from the group consisting of anti-CTLA4 antibody, anti-PD1 antibody, anti-PDL1 antibody, anti-OX40, and anti-4-1BB antibody.

The dosage of the composition containing the hyaluronic acid derivative and the antigen when administered in combination is, for example, 0.01 to 100 mg/administration, preferably 0.1 to 50 mg/ administration, and more preferably 0.1 to 20 mg/ administration. The dosage of the antibody is, for example, 0.01 to 200 mg/kg body weight, preferably 0.1 to 100 mg/kg body weight, and more preferably 1 to 40 mg/kg body weight.

The antibody may be administered at the same timing as or different timings from the composition containing the hyaluronic acid derivative and the antigen (including the case in which the antibody is included in a preparation). When administered at different timings, it is preferable, for example, to administer one within one minute to 24 hours, preferably one minute to five hours after administering the other.

The composition containing the hyaluronic acid derivative and the antigen may be administered in combination with both the above-mentioned adjuvant and antibody. In that case, the dosage of the composition containing the hyaluronic acid derivative and the antigen is, for example, 0.01 to 100 mg/administration, preferably 0.1 to 50 mg/ administration, and more preferably 0.1 to 20 mg/ administration, the dosage of the adjuvant is, for example, 0.01 to 100 mg/kg body weight, preferably 0.1 to 50 mg/kg body weight, and more preferably 0.1 to 10 mg/kg body weight, and the dosage of the antibody is, for example, 0.01 to 200 mg/kg body weight, preferably 0.1 to 100 mg/kg body weight, and more preferably 1 to 40 mg/kg body weight. The adjuvant and antibody may be administered at the same timing as or different timings from the composition containing the hyaluronic acid derivative and the antigen (including the case in which the adjuvant is included in a preparation). When administered at different timings, it is preferable that, for example, a vaccine preparation, adjuvant, and antibody all be administered within one minute to 24 hours, preferably one minute to five hours.

In one embodiment, the present invention provides a composition for improving, promoting, or enhancing uptake of a medicinal ingredient into immune cells, the composition containing the above-mentioned complex of the medicinal ingredient and the hyaluronic acid derivative.

In one embodiment, the present invention provides a composition for improving, promoting, or enhancing the activity of immune cells, the composition containing the above-mentioned complex of the medicinal ingredient and the hyaluronic acid derivative.

In one embodiment, the present invention provides a composition for improving, promoting, maintaining or enhancing the expression of co-stimulatory molecules (particularly CD80 and CD86) of DC (particularly cDC1), the composition containing the above-mentioned complex of the medicinal ingredient and the hyaluronic acid derivative.

In one embodiment, the present invention provides a method for improving, promoting, or enhancing uptake of a medicinal ingredient into immune cells in vivo or in vitro, the method including administering the composition containing the complex of the medicinal ingredient and the hyaluronic acid derivative.

In one embodiment, the present invention provides a method for improving, promoting, or enhancing the activity of immune cells in vivo or in vitro, the method including administering the composition containing the complex of the medicinal ingredient and the hyaluronic acid derivative.

In one embodiment, the present invention provides a method for improving, promoting, maintaining or enhancing the expression of co-stimulatory molecules (particularly CD80 and CD86) of DC (particularly cDC1) in vivo or in vitro, the method including administering the complex of the medicinal ingredient and the hyaluronic acid derivative.

### [Examples]

Hereinafter, the present invention will be explained in detail with reference to examples, but these are not intended to limit the scope of the present invention.

### <Synthesis of hyaluronic acid derivative>

### [Synthesis Example 1-1]

A hyaluronic acid derivative was prepared by the following steps 1 to 3.

### 1. Step 1

### (Synthesis of cholesteryl 6-aminohexylcarbamate hydrochloride)

Cholesteryl 6-aminohexylcarbamate hydrochloride (Chol hydrochloride) was synthesized by the following step 1-1 and then step 1-2.

### (1) Step 1-1

Triethylamine (TEA, 1.05 mL) was added to a solution of cholesteryl chloroformate (3.37 g, 7.5 mmol) in anhydrous dichloromethane (20 mL) and stirred under an argon atmosphere. 6-(t-Butoxycarbonyl)amino-1-aminohexane (1.12 mL, 5 mmol) was added dropwise thereto under ice cooling, and the mixture was stirred for 30 minutes under ice cooling, followed by warming the mixture to room temperature (approximately 25°C) and then stirring the mixture overnight. The reaction mixture was washed with ultrapure water and saturated saline and dried over anhydrous magnesium sulfate, and then the solvent was distilled off under reduced pressure. The resulting residue was purified by silica gel column chromatography (eluent: ethyl acetate:n-hexane=1:4), the fractions containing the target product were combined, and then the solvent was distilled off under reduced pressure.

### (2) Step 1-2

The obtained residue was dissolved in ethyl acetate (40 mL), and then a 4N hydrochloric acid/ethyl acetate solution (40 mL) was added thereto, followed by stirring the mixture at room temperature (about 25°C) overnight. The resulting precipitate was collected by centrifugation. The obtained solid was washed four times with ethyl acetate and then dried under reduced pressure to obtain 1.2 g of cholesteryl 6-aminohexylcarbamate hydrochloride (Chol hydrochloride).

### 2. Step 2

### (Preparation of tetrabutylammonium (TBA) salt of hyaluronic acid)

A TBA salt of hyaluronic acid (HA) (HA-TBA) was prepared by the following step 2-1 and then step 2-2.

### (1) Step 2-1

DOWEX (registered trademark) 50WX-8-400 (manufactured by Aldrich) was suspended in ultrapure water, and the resin was washed with ultrapure water about three times by decantation. An aqueous solution containing 40 wt% of tetrabutylammonium hydroxide (TBA-OH) (manufactured by Aldrich) was added thereto in a molar amount equivalent to about 1.5 times as much as the cation exchange capacity of the resin, and the mixture was stirred at room temperature (approximately 25°C) for 30 minutes. After removing an excess TBA-OH solution by decantation, the resultant was further washed with excess ultrapure water to obtain a TBA-chlorinated cation-exchange resin.

### (2) Step 2-2

A raw material sodium hyaluronate (HA-Na) having a weight-average molecular weight (absolute molecular weight) of 10,000 (10 kDa) was dissolved in ultrapure water at a concentration of 15 mg/mL. The suspension of the TBA-chlorinated cation-exchange resin in "(1) Step 2-1" was added thereto in a molar amount equivalent to five times as much as the molar amount of the HA unit (unit molecular weight: 401.3) based on the ion exchange capacity of the resin. After stirring the mixture at room temperature (approximately 25°C) for 15 minutes, filtration was carried out using a 0.45 µm filter, and the filtrate was freeze-dried to obtain a TBA salt of hyaluronic acid (HA-TBA) as a white solid.

### 3. Step 3

An anhydrous DMSO solution (10 mg/mL) of the HA-TBA prepared in "2. (2) Step 2-2" was prepared. Thereafter, the Chol hydrochloride was added thereto such that the molar ratio of the added Chol hydrochloride to the disaccharide repeating unit (HA unit) present in the HA-TBA synthesized in "1. Step 1" was 44/100. Next, 4-(4,6-dimethoxy-1,3,5-triazin-2-yl)-4-methylmorpholinium chloride (DMT-MM) was added thereto such that the molar ratio of the added DMT-MM to the HA unit was 48/100, followed by stirring the mixture at room temperature (approximately 25°C) overnight. The reaction solution was subjected to dialysis with a 0.3M ammonia acetate/DMSO solution, 0.15M aqueous NaCl solution, and ultrapure water in this order using a SpectraPor 7 dialysis membrane (manufactured by Spectrum Laboratories, molecular weight cutoff (MWCO): 3,500). The obtained dialysate was freeze-dried to obtain the target product (HA-C₆-Chol) as a white solid.

The cholesterol introduction rate in the obtained white solid was calculated by the following method.

A 0.02N DCl DMSO-d₆/D₂O mixture liquid (2N DCl D₂O:DMSO-d₆=1:99) was used as the measurement solvent, and JNM-ECS400 (manufactured by JEOL Ltd.) was used as the NMR device to carry out the measurement at 85°C, thereby obtaining a ¹H-NMR spectrum of the white solid. In the obtained ¹H-NMR spectrum, a peak derived from the acetyl group of N-acetyl-D-glucosamine (COCH₃, 1.6 ppm or more and 2.0 ppm or less, 3H) and a peak derived from the methyl group in the cholesteryl group (CH₃, 0.7 ppm, 3H) were confirmed, and the cholesterol introduction rate was 44%.

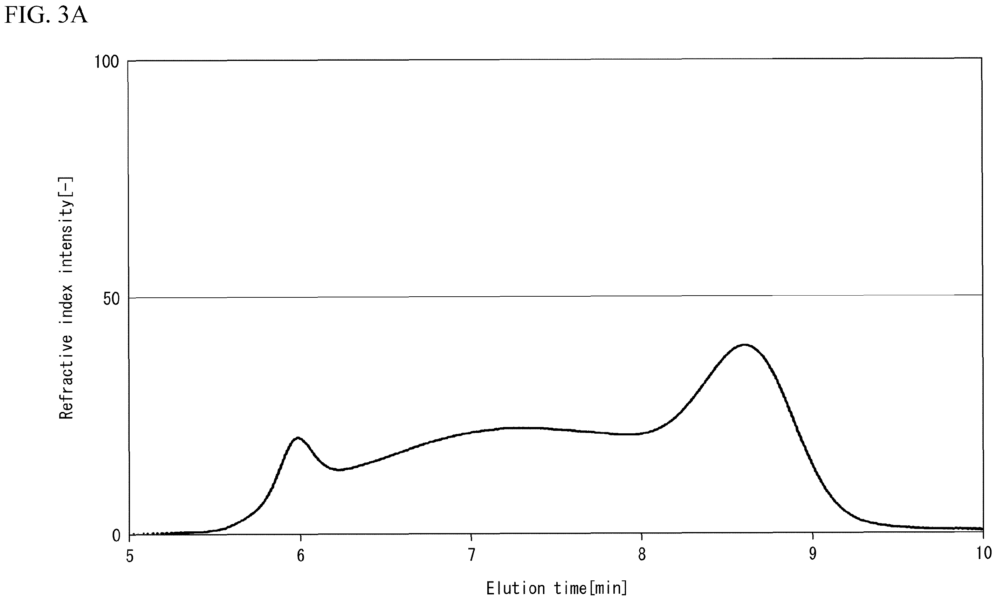

A freeze-dried hyaluronic acid derivative (10k HA-C₆-Chol-44%) was dissolved in injection water at 1 mg/mL and subjected to a measurement by gel permeation chromatography under the following conditions. Fig. 3A is the resulting chromatogram.

### (Measurement conditions)

Device: HLC8420-GPC (manufactured by TOSOH CORPORATION)
Column: G4000SWXL (manufactured by TOSOH CORPORATION, particle size 8 µm, inner diameter 7.8 mm, length 30 cm, product number:8542)
Eluent: 10 mM phosphate buffer (pH 7.4)
Flow rate: 1 mL/min
Injection amount: 50 µL
Detector: RI
Temperature: 30°C

### [Synthesis Example 1-2]

A hyaluronic acid derivative was synthesized by the same procedure as in Synthesis Example 1-1.

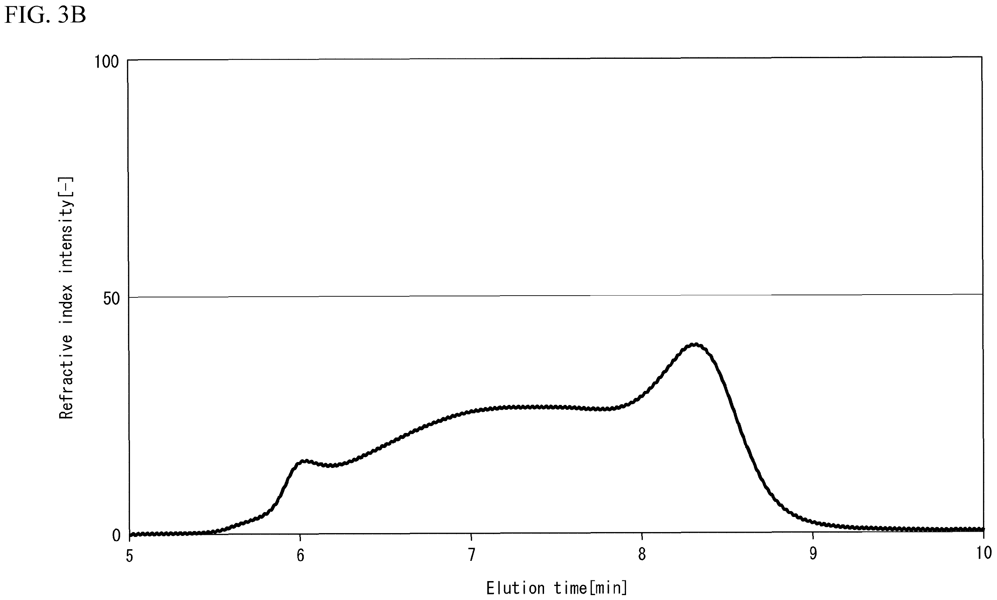

In the ¹H-NMR spectrum of the resultant product, a peak derived from the acetyl group of N-acetyl-D-glucosamine (COCH₃, 1.6 ppm or more and 2.0 ppm or less, 3H), and a peak derived from the methyl group in the cholesteryl group (CH₃, 0.7 ppm, 3H) were confirmed, and the cholesterol introduction rate was 44%. Measurement was carried out by gel permeation chromatography under the conditions shown in Synthesis Example 1-1 mentioned above, and the obtained chromatogram is shown in FIG. 3B.

### [Synthesis Example 1-3]

A hyaluronic acid derivative was synthesized by the same procedure as in Synthesis Example 1-1.

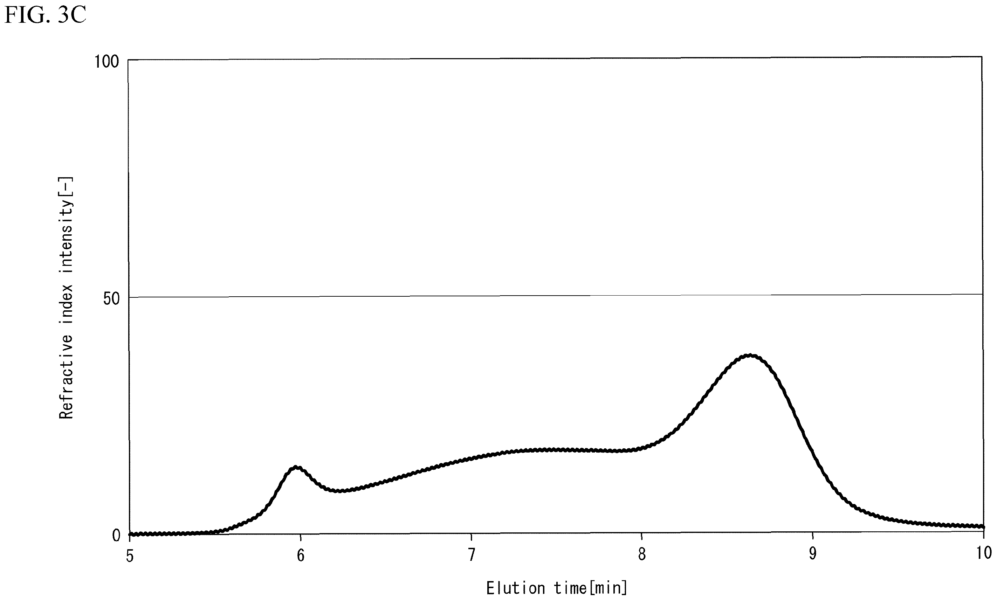

In the ¹H-NMR spectrum of the resultant product, a peak derived from the acetyl group of N-acetyl-D-glucosamine (COCH₃, 1.6 ppm or more and 2.0 ppm or less, 3H), and a peak derived from the methyl group in the cholesteryl group (CH₃, 0.7 ppm, 3H) were confirmed, and the cholesterol introduction rate was 44%. Measurement was carried out by gel permeation chromatography under the conditions shown in Synthesis Example 1-1 mentioned above, and the obtained chromatogram is shown in FIG. 3C.

### [Example 1-1]

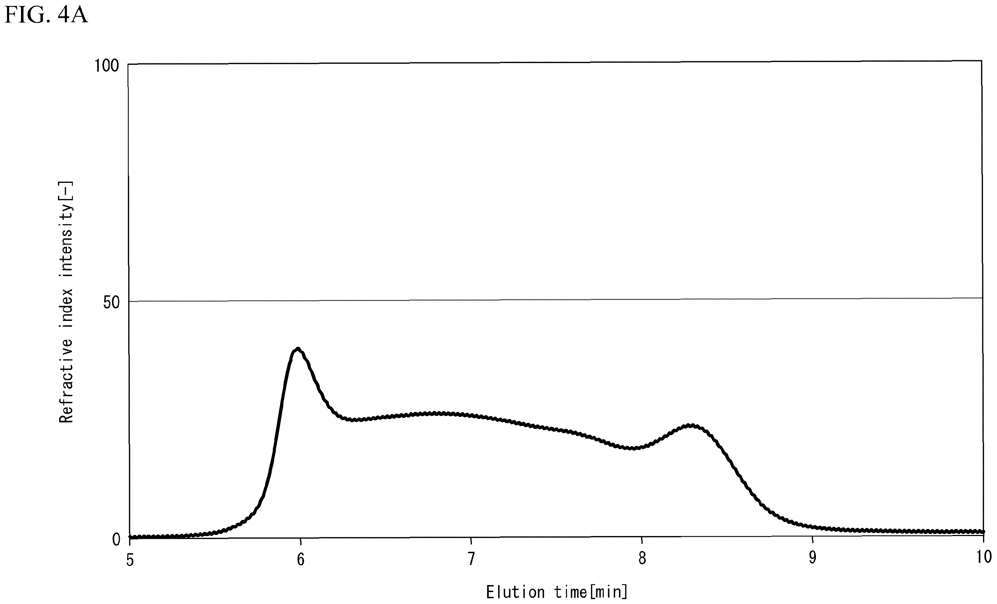

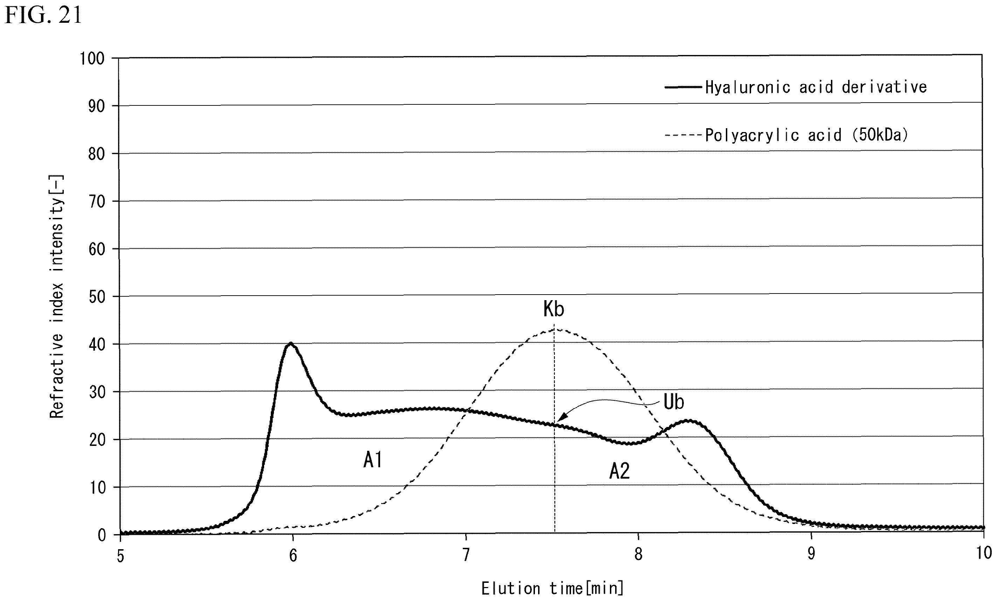

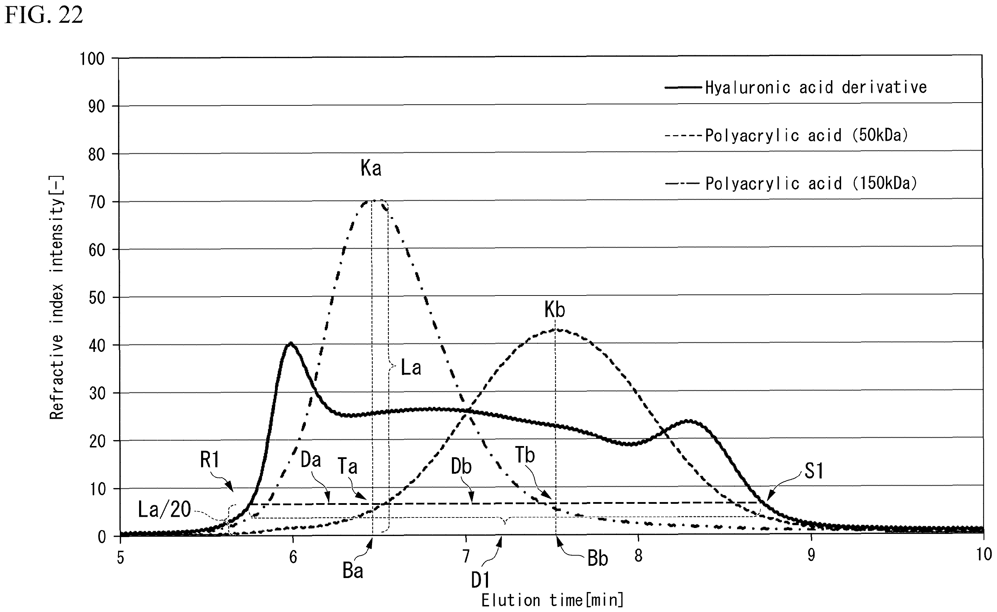

The hyaluronic acid derivative obtained in Synthesis Example 1-1 was fractionated as follows. The freeze-dried hyaluronic acid derivative (10k HA-C₆-Chol-44%) was dissolved in injection water at 5 mg/mL, transferred to a dialysis cassette (Float-A-Lyzer G2, MWCO: 300,000, Ieda Boeki Co., Ltd.), and subjected to dialysis nine times with 10 mM phosphate buffer pH 7.4. The hyaluronic acid derivative in the obtained internal dialysate was subjected to a measurement by gel permeation chromatography. Fig. 4A is the resulting chromatogram. Fig. 21 and Fig. 22 show chromatograms of the hyaluronic acid derivative obtained in Example 1-1 and polyacrylic acids as standard substances, the chromatograms being superimposed in order to explain the method of calculating the below-mentioned area ratio A1/A2 and distance ratio Da/Db.

The filtrate was concentrated to a desired concentration using an ultraconcentrator (Vivaspin 20, MWCO: 10,000, Sartorius).

### [Example 1-2]

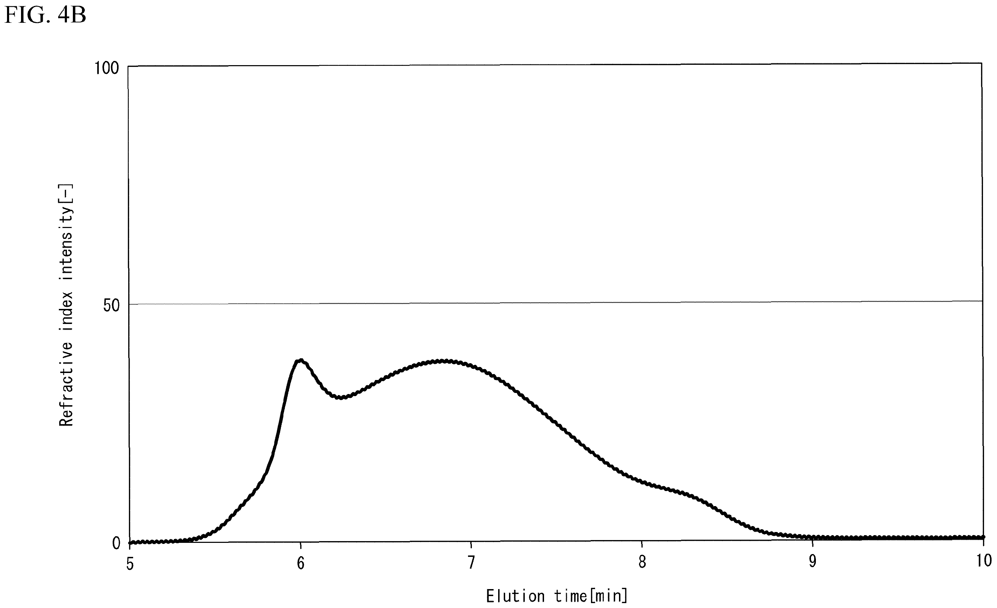

The hyaluronic acid derivative obtained in Synthesis Example 1-2 was fractionated using the same procedure as in Example 1-1. The hyaluronic acid derivative in the obtained internal dialysate was subjected to measurement by gel permeation chromatography. Fig. 4B is the resulting chromatogram.

### [Example 1-3]

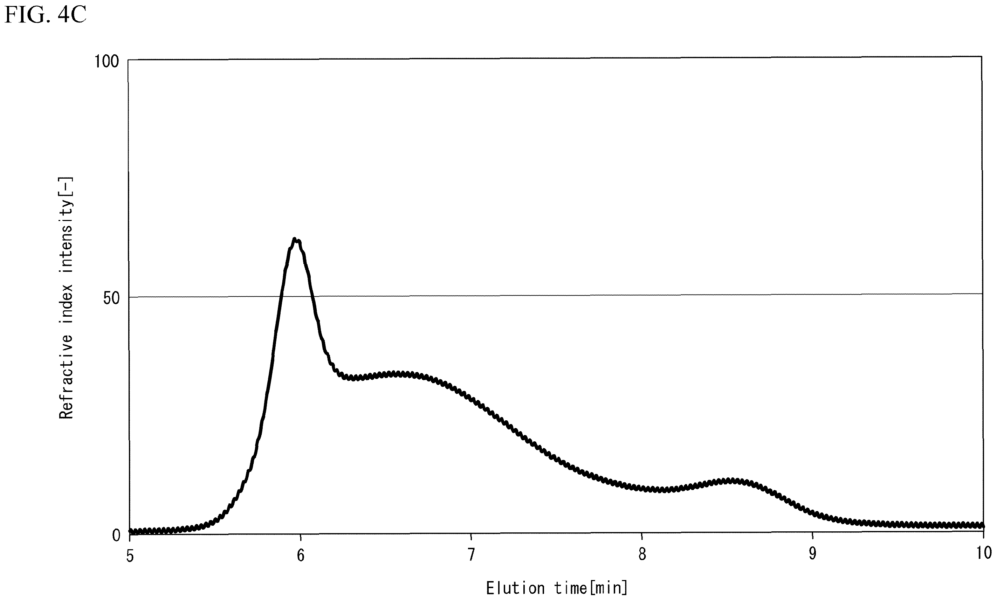

The hyaluronic acid derivative obtained in Synthesis Example 1-3 was fractionated using the same procedure as in Example 1-1. The hyaluronic acid derivative in the obtained internal dialysate was subjected to measurement by gel permeation chromatography. Fig. 4C is the resulting chromatogram.

### [Comparative Example 1-1]

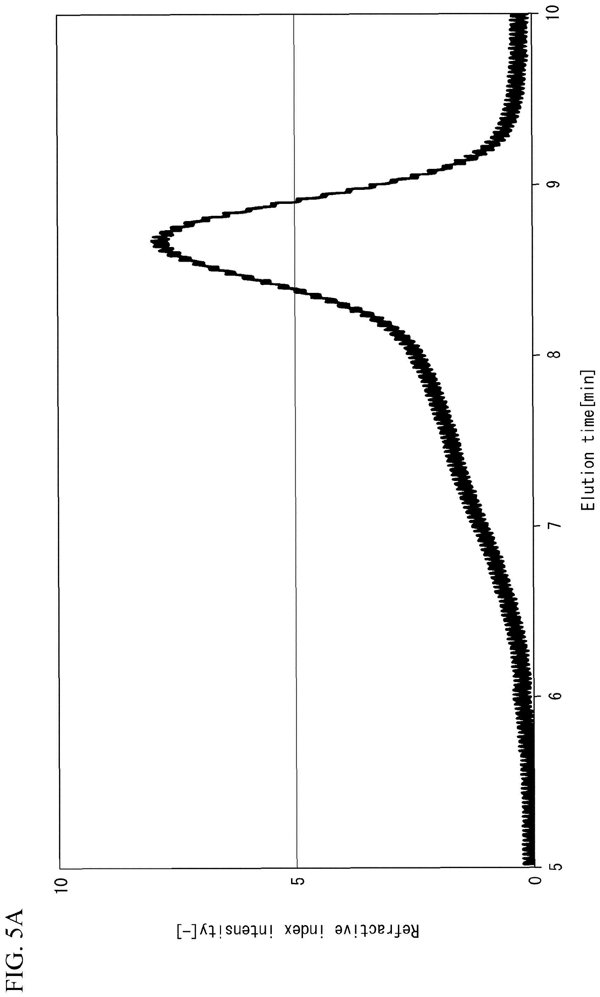

The hyaluronic acid derivative obtained in Synthesis Example 1-1 was fractionated as follows. The freeze-dried hyaluronic acid derivative (10k HA-C₆-Chol-44%) was dissolved in injection water at 10 mg/mL, and diluted with 100 mM phosphate buffer to obtain 10 mM phosphate buffer (pH 7.4). Then, the resultant was filtered using an ultrafiltration device (Vivaspin (registered trademark) 20, MWCO: 300,000, Sartorius), and the hyaluronic acid derivative in the obtained filtrate was subjected to measurement by gel permeation chromatography. Fig. 5A is the resulting chromatogram. The filtrate was concentrated to a desired concentration using an ultraconcentrator (Vivaspin 20, MWCO: 10,000, Sartorius).

### [Comparative Example 1-2]

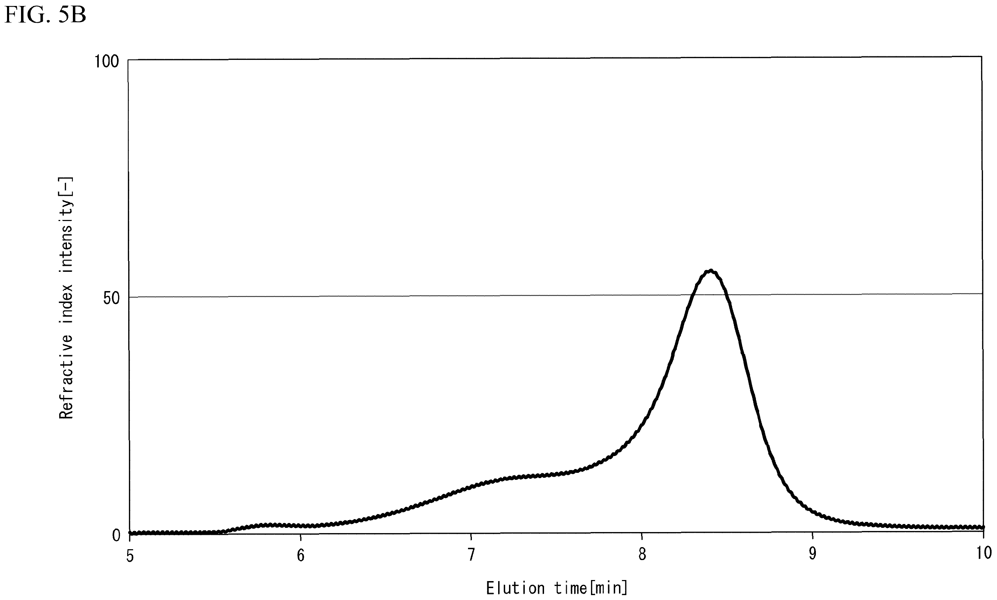

The hyaluronic acid derivative obtained in Synthesis Example 1-2 was fractionated using the same procedure as in Comparative Example 1-1. The hyaluronic acid derivative in the obtained filtrate was concentrated to a desired concentration using an ultraconcentrator (Vivaspin 20, MWCO: 10,000, Sartorius). Thereafter, the resultant was diluted with injection water to a concentration of 1 mg/mL, and subjected to measurement by gel permeation chromatography. Figure 5B is the resulting chromatogram.

### [Comparative Example 1-3]

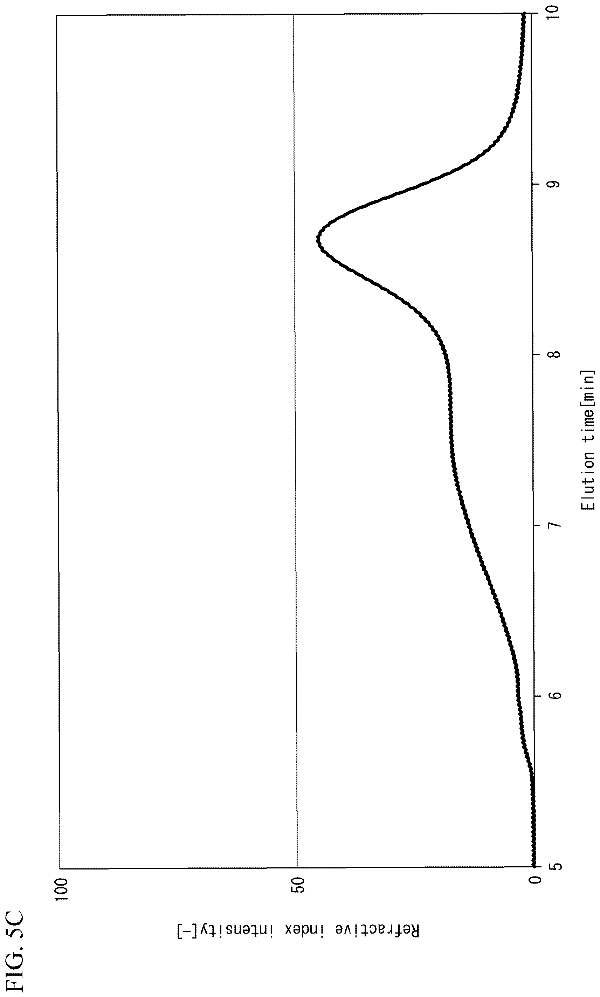

The hyaluronic acid derivative obtained in Synthesis Example 1-3 was fractionated using the same procedure as in Comparative Example 1-2. The hyaluronic acid derivative in the obtained filtrate was concentrated to a desired concentration using an ultraconcentrator (Vivaspin 20, MWCO: 10,000, Sartorius). Thereafter, the resultant was diluted with injection water to a concentration of 1 mg/mL, and subjected to measurement by gel permeation chromatography. Figure 5C is the resulting chromatogram.

### [Comparative Example 1-4]

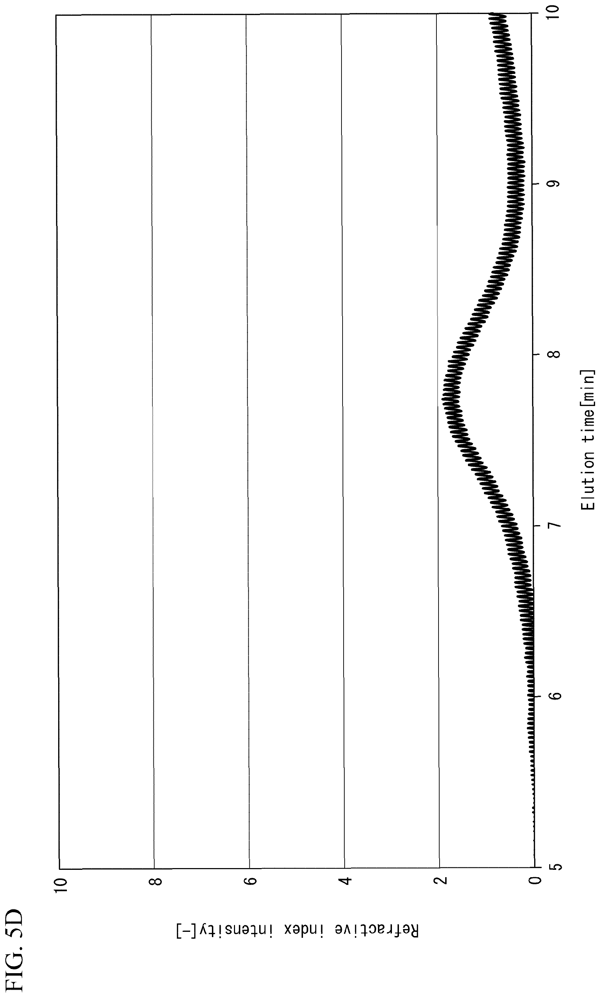

A freeze-dried product of cholesteryl-modified pullulan (CHP: manufactured by NOF CORPORATION under the product number of CHP-80T) was dissolved in injection water at 1 mg/mL, and subjected to a measurement by gel permeation chromatography. Figure 5D is the resulting chromatogram.

### [Evaluation of particle size distribution of the hyaluronic acid derivative: area ratio A1/A2 and distance ratio Da/Db]

The area ratio A1/A2 and distance ratio Da/Db were calculated from gel permeation chromatograms of the hyaluronic acid derivative obtained in Example 1-1 and polyacrylic acids as standard substances.

ORDER No. PSS-Paa 50k (sodium polyacrylate) manufactured by Polymer Standards Service-USA was used as the polyacrylic acid (50 kDa) as a standard substance.

ORDER No. PSS-Paa 150k (sodium polyacrylate) manufactured by Polymer Standards Service-USA was used as the polyacrylic acid (150 kDa) as a standard substance.

Powders of the polyacrylic acids as standard substances were dissolved in injection water such that the concentration thereof became 2 mg/mL. All polyacrylic acids used as standard substances described hereinafter were manufactured by Polymer Standards Service-USA (sodium polyacrylates).

The areas A1 and A2 were calculated from gel permeation chromatograms shown in Fig. 21 by the following method to calculate the area ratio A1/A2.

(i) First, an intersection point Ub was determined by: a perpendicular line drawn from the maximum point Kb of the refractive index intensity on a chromatogram of a 50 kDa polyacrylic acid serving as a standard substance to the baseline B; and the chromatogram of the hyaluronic acid derivative, and an intersection point Bb was determined by: the perpendicular line drawn from the maximum point Kb of the refractive index intensity to the baseline B; and the baseline. The elution time at the maximum point Kb of the refractive index intensity was 7.41 minutes.
(ii) Next, the area A2 surrounded by: a curve from the intersection point Ub to the end point on the chromatogram of the hyaluronic acid derivative; a perpendicular line drawn from the maximum point Kb of the refractive index intensity to the baseline B; and the baseline was determined, and the area A1 surrounded by: a curve from the starting point to the intersection point Ub on the chromatogram of the hyaluronic acid derivative; the perpendicular line drawn from the maximum point Kb of the refractive index intensity to the baseline B; and the baseline was determined.

The distances Da and Db were calculated from gel permeation chromatograms shown in Fig. 22 by the following method to calculate the distance ratio Da/Db.

(i) First, an intersection point Ba was determined by: a perpendicular line drawn from the maximum point Ka of the refractive index intensity on the chromatogram of a 150 kDa polyacrylic acid serving as a standard substance to the baseline B; and the baseline, and the length La between the maximum point Ka of the refractive index intensity and the intersection point Ba was determined. The elution time at the maximum point Ka of the refractive index intensity was 6.46 minutes.
(ii) Then, among two points at which the refractive index intensity on the chromatogram reached La/20, one point at which the elution time was earlier was determined as the point R1, and the other point at which the elution time was later was determined as the point S1. The elution time at the point R1 was 5.74 minutes and the elution time at the point S1 was 7.68 minutes.
(iii) Then, an intersection point Bb was determined by: a perpendicular line drawn from the maximum point Kb of the refractive index intensity on a chromatogram of a 50 kDa polyacrylic acid serving as a standard substance to the baseline B; and the baseline. The elution time at the maximum point Kb of the refractive index intensity was as described above.
(iv) Then, an intersection point Ta was determined by: the straight line D1 that connected the point R1 and the point S1; and a perpendicular line drawn from the maximum point Ka of the refractive index intensity to the baseline B, and an intersection point Tb was determined by the perpendicular line drawn from the maximum point Kb of the refractive index intensity to the baseline B; and the straight line D1.
(v) Finally, the distance Da between the point R1 and the intersection point Ta was determined and the distance Db between the intersection point Ta and the intersection point Tb was determined.

The area ratio A1/A2 and distance ratio Da/Db were calculated from gel permeation chromatograms of each hyaluronic acid derivative obtained in Synthesis Examples 1-1 to 1-3, Examples 1-2 to 1-3 and Comparative Examples 1-1 to 1-4 and polyacrylic acids as standard substances in the same manner as the hyaluronic acid derivative obtained in Example 1-1.

The calculated area ratio A1/A2 and distance ratio Da/Db are shown in Table 1 below. In Table 1, the distance ratio Da/Db of cholesteryl-modified pullulan (CHP) of Comparative Example 3 could not be calculated because the RI values were small.

**[Table 1]**

| | A1/A2 | Da/Db |
|---|---|---|
| Synthesis Example 1-1 | 0.51 | 0.68 |
| Synthesis Example 1-2 | 0.89 | 0.69 |
| Synthesis Example 1-3 | 0.60 | 0.71 |
| Example 1-1 | 1.79 | 0.76 |
| Example 1-2 | 4.24 | 0.91 |
| Example 1-3 | 3.87 | 0.92 |
| Comparative Example 1-1 | 0.31 | 0.22 |
| Comparative Example 1-2 | 0.245 | 0.021 |
| Comparative Example 1-3 | 0.350 | 0.28 |
| Comparative Example 1-4 | 0.343 | - |

As shown in Table 1, the area ratio A1/A2 of the hyaluronic acid derivatives of Examples 1-1 to 1-3 was higher than that of the hyaluronic acid derivatives of Synthesis Examples 1-1 to 1-3 before purification.

### <Production of pharmaceutical composition>

### [Example 2-1]

The hyaluronic acid derivative obtained in Example 1-1 was diluted with 10 mM phosphate buffer pH 7.4 to a concentration of 5 mg/mL.

In contrast, a fluorescent-labeled peptide was dissolved in dimethyl sulfoxide (DMSO) in another container such that the concentration thereof became 50 mg/mL. As the fluorescently labeled peptide, a fluorescent-labeled peptide manufactured by Biologica, Inc., and having the following amino acid sequence bonded with fluorescein at the N-terminus thereof was used.

NDHIAYFLYQILRGLQYIHSANVLHRDLKPSNLLLNT (SEQ ID No. 1)

The aqueous solution of the hyaluronic acid derivative and the DMSO solution of the fluorescent-labeled peptide were mixed at a volume ratio of 100:1 at room temperature (about 25°C), and stirred at room temperature (about 25°C) for 24 hours to obtain a composition containing a complex of the fluorescent-labeled peptide and the hyaluronic acid derivative.

Thereafter, a solid purified sucrose (manufactured by FUJIFILM Wako Pure Chemical Corporation, for production only, under the product number of 198-18385) was added to the composition containing the complex of the fluorescent-labeled peptide and the hyaluronic acid derivative such that the amount of sucrose relative to the total mass of the composition and the sucrose became 10% by mass, and stirred at room temperature (approximately 25°C) for one hour or more. After confirming that the sucrose was sufficiently dissolved, the resultant was diluted with 10 mM phosphate buffer pH 7.4 containing 10% by mass of sucrose such that the concentration of the peptide became approximately 0.3 mg/mL, followed by subjecting the resultant to sterilization filtration using a 0.2 µm PES (polyethersulfone) (manufactured by Pall Corporation, acrodisc syringe filter, 25 mmΦ) to obtain a pharmaceutical composition containing a complex of the fluorescent-labeled peptide and the hyaluronic acid derivative. The amount of the fluorescent-labeled peptide relative to 100 parts by mass of the pharmaceutical composition was 0.0317 parts by mass.

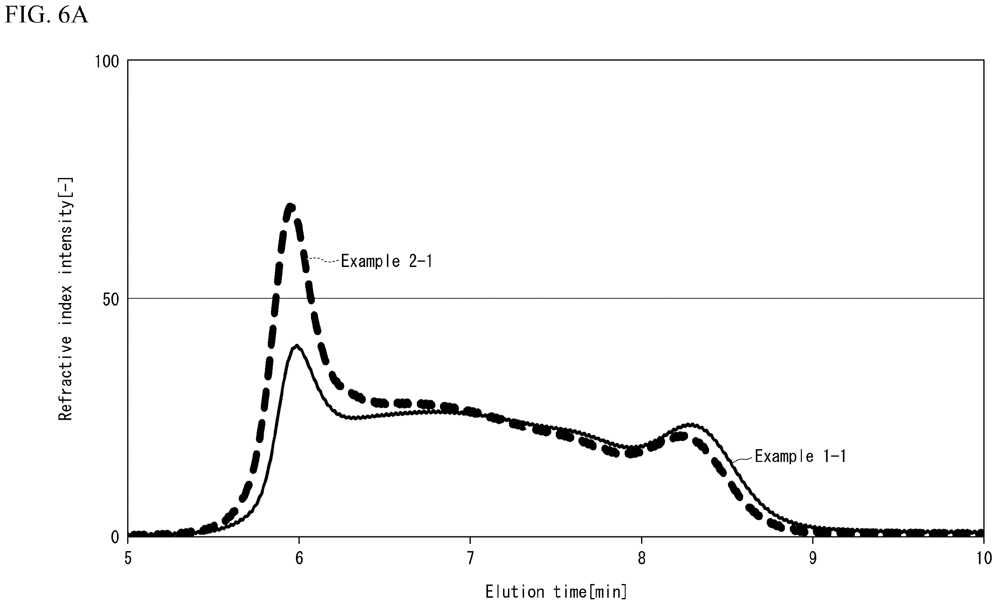

The resultant was diluted by the method described in Synthesis Example 1-1 such that the concentration of the hyaluronic acid derivative became 1 mg/mL and then subjected to measurement by gel permeation chromatography, thereby confirming no significant change in the chromatogram of the hyaluronic acid derivative between before and after encapsulating the peptide (Fig. 6A). At this time, it was also confirmed that the concentration of the peptide was 0.1 mg/mL, and 100% of the charged peptide was encapsulated.

### [Example 2-2]

A pharmaceutical composition containing a complex of the fluorescent-labeled peptide and the hyaluronic acid derivative was obtained using the hyaluronic acid derivative obtained in Example 1-2 by the same procedure as in Example 2-1. The amount of the fluorescent-labeled peptide relative to 100 parts by mass of the pharmaceutical composition was 0.0478 parts by mass.

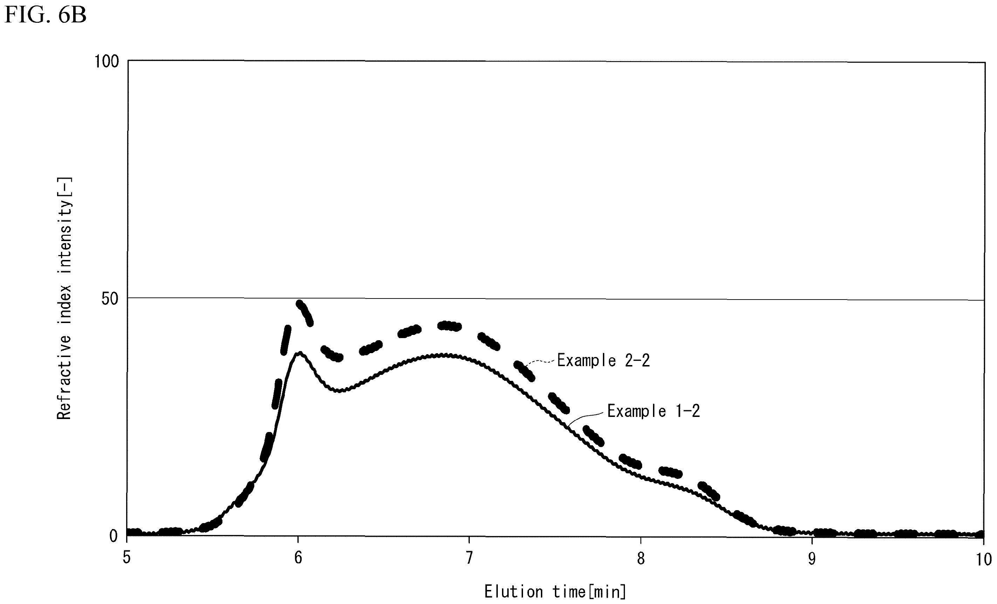

When the resultant was subjected to a measurement by gel permeation chromatography by the method described in Synthesis Example 1-1, no significant change was confirmed in the chromatogram of the hyaluronic acid derivative between before and after encapsulating the peptide (Fig. 6B).

### [Example 2-3]

A pharmaceutical composition containing a complex of the fluorescent-labeled peptide and the hyaluronic acid derivative was obtained using the hyaluronic acid derivative obtained in Example 1-3 by the same procedure as in Example 2-1. The amount of the fluorescent-labeled peptide relative to 100 parts by mass of the pharmaceutical composition was 0.0303 parts by mass.

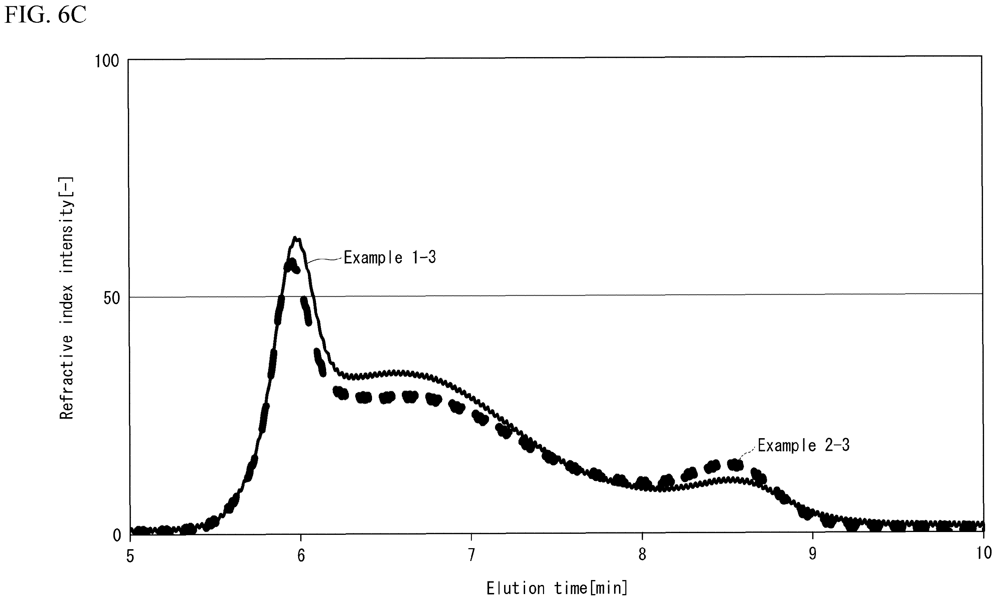

When the resultant was subjected to a measurement by gel permeation chromatography by the method described in Synthesis Example 1-1, no significant change was confirmed in the chromatogram of the hyaluronic acid derivative between before and after encapsulating the peptide (Fig. 6C).

### [Comparative Example 2-1]

The hyaluronic acid derivative obtained in Comparative Example 1-1 was diluted with 10 mM phosphate buffer pH 7.4 to a concentration of 5 mg/mL.

**In** contrast, a fluorescent-labeled peptide was dissolved in DMSO in another container such that the concentration thereof became 50 mg/mL.

The aqueous solution of the hyaluronic acid derivative and the dimethyl sulfoxide solution of the fluorescent-labeled peptide were mixed at a volume ratio of 100:1 at room temperature (about 25°C), and stirred at room temperature (about 25°C) for 24 hours to obtain a composition containing a complex of the fluorescent-labeled peptide and the hyaluronic acid derivative.

Thereafter, a solid purified sucrose (manufactured by FUJIFILM Wako Pure Chemical Corporation, for production only, under the product number of 198-18385) was added to the composition containing the complex of the fluorescent-labeled peptide and the hyaluronic acid derivative such that the amount of sucrose relative to the total mass of the composition and the sucrose became 10% by mass, and stirred at room temperature (approximately 25°C) for one hour or more. After confirming that the sucrose was sufficiently dissolved, the resultant was diluted with 10 mM phosphate buffer pH 7.4 containing 10% by mass of sucrose such that the concentration of the peptide became 0.3 mg/mL, followed by subjecting the resultant to sterilization filtration using a 0.2 µm PES (polyethersulfone) (manufactured by Pall Corporation, acrodisc syringe filter, 25 mmΦ) to obtain a pharmaceutical composition containing a complex of the fluorescent-labeled peptide and the hyaluronic acid derivative. The amount of the fluorescent-labeled peptide relative to 100 parts by mass of the pharmaceutical composition was 0.0308 parts by mass.

### [Comparative Example 2-2]

A composition containing a complex of the fluorescent-labeled peptide and the hyaluronic acid derivative was obtained using the hyaluronic acid derivative obtained in Comparative Example 1-2 by the same method as Comparative Example 2-1. The amount of the fluorescent-labeled peptide relative to 100 parts by mass of the pharmaceutical composition was 0.0332 parts by mass.

### [Comparative Example 2-3]

A composition containing a complex of the fluorescent-labeled peptide and the hyaluronic acid derivative was obtained using the hyaluronic acid derivative obtained in Comparative Example 1-3 by the same method as Comparative Example 2-1. The amount of the fluorescent-labeled peptide relative to 100 parts by mass of the pharmaceutical composition was 0.0348 parts by mass.

### [Comparative Example 3]

A freeze-dried product of CHP (manufactured by NOF CORPORATION under the product number of CHP-80T) was dissolved in phosphate-buffered saline pH 7.4 containing 6M urea such that the concentration of the freeze-dried product became 10 mg/mL.

**In** contrast, a fluorescent-labeled peptide was dissolved in dimethyl sulfoxide in another container such that the concentration thereof became 50 mg/mL.

The aqueous solution of the CHP containing 6M urea and the dimethyl sulfoxide solution of the fluorescent-labeled peptide were mixed at a volume ratio of 100:1 at room temperature (about 25°C), and stirred at room temperature (about 25°C) for 24 hours to obtain a composition containing a complex of the fluorescent-labeled peptide and the CHP.

Thereafter, the resultant was transferred to dialysis cassettes (manufactured by Thermo, Slide-A-Lyzer G2 Dialysis cassettes, MWCO: 3.5 K, 15 mL, No. 87724), and subjected to dialysis with phosphate-buffered saline containing 0.6 M urea, pH 7.4. Then, dialysis was carried out using phosphate-buffered saline containing 0.06 M urea, pH 7.4 and phosphate-buffered saline, pH 7.4 in this order. Then, the resultant was concentrated to a desired concentration using an ultraconcentrator (Vivaspin 20, MWCO: 10,000, Sartorius).

Finally, the resultant was subjected to sterilization filtration using a 0.2 µm PES (polyethersulfone) (manufactured by Pall Corporation, acrodisc syringe filter, 25 mmΦ), thereby obtaining a pharmaceutical composition containing a complex of the fluorescent-labeled peptide and the CHP. The amount of the fluorescent-labeled peptide relative to 100 parts by mass of the pharmaceutical composition was 0.0420 parts by mass.

### [Comparative Example 4-1]

The freeze-dried hyaluronic acid derivative obtained in Synthesis Example 1-1 was weighed and injection water was added thereto such that the concentration thereof became 5 mg/mL, followed by dissolving the freeze-dried hyaluronic acid derivative sufficiently by stirring overnight.

In contrast, a fluorescent-labeled peptide was dissolved in DMSO in another container such that the concentration thereof became 50 mg/mL.

The aqueous solution of the hyaluronic acid derivative and the dimethyl sulfoxide solution of the fluorescent-labeled peptide were mixed at a volume ratio of 100:1 at room temperature (about 25°C), and stirred at room temperature (about 25°C) for 24 hours to obtain a composition containing a complex of the fluorescent-labeled peptide and the hyaluronic acid derivative.

Thereafter, a solid purified sucrose (manufactured by FUJIFILM Wako Pure Chemical Corporation, for production only, under the product number of 198-18385) was added to the composition containing the complex of the fluorescent-labeled peptide and the hyaluronic acid derivative such that the amount of sucrose relative to the total mass of the composition and the sucrose became 10% by mass, and stirred at room temperature (approximately 25°C) for one hour or more. After confirming that the sucrose was sufficiently dissolved, the resultant was diluted with 25 mM phosphate buffer pH 7.4 containing 10% by mass of sucrose such that the concentration of the peptide became 0.3 mg/mL, followed by subjecting the resultant to sterilization filtration using a 0.2 µm PES (polyethersulfone) (manufactured by Pall Corporation, acrodisc syringe filter, 25 mm(D) to obtain a pharmaceutical composition containing a complex of the fluorescent-labeled peptide and the hyaluronic acid derivative. The amount of the fluorescent-labeled peptide relative to 100 parts by mass of the pharmaceutical composition was 0.0316 parts by mass.

### [Comparative Example 4-2]

A composition containing a complex of the fluorescent-labeled peptide and the hyaluronic acid derivative was obtained using the freeze-dried hyaluronic acid derivative obtained in Synthesis Example 1-2 by a similar procedure to Comparative Example 4-1.

Thereafter, a solid purified sucrose (manufactured by FUJIFILM Wako Pure Chemical Corporation, for production only, under the product number of 198-18385) was added to the composition containing the complex of the fluorescent-labeled peptide and the hyaluronic acid derivative such that the amount of sucrose relative to the total mass of the composition and the sucrose became 10% by mass, and stirred at room temperature (approximately 25°C) for one hour or more. After confirming that the sucrose was sufficiently dissolved, the resultant was diluted with 30 mM phosphate buffer pH 7.4 containing 10% by mass of sucrose such that the concentration of the peptide became 0.33 mg/mL, followed by subjecting the resultant to sterilization filtration using a 0.2 µm PES (polyethersulfone) (manufactured by Pall Corporation, acrodisc syringe filter, 25 mmΦ) to obtain a pharmaceutical composition containing a complex of the fluorescent-labeled peptide and the hyaluronic acid derivative. The amount of the fluorescent-labeled peptide relative to 100 parts by mass of the pharmaceutical composition was 0.0358 parts by mass.

### [Confirmation of concentration of fluorescent-labeled peptide]

A high-performance liquid chromatography measurement was carried out under the following conditions to confirm that the fluorescent-labeled peptide was complexed with the hyaluronic acid derivative to be solubilized in the pharmaceutical composition containing a complex of the fluorescent-labeled peptide and the hyaluronic acid derivative of each of Examples 2-1 to 2-3 and Comparative Examples 2-1 to 2-3, 3, 4-1, and 4-2, and provide the concentration of the fluorescent-labeled peptide in each pharmaceutical composition.

### (Measurement condition)

HPLC device: HPLC-EXTREMA manufactured by JASCO Corporation
Column: PLRP-S 1000A, 8 µm, length 50 mm × inner diameter 4.6 mm (manufactured by Agilent Technologies, Inc., product number: PL1512-1802)
Column temperature: 40°C
Mobile phase:
   (A) 0.1 v/v% trifluoroacetic acid/acetonitrile
   (B) 0.1 v/v% trifluoroacetic acid/water
Flow rate: 2 mL/min
Injection amount: 30 µL
Detector: UV (215 nm)
Gradient program: shown in the following Table 2. In Table 2, "%" means "v/v%".

**[Table 2]**

| Time (min) | Mobile phase A (%) | Mobile phase B (%) |
|---|---|---|
| 0 → 0.5 | 5 | 95 |
| 0.5 → 3.4 | 5 → 100 | 95 → 0 |
| 3.4 → 3.45 | 100 → 5 | 0 → 95 |
| 3.45 → 13.0 | 100 → 5 | 0 → 95 |

### [Test Example 1-1]

### (Analysis of uptake of peptide into myeloid cells in lymph nodes and activation thereof)

### 1. Materials

Adjuvant: CpG oligo DNA1668 (purchased from Ajinomoto Bio-Pharma Services, GeneDesign)
Compositions administered: Pharmaceutical compositions of Example 2-1, Comparative Example 2-1, Comparative Example 3, and Comparative Example 4-1

The fluorescent-labeled antibodies used for evaluation were as follows. All were purchased from BioLegend, Inc.
Brilliant Violet 421 labeled anti-mouse F4/80 antibody (clone BM8);
Brilliant Violet 510 labeled anti-mouse CD11c antibody (clone N418);
APC-Cy7 labeled anti-mouse XCR1 antibody (clone ZET);
APC-labeled anti-mouse CD80 antibody (clone 16-10A1);
APC-labeled anti-mouse CD86 antibody (clone GL-1); and
Purified CD16/32 antibody (clone 93).

The combinations of antibodies used in the below-mentioned FACS analysis were as follows.

**[Table 3]**

| Type of cells | First antibody | Second antibody | Third antibody |
|---|---|---|---|
| Macrophage (Mph) | V421 labeled anti-mouse F4/80 antibody | APC-labeled anti-mouse CD80 antibody | - |
| | V421 labeled anti-mouse F4/80 antibody | APC-labeled anti-mouse CD86 antibody | - |
| Dendritic cell (DC) | V510 labeled anti-mouse CD11c antibody | APC-labeled anti-mouse CD80 antibody | - |
| | V510 labeled anti-mouse CD11c antibody | APC-labeled anti-mouse CD86 antibody | - |
| Type 1 dendritic cell (cDC1) | V510 labeled anti-mouse CD11c antibody | APC-labeled anti-mouse CD80 antibody | APC-Cy7 labeled anti-mouse XCR1 antibody |
| | V510 labeled anti-mouse CD11c antibody | APC-labeled anti-mouse CD86 antibody | APC-Cy7 labeled anti-mouse XCR1 antibody |

### 2. Analysis method

Each pharmaceutical composition of Example 2-1, Comparative Example 2-1 and Comparative Example 4-1 was diluted with 10 mM phosphate buffer containing 10% by mass of sucrose such that the concentration of the fluorescent- (fluorescein-) labeled peptide was about 0.3 mg/mL, and the pharmaceutical composition of Comparative Example 3 was diluted with phosphate-buffered saline pH 7.4, followed by administering subcutaneously the diluted solution of each pharmaceutical composition to the right dorsal region of BALB/c mice at a peptide dose of 60 µg. At the same time, 50 µg of CpG oligo DNA 1668 (Ajinomoto Bio-Pharma Service, GeneDesign) dissolved in PBS was administered as an adjuvant.

Twenty hours after administration, the lymph node to which the administration region belonged (right inguinal lymph node) was collected, and the lymph node was ground using a slide glass, followed by suspending the cells in RPMI 1640 medium. At this time, the cells from two mice per group were pooled. After centrifugation (400×g, five minutes, 4°C), the supernatant was removed from the resultant, followed by washing the resultant twice with RPMI 1640 medium, and then suspending the resultant in RPMI 1640 medium containing 10 v/v% FBS. The number of cells was measured using a hemocytometer, and the cell concentration was adjusted to 1.2×10⁷ cells/mL.

50 µL of the cell suspension was added to a 96-well V-bottom microplate (Nunc, Thermo Fisher Scientific) at 6×10⁵ cells per well. The cell suspension was centrifuged (2000 rpm, two minutes, 4°C), the supernatant was removed, and the resultant was washed twice with 200 µL of staining buffer (PBS containing 0.5 v/v% FBS) and then suspended. After centrifugation and removal of the supernatant, 50 µL of a solution containing anti-mouse CD16/CD32 antibody diluted 50 times was added, and the mixture was left still in a dark place at 4°C for ten minutes. After adding 150 µL of staining buffer and removing the supernatant by centrifugation, the cells were further washed twice with 200 µL of staining buffer. 50 µL of a solution containing the first, second, and third antibodies in the combinations listed in Table 3 above at the usage concentration recommended by each antibody manufacturer was added to the cells and mixed, and the mixture was left still in a dark place at 4°C for 15 minutes. After adding 150 µL of staining buffer and removing the supernatant by centrifugation, the cells were further washed twice with 200 µL of staining buffer. After centrifuging and removing the supernatant, the cells were resuspended in 200 µL of staining buffer and transferred to a round bottom polystyrene tube (BD Biosciences). The cells were analyzed using a flow cytometer FACS Canto II (BD Biosciences) and the attached analysis software (FACSDiva).

Dendritic cells (DC) were detected as a CD11c-positive group, macrophages (Mph) were detected as a F4/80-positive group, and conventional type 1 DC (cDC1) were detected as a CD11c-positive and XCR1-positive group.

In each cell type, FITC (fluorescein)-positive cells were detected as peptide-uptake cells.

The expression of co-stimulatory molecules CD80 and CD86 was analyzed on FITC-positive cells in each cell type.

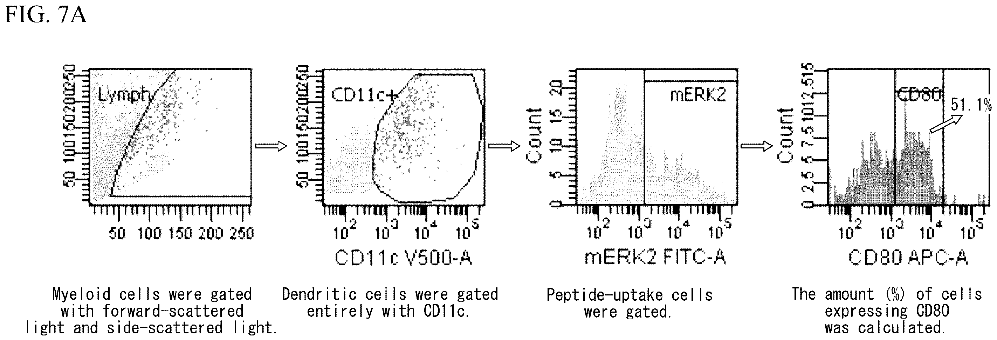

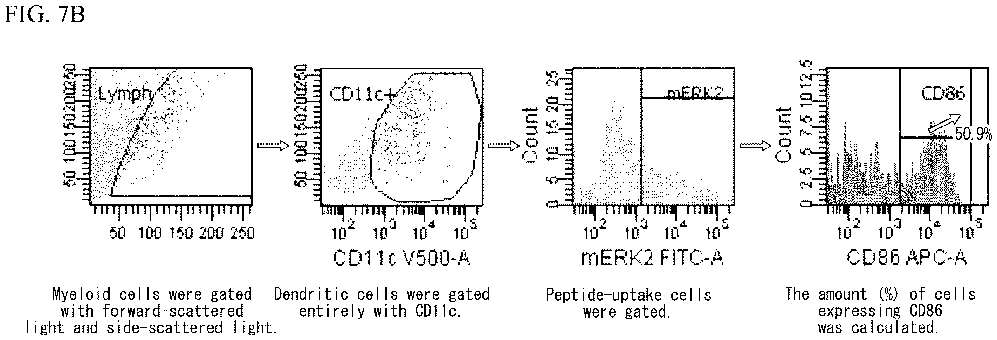

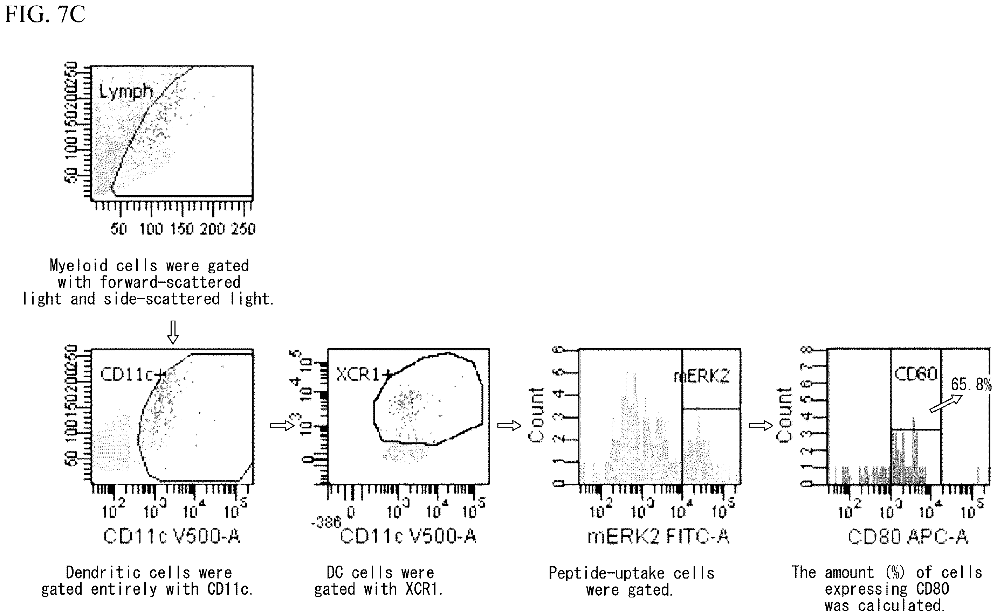

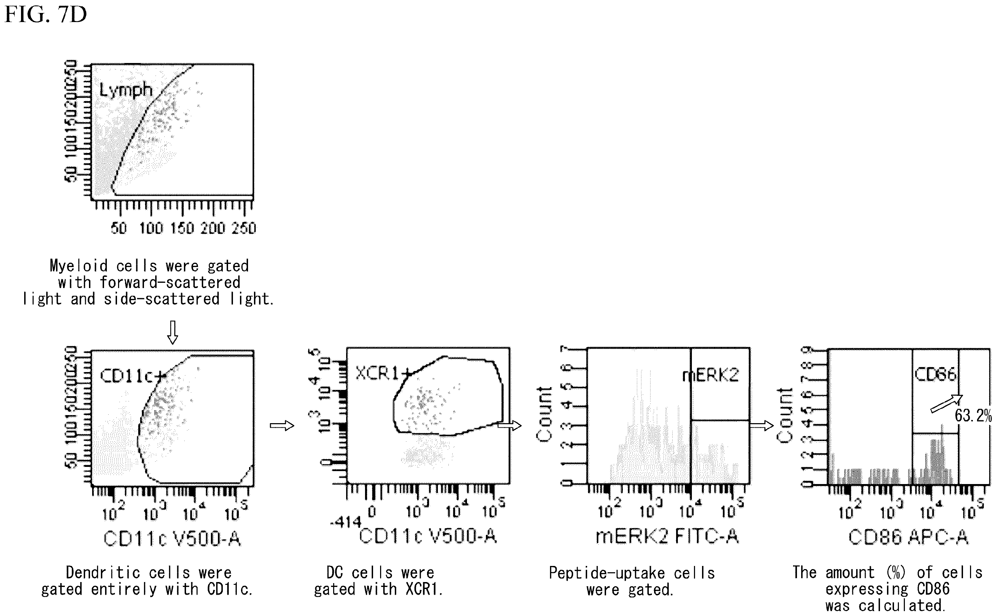

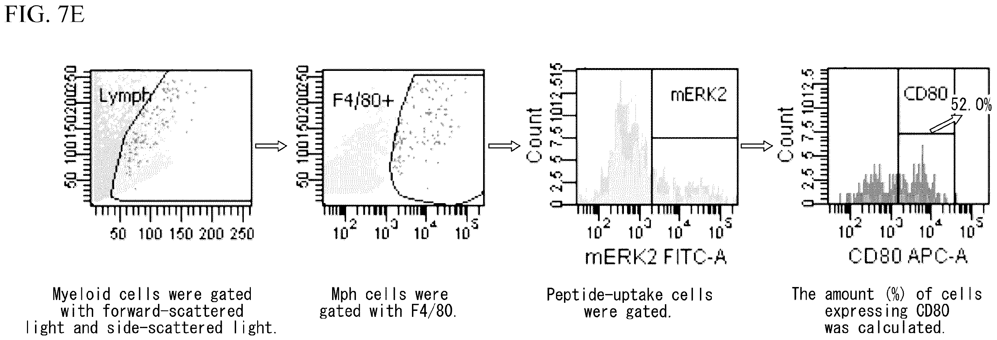

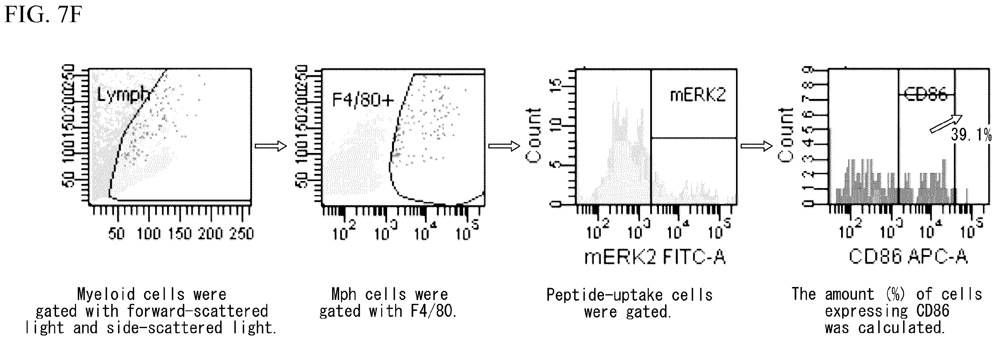

The FACS analysis method is shown in Figs. 7A to 7F.

The analysis results are shown in Tables 4-1 to 4-3. For example, in Table 4-1, the term "peptide + CD80 + DC" means "peptide-uptake dendritic cells (DC) that express CD80", the term "peptide + CD86 + DC" means "peptide-uptake dendritic cells (DC) that express CD86", and the term "peptide+DC" means "peptide-uptake dendritic cells (DC)."

**[Table 4-1]**

| DC (unit: %) | peptide + CD80 + DC/ peptide + DC | peptide + CD86 + DC/ peptide + DC |
|---|---|---|
| Example 2-1 | 51.1 | 50.9 |
| Comparative Example 2-1 | 44.1 | 31.7 |
| Comparative Example 4-1 | 50.2 | 46.3 |
| Comparative Example 3 | 27.4 | 23.9 |

**[Table 4-2]**

| cDC1 (unit: %) | peptide + CD80 + cDC1/ peptide + cDC1 | peptide + CD86 +cDC1/ peptide + cDC1 |
|---|---|---|
| Example 2-1 | 65.8 | 63.2 |
| Comparative Example 2-1 | 44.7 | 45.3 |
| Comparative Example 4-1 | 53.7 | 37.1 |
| Comparative Example 3 | 23.7 | 17.0 |

**[Table 4-3]**

| Mph (unit: %) | peptide + CD80 + Mph/ peptide + Mph | peptide + CD86 + Mph / peptide + Mph |
|---|---|---|
| Example 2-1 | 52.0 | 39.1 |
| Comparative Example 2-1 | 40.5 | 19.5 |
| Comparative Example 4-1 | 43.8 | 30.1 |
| Comparative Example 3 | 57.8 | 37.0 |

As shown in Tables 4-1 to 4-3, higher expression of co-stimulatory molecules (CD80 and CD86) in macrophages and DC (especially cDC1) was confirmed in the mice group administered with the pharmaceutical composition of Example 2-1 than the mice group administered with the pharmaceutical composition of Comparative Example 2-1 or Comparative Example 4-1 (hyaluronic acid derivative before fractionation). Furthermore, regarding DC (particularly cDC1), higher expression of co-stimulatory molecules (CD80 and CD86) in macrophages and DC (especially cDC1) was confirmed in the mice group administered with the pharmaceutical composition of Example 2-1 than the mice group administered with the pharmaceutical composition of Comparative Example 3, which was a derivative of another polysaccharide (pullulan).

### [Test Example 1-2]

The test was carried out by the same method as Test Example 1-1, except that each pharmaceutical composition of Example 2-2, Comparative Example 2-2 and Comparative Example 4-2 was diluted with 10 mM phosphate buffer, pH 7.4, containing 10% by mass of sucrose such that the concentration of the fluorescent- (fluorescein-) labeled peptide was about 0.2 mg/mL, followed by administering subcutaneously the diluted solution of each pharmaceutical composition at a peptide dose of 40 µg. The analysis results are shown in Table 5-1 to Table 5-3.

**[Table 5-1]**

| DC (unit: %) | peptide + CD80 + DC/ peptide + DC | peptide + CD86 + DC/ peptide + DC |
|---|---|---|
| Example 2-2 | 46.3 | 37.0 |
| Comparative Example 2-2 | 30.7 | 25.4 |
| Comparative Example 4-2 | 39.6 | 33.7 |

**[Table 5-2]**

| cDC 1 (unit: %) | peptide + CD80 + cDC1/ peptide + cDC1 | peptide + CD86 +cDC1/ peptide + cDC1 |
|---|---|---|
| Example 2-2 | 82.1 | 75.9 |
| Comparative Example 2-2 | 59.0 | 55.7 |
| Comparative Example 4-2 | 73.7 | 66.3 |

**[Table 5-3]**

| Mph (unit: %) | peptide + CD80 + Mph/ peptide + Mph | peptide + CD86 + Mph / peptide + Mph |
|---|---|---|
| Example 2-2 | 46.9 | 36.5 |
| Comparative Example 2-2 | 34.7 | 13.4 |
| Comparative Example 4-2 | 31.3 | 20.9 |

**In** a similar manner to Test Example 1-1, higher expression of co-stimulatory molecules (CD80 and CD86) in macrophages and DC (especially cDC1) was confirmed in the mice group administered with the pharmaceutical composition of Example 2-2 than the mice group administered with the pharmaceutical composition of Comparative Example 2-2 or Comparative Example 4-2 (hyaluronic acid derivative before fractionation). Although the expression of CD80 and CD86 on cDC1 was relatively high in the mice group administered with the pharmaceutical composition of Comparative Example 4-2, the expression of CD80 and CD86 on macrophages was low and inferior to that in the mice group administered with the pharmaceutical composition of Example 2-2.

### [Test Example 1-3]

The test was carried out by the same method as Test Example 1-1, except that the pharmaceutical composition of Example 2-3 was used to be administered subcutaneously at a peptide dose of 40 µg. The analysis results are shown in Table 6-1 to Table 6-3.

**[Table 6-1]**

| DC (unit: %) | peptide + CD80 + DC/ peptide + DC | peptide + CD86 + DC/ peptide + DC |
|---|---|---|
| Example 2-3 | 79.4 | 67.7 |

**[Table 6-2]**

| cDC1 (unit: %) | peptide + CD80 + cDC1/ peptide + cDC1 | peptide + CD86 +cDC1/ peptide + cDC1 |
|---|---|---|
| Example 2-3 | 85.4 | 86.3 |

**[Table 6-3]**

| Mph (unit: %) | peptide + CD80 + Mph/ peptide + Mph | peptide + CD86 + Mph / peptide + Mph |
|---|---|---|
| Example 2-3 | 66.7 | 51.2 |

In a similar manner to Test Example 1-1 and Test Example 1-2, high expression of co-stimulatory molecules (CD80 and CD86) in macrophages and DC (especially cDC1) was confirmed in the mice group administered with the pharmaceutical composition of Example 2-3.

Here, it has been reported that the decrease in the expression of CD86 on macrophages acts or is acting on immunosuppression (Reference Document 3: Taams LS et al., "Modulation of monocyte/macrophage function by human CD4+CD25+ regulatory T cells.", Hum Immunol., Vol. 66, Issue 3, pp. 222-230, 2005.: Reference Document 4: Sansom DM et al., "What's the difference between CD80 and CD86?", Trends in Immunology, Vol. 24, Issue 6, pp. 313-318, 2003). The pharmaceutical compositions of Example 2-1, Example 2-2 and Example 2-3, in which CD86 was strongly expressed not only on DC but also on macrophages, are expected to induce stronger immunity in view of the report in Reference Document 3 and the results of Test Example 1-1 to Test Example 1-3.

It is assumed from these results that the use of the pharmaceutical composition of the present embodiment made it possible to stably and efficiently deliver the medicinal ingredient such as a peptide to immune cells (especially cDC1 or macrophages), furthermore improve, promote, or enhance the uptake of the medicinal ingredient into immune cells, and simultaneously improve, promote, maintain or enhance the activation of the immune cells (especially, expression of co-stimulatory molecules CD80 and CD86 on cDC1).

### <Study of production conditions of pharmaceutical composition>

### [Reference Example 1]

A pharmaceutical composition containing a complex of a peptide and a hyaluronic acid derivative was prepared using the hyaluronic acid derivative obtained in Synthesis Example 1-1.

As the peptide, a gp100RP2RP1_6Y peptide manufactured by Biologica, Inc., and having the following amino acid sequence was used.

SVYDFFVWLYYYYYYTWHRYHLLYYYYYYEGSRNQDWL (SEQ ID No. 2)

Specifically, the freeze-dried hyaluronic acid derivative obtained in Synthesis Example 1-1 was weighed, injection water was added thereto such that the concentration of the freeze-dried hyaluronic acid derivative was as shown in Table 7 below, and the mixture was stirred overnight to ensure sufficient dissolution.

In contrast, a peptide was dissolved in DMSO in another container such that the concentration thereof became 50 mg/mL.

After confirming the dissolution, an aqueous solution containing 1 mol/L sodium hydroxide (manufactured by FUJIFILM Wako Pure Chemical Corporation) was added to the above-mentioned aqueous solution of a hyaluronic acid derivative as shown in Table 7 below.

The aqueous solution of the hyaluronic acid derivative and the dimethyl sulfoxide solution of the peptide were mixed at the volume ratio shown in Table 7 below at room temperature (approximately 25°C), followed by stirring the mixture at room temperature (approximately 25°C) for 24 hours to obtain a composition containing a complex of a peptide and a hyaluronic acid derivative. At this time, the pH of the solution was also measured.

### (Stability of complex of peptide and hyaluronic acid derivative: external appearance of aqueous solution)

After 24 hours, the appearance of the composition (aqueous solution) containing the complex of the peptide and the hyaluronic acid derivative was visually judged to confirm whether the composition was cloudy. The results are shown in Table 7 below.

**[Table 7]**

| R. Ex. | Concentration of aqueous solution of hyaluronic acid derivative (mg/mL) | Aqueous solution of hyaluronic acid derivative (volume ratio) | Aqueous solution of 1 mol/L sodium hydroxide (volume ratio) | DMSO solution of peptide (volume ratio) | pH | Appearance of complex of peptide and hyaluronic acid derivative | Concentration of peptide in pharmaceutical composition (mg/mL) | Average particle size (nm) |
|---|---|---|---|---|---|---|---|---|
| 1 | 5 | 100 | 2 | 1 | 8.90 | Transparent | 0.302 | 86.7 |

| | | | | | | | | |
|---|---|---|---|---|---|---|---|---|
| (R. Ex.: Reference Example) | | | | | | | | |

Thereafter, a solid purified sucrose (manufactured by FUJIFILM Wako Pure Chemical Corporation, for production only, under the product number of 198-18385) was added to the composition containing the complex of the peptide and the hyaluronic acid derivative such that the amount of sucrose relative to the total mass of the composition and the sucrose became 10% by mass, and stirred at room temperature (approximately 25°C) for one hour or more. Then, the resultant was diluted with 25 mM phosphate buffer pH 7.4 containing 10% by mass of sucrose such that the theoretical concentration of the peptide became 0.3 mg/mL, followed by subjecting the resultant to sterilization filtration using a 0.2 µm PES (polyethersulfone) (manufactured by Pall Corporation, acrodisc syringe filter, 25 mmΦ) to obtain a pharmaceutical composition containing a complex of the peptide and the hyaluronic acid derivative. The concentration of the peptide in the pharmaceutical composition containing the complex of the peptide and the hyaluronic acid derivative was quantified by the same method as Example 2-1, and the result is shown in Table 7 above.

### (Average particle size)

A composition containing a complex of a peptide and a hyaluronic acid derivative produced by the below-mentioned method was subjected to a measurement by dynamic light scattering (DLS) under the following conditions to obtain the z-average particle size. The result is shown in Table 7 above.

### (Measurement condition)

DLS device: ELSZ 2000 manufactured by Otsuka Electronics Co., Ltd.
Cell: Micro-volume particle size cell
Temperature: 25°C
Concentration of complex of peptide and hyaluronic acid derivative: 4.95mg/mL

As shown in Table 7 above, it became apparent that the adjustment of the pH of the mixture solution within a specific range in the production of the pharmaceutical composition made it possible to maintain the particle size of the complex of a medicinal ingredient such as a peptide and a hyaluronic acid derivative within a certain range and improve the stability of the complex in the solution.

### [Reference Examples 2-1 to 2-3]

A pharmaceutical composition containing a complex of a peptide and a hyaluronic acid derivative was prepared using the hyaluronic acid derivative obtained in Synthesis Example 1-1 and a peptide different from that of Reference Example 1.

As the peptide, a peptide manufactured by Biologica, Inc., and having the following amino acid sequence was used.

GSNPARYEFLWGPRALAETSYVKVLEHVVRVNARVRIAYP (SEQ ID No. 3)

Specifically, the freeze-dried hyaluronic acid derivative obtained in Synthesis Example 1-1 was weighed, injection water was added thereto such that the concentration of the freeze-dried hyaluronic acid derivative was as shown in Table 8 below, and the mixture was stirred overnight to ensure sufficient dissolution.

In contrast, a peptide was dissolved in DMSO in another container such that the concentration thereof became 50 mg/mL.

After confirming the dissolution, an aqueous solution containing 1 mol/L sodium hydroxide (manufactured by FUJIFILM Wako Pure Chemical Corporation) was added to the above-mentioned aqueous solution of a hyaluronic acid derivative as shown in Table 8 below.

The aqueous solution of the hyaluronic acid derivative and the DMSO solution of the peptide were mixed at the volume ratio shown in Table 8 below at room temperature (approximately 25°C), followed by stirring the mixture at room temperature (approximately 25°C) for 24 hours to obtain a composition containing a complex of a peptide and a hyaluronic acid derivative. At this time, the pH of the solution was also measured.

Thereafter, a solid purified sucrose (manufactured by FUJIFILM Wako Pure Chemical Corporation, for production only, under the product number of 198-18385) was added to the composition containing the complex of the peptide and the hyaluronic acid derivative such that the amount of sucrose relative to the total mass of the composition and the sucrose became 10% by mass, and stirred at room temperature (approximately 25°C) for one hour or more. Then, the resultant was subjected to sterilization filtration using a 0.2 µm PES (polyethersulfone) (manufactured by Pall Corporation, acrodisc syringe filter, 25 mmΦ) to obtain a pharmaceutical composition containing a complex of the peptide and the hyaluronic acid derivative. The concentration of the peptide in the pharmaceutical composition containing the complex of the peptide and the hyaluronic acid derivative at this time was quantified in the same manner as in Example 2-1 mentioned above, and the average particle size of the pharmaceutical composition containing the complex of the peptide and the hyaluronic acid derivative was measured by the same method as Reference Example 1 mentioned above. The results are shown in Table 8 below.

**[Table 8]**

| R. Ex. | Concentration of aqueous solution of hyaluronic acid derivative (mg/mL) | Aqueous solution of hyaluronic acid derivative (volume ratio) | Aqueous solution of 1 mol/L sodium hydroxide (volume ratio) | DMSO solution of peptide (volume ratio) | pH | Concentration of peptide in pharmaceutical composition (mg/mL) | Average particle size (nm) |
|---|---|---|---|---|---|---|---|
| 2-1 | 5 | 100 | 0 | 1 | 6.23 | 0.188 | 85.7 |
| 2-2 | 5 | 100 | 0.5 | 1 | 7.24 | 0.256 | 64.3 |
| 2-3 | 5 | 100 | 2 | 1 | 9.10 | 0.256 | - |

| | | | | | | | |
|---|---|---|---|---|---|---|---|
| (R. Ex.: Reference Example) | | | | | | | |

As shown in Table 8 above, even when the pharmaceutical composition was produced using a peptide different from that of Reference Example 1, the adjustment of the pH of the mixture solution within a specific range made it possible to maintain the particle size of the complex of a medicinal ingredient such as a peptide and a hyaluronic acid derivative within a certain range, improve the stability of the complex in the solution, and increase the concentration of the peptide in the pharmaceutical composition.

### <Production conditions to maintain appropriate particle size distribution>

### [Test Examples 2-1 to 2-8]

An aqueous solution containing 5 mg/mL of the hyaluronic acid derivative fractionated by the same method as in Example 1-1 was prepared, and a detailed study was carried out on the mixing ratio of the aqueous phase containing the hyaluronic acid derivative and the oil phase.

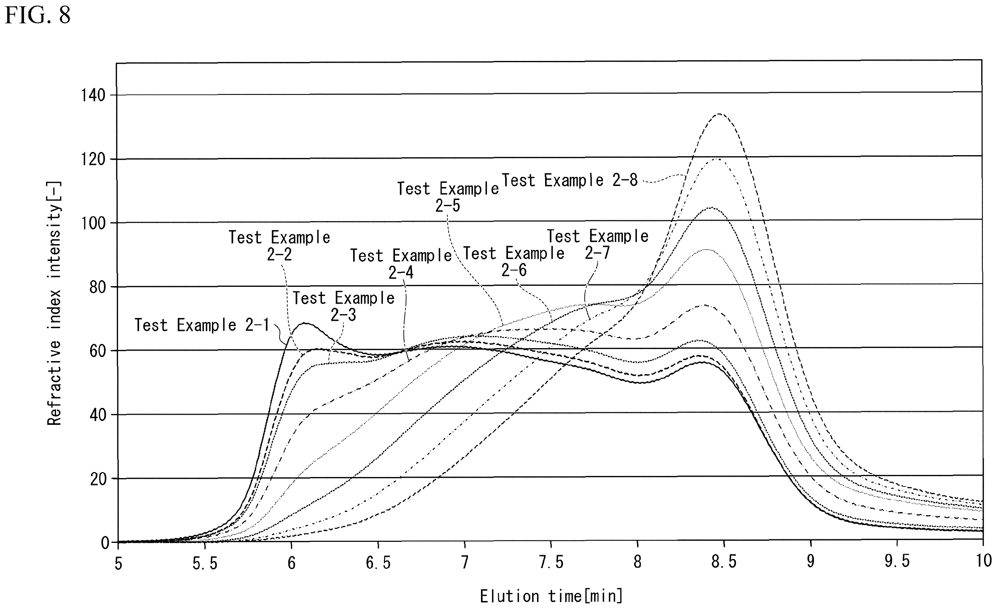

DMSO as an oil phase was mixed with 400 µL of 5 mg/mL of the hyaluronic acid derivative as shown in Table 9 below. After mixing sufficiently, injection water was added to the mixture as shown in Table 9 below such that the final concentration of the hyaluronic acid derivative was constant. Furthermore, after incubation at room temperature (25°C) for 24 hours, the resultant was subjected to measurement by gel permeation chromatography under the following conditions, and the area ratio A1/A2 was calculated by the same method as Example 1-1 (Table 9). The obtained gel permeation chromatogram is shown in FIG. 8.

### (Measurement conditions)

Device: HLC8420-GPC (manufactured by TOSOH CORPORATION)
Column: G4000SWXL (manufactured by TOSOH CORPORATION, particle size 8 µm, inner diameter 7.8 mm, length 30 cm, product number:8542)
Eluent: 10 mM phosphate buffer (pH 7.4)
Flow rate: 1 mL/min
Injection amount: 50 µL
Detector: RI
Temperature: 30°C

**[Table 9]**

| Test Example | Oil phase DMSO Mixed amount (µL) | Injection water Mixed amount (µL) | Amount of oil phase relative to aqueous phase (100% by volume) (% by volume) | A1/A2 |
|---|---|---|---|---|
| 2-1 | 4 | 396 | 1 | 1.469 |
| 2-2 | 20 | 380 | 5 | 1.367 |
| 2-3 | 40 | 360 | 10 | 1.239 |
| 2-4 | 80 | 320 | 20 | 0.927 |
| 2-5 | 160 | 240 | 40 | 0.655 |
| 2-6 | 240 | 160 | 60 | 0.457 |
| 2-7 | 320 | 80 | 80 | 0.303 |
| 2-8 | 400 | 0 | 100 | 0.205 |

### [Test Examples 3-1 to 3-6]

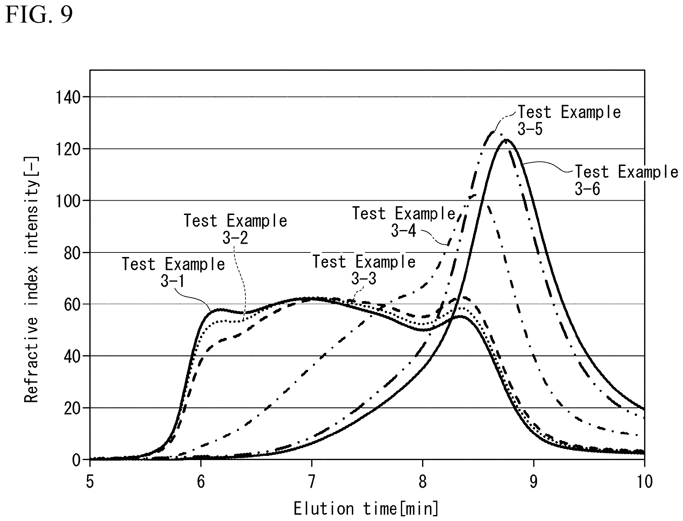

The mixing ratio for maintaining an appropriate particle size distribution was studied in the same manner as in Test Examples 2-1 to 2-8, except that ethanol was used instead of DMSO as an oil phase. Measurement was carried out by gel permeation chromatography, the area ratio A1/A2 was calculated in the same manner as in Example 1-1, and the results are shown in Table 10 below. The obtained gel permeation chromatogram is shown in FIG. 9.

**[Table 10]**

| Test Example | Oil phase Ethanol Mixed amount (µL) | Injection water Mixed amount (µL) | Amount of oil phase relative to aqueous phase (100% by volume) (% by volume) | A1/A2 |
|---|---|---|---|---|
| 3-1 | 4 | 396 | 1 | 1.438 |
| 3-2 | 20 | 380 | 5 | 1.334 |
| 3-3 | 40 | 360 | 10 | 1.170 |
| 3-4 | 160 | 240 | 40 | 0.345 |
| 3-5 | 240 | 160 | 60 | 0.075 |
| 3-6 | 400 | 0 | 100 | 0.062 |

It was revealed from the results of Test Examples 2-1 to 2-8 and 3-1 to 3-6 that the particle size of the hyaluronic acid derivative, which exhibited excellent medicinal efficacy, could be maintained through the mixing step of making the mixing ratio, based on volume, of an oil phase and an aqueous phase containing the hyaluronic acid derivative be 20:100 to 0.01:100.

### [Comparative Example 5-1]

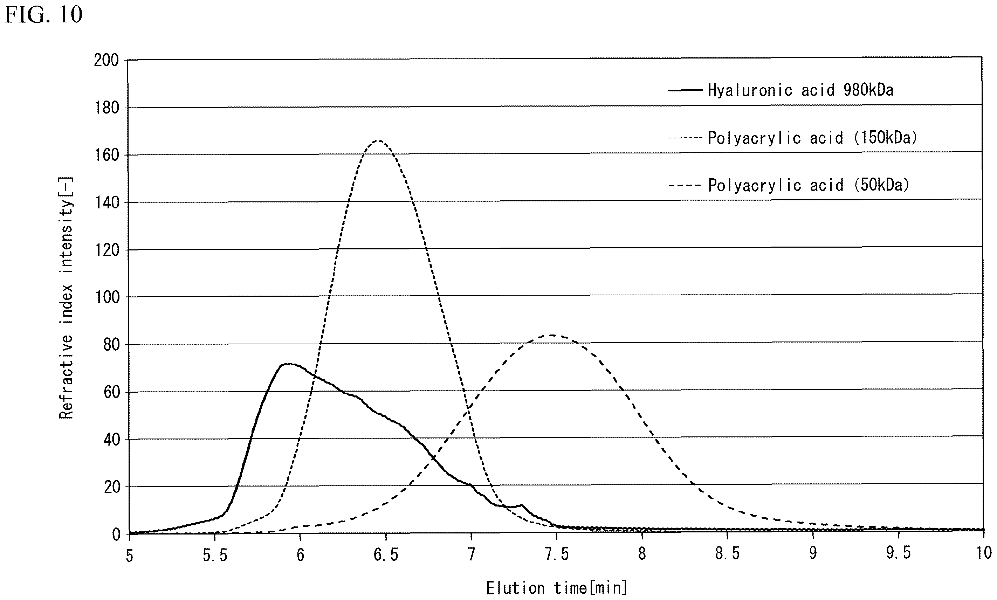

Hyaluronic acid powders having a weight-average molecular weight Mw (absolute molecular weight) of 980 kDa (manufactured by Kikkoman Corporation) were dissolved in injection water such that the concentration thereof was 1 mg/mL, and then subjected to measurement by gel permeation chromatography under the same conditions as in Synthesis Example 1-1. The chromatogram obtained is shown in FIG. 10.

Furthermore, hyaluronic acid powders having a weight-average molecular weight Mw (absolute molecular weight) of 980 kDa were dissolved in injection water such that the concentration thereof was 10 mg/mL, and then diluted with injection water such that the concentration thereof was 5 mg/mL to obtain an aqueous solution of the hyaluronic acid.

In contrast, an antigenic peptide was dissolved in dimethyl sulfoxide (DMSO) in another container such that the concentration thereof became 50 mg/mL, thereby obtaining a DMSO solution of the antigenic peptide. A peptide manufactured by Biologica, Inc., and having the following amino acid sequence was used as the antigenic peptide.

NDHIAYFLYQILRGLQYIHSANVLHRDLKPSNLLLNT (SEQ ID No. 1)

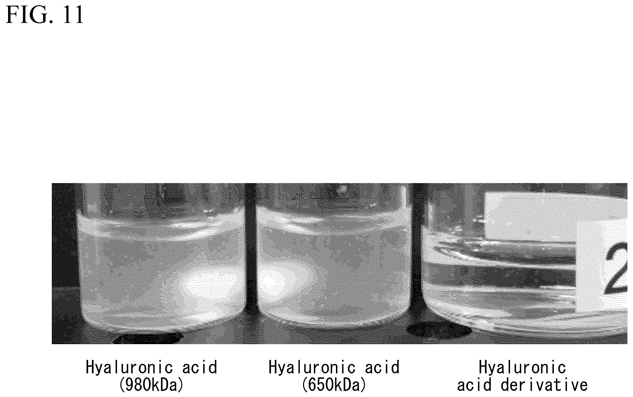

A complex of an antigenic peptide and a hyaluronic acid was tried to be prepared by mixing the aqueous solution of the hyaluronic acid and the DMSO solution of the antigenic peptide at a volume ratio of 100:1 at room temperature (about 25°C), and then stirring the mixture at room temperature (about 25°C) for 24 hours. The resulting mixture solution was cloudy, as confirmed by the photograph indicated as "hyaluronic acid (980 kDa)" in FIG. 11, which suggested that the antigenic peptide was not dissolved and a large amount of peptide was not encapsulated.

Thereafter, a solid purified sucrose (manufactured by FUJIFILM Wako Pure Chemical Corporation, for production only, under the product number of 198-18385) was added to the composition containing the complex of the peptide and the hyaluronic acid such that the amount of sucrose relative to the total mass of the composition and the sucrose became 10% by mass, and stirred at room temperature for one hour or more. After confirming that the sucrose was sufficiently dissolved, the resultant was diluted with 10 mM phosphate buffer pH 7.4 containing 10% by mass of sucrose such that the concentration of the peptide became about 0.30 mg/mL, followed by subjecting the resultant to sterilization filtration using a 0.2 µm PES (polyethersulfone) (manufactured by Pall Corporation, acrodisc syringe filter, 25 mmΦ) to obtain a pharmaceutical composition containing a complex of the peptide and the hyaluronic acid. The amount of the peptide in the pharmaceutical composition was quantified by a high-performance liquid chromatography measurement in the same manner as Example 2-1. The amount of the peptide in the pharmaceutical composition was less than the detection limit.

### [Comparative Example 5-2]

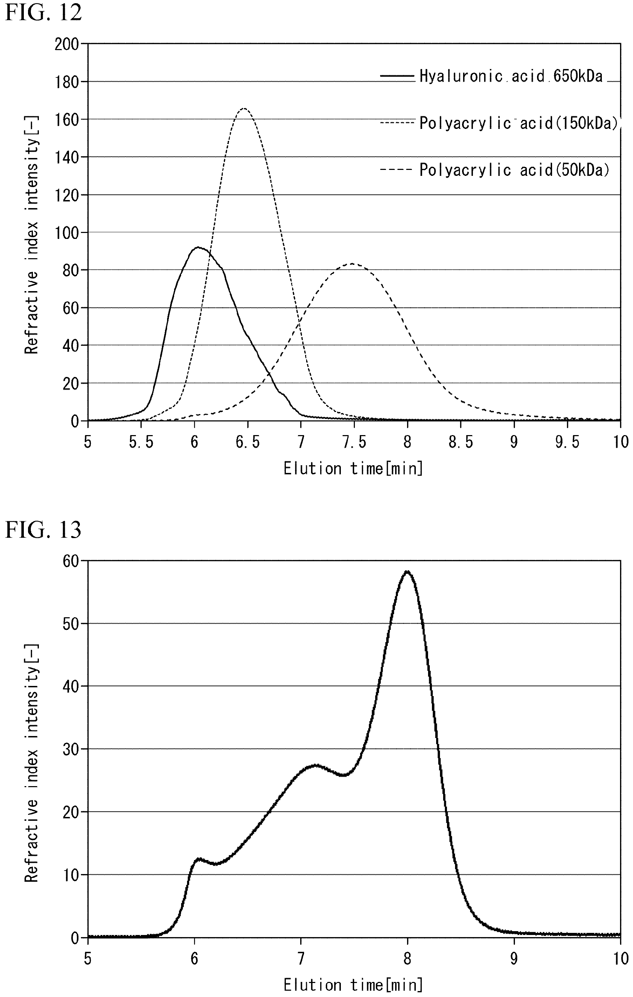

Hyaluronic acid powders having a weight-average molecular weight Mw (absolute molecular weight) of 650 kDa (manufactured by Kewpie Co., Ltd.) were dissolved in injection water such that the concentration thereof was 1 mg/mL, and then subjected to measurement by gel permeation chromatography under the same conditions as in Synthesis Example 1-1. The chromatogram obtained is shown in FIG. 12.

Hyaluronic acid powders having a weight-average molecular weight Mw (absolute molecular weight) of 650 kDa were dissolved in injection water such that the concentration thereof was 10 mg/mL, and then diluted with injection water such that the concentration thereof was 5 mg/mL to obtain an aqueous solution of the hyaluronic acid.

In contrast, an antigenic peptide was dissolved in dimethyl sulfoxide (DMSO) in another container such that the concentration thereof became 50 mg/mL, thereby obtaining a DMSO solution of the antigenic peptide. A peptide manufactured by Biologica, Inc., and having the following amino acid sequence was used as the antigenic peptide.

NDHIAYFLYQILRGLQYIHSANVLHRDLKPSNLLLNT (SEQ ID No. 1)

A complex of an antigenic peptide and a hyaluronic acid was tried to be prepared by mixing the aqueous solution of the hyaluronic acid and the DMSO solution of the antigenic peptide at a volume ratio of 100:1 at room temperature (about 25°C), and stirring the mixture at room temperature (about 25°C) for 24 hours. The resulting mixture solution was cloudy, as confirmed by the photograph indicated as "hyaluronic acid (640 kDa)" in FIG. 11, which suggested that the antigenic peptide was not dissolved and a large amount of peptide was not encapsulated.

Thereafter, a solid purified sucrose (manufactured by FUJIFILM Wako Pure Chemical Corporation, for production only, under the product number of 198-18385) was added to the composition containing the complex of the peptide and the hyaluronic acid such that the amount of sucrose relative to the total mass of the composition and the sucrose became 10% by mass, and stirred at room temperature (approximately 25°C) for one hour or more. After confirming that the sucrose was sufficiently dissolved, the resultant was diluted with 10 mM phosphate buffer pH 7.4 containing 10% by mass of sucrose such that the concentration of the peptide became about 0.30 mg/mL, followed by subjecting the resultant to sterilization filtration using a 0.2 µm PES (polyethersulfone) (manufactured by Pall Corporation, acrodisc syringe filter, 25 mmΦ) to obtain a pharmaceutical composition containing a complex of the peptide and the hyaluronic acid. The amount of the peptide in the pharmaceutical composition was quantified by a high-performance liquid chromatography measurement in the same manner as Example 2-1. The amount of the peptide in the pharmaceutical composition was less than the detection limit.

It was confirmed from the results of Comparative Examples 5-1 and 5-2 that it was difficult for the high-molecular-weight hyaluronic acids having an area ratio A1/A2 of 0.9 or more to encapsulate the peptide, stably and efficiently deliver the medicinal ingredient such as a peptide to immune cells (especially cDC1 or macrophages), and furthermore improve, promote, or enhance the uptake of the medicinal ingredient into immune cells.

### <Evaluation of properties as infectious disease vaccine (evaluation of antibody production against foreign antigens) and evaluation of in-vivo production of antibodies for immune disease treatment>

### [Test Example 4]

### [Comparative Example 6-1]

A hyaluronic acid derivative was synthesized in the same manner as in Synthesis Example 1-1. The hyaluronic acid derivative was used directly without fractionation. The obtained freeze-dried hyaluronic acid derivative was weighed, and injection water was added thereto such that the concentration thereof became 5 mg/mL, followed by stirring the mixture overnight at room temperature to ensure sufficient dissolution. The aqueous solution of the hyaluronic acid derivative was diluted five times with injection water to a concentration of 1 mg/mL, and then subjected to measurement by gel permeation chromatography. FIG. 13 shows the gel permeation chromatogram obtained. In addition, the particle size distribution of the hyaluronic acid derivative was evaluated in the same manner as in Example 1-1, and the results are shown in Table 11 below.

### [Example 6-1]

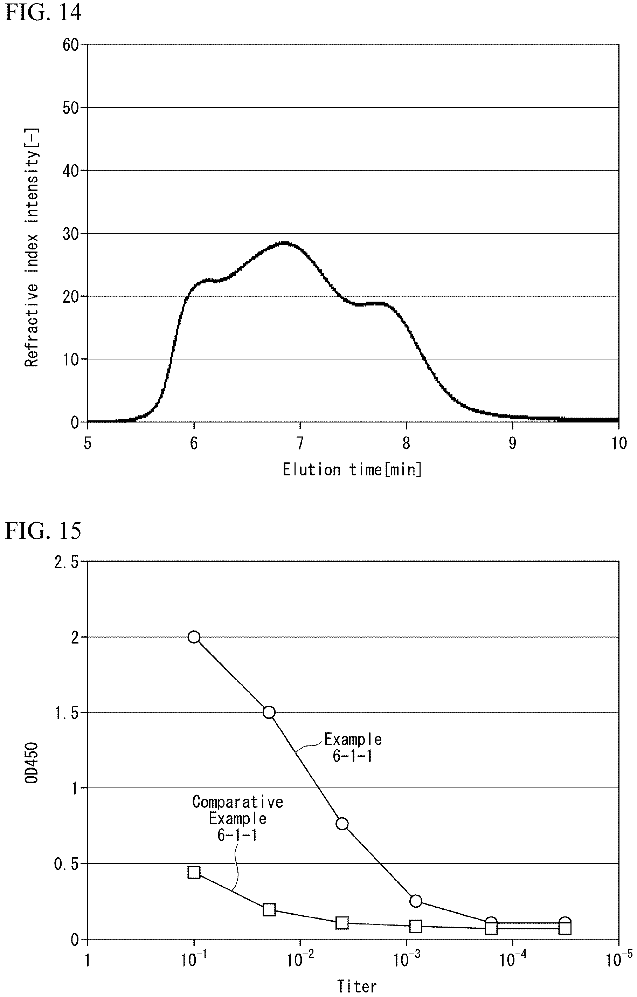

A hyaluronic acid derivative synthesized in the same manner as in Synthesis Example 1-1 was fractionated by the same method as Example 1-1 to obtain a hyaluronic acid derivative. The hyaluronic acid derivative in the obtained internal dialysate was subjected to measurement by gel permeation chromatography. FIG. 14 shows the gel permeation chromatogram obtained. In addition, the particle size distribution of the hyaluronic acid derivative was evaluated in the same manner as in Example 1-1, and the results are shown in Table 11 below.

**[Table 11]**

| | A1/A2 | Da/Db |
|---|---|---|
| Comparative Example 6-1 | 0.81 | 0.62 |
| Example 6-1 | 2.03 | 0.79 |

### <Production of pharmaceutical composition>

### [Example 6-1-1]

The concentration of the hyaluronic acid derivative in the aqueous solution of the hyaluronic acid derivative obtained in Example 6-1, the concentration being calculated from the area of the known concentration of the hyaluronic acid derivative, was 4.28 mg/mL.

In another container, ovalbumin (OVA, low endotoxin product manufactured by FUJIFILM Wako Pure Chemical Corporation) was dissolved in PBS (pH 7.4) (manufactured by FUJIFILM Wako Pure Chemical Corporation under the product number of 166-23555) such that the concentration thereof was 5 mg/mL to obtain an OVA/PBS solution.

After mixing injection water, the aqueous solution of the hyaluronic acid derivative, and the OVA/PBS solution in this order, sucrose was added as a solid to make a 10% sucrose isotonic solution, and the mixture was thoroughly stirred at room temperature until a clear solution was obtained. A 10% sucrose solution containing 0.15 mg/mL of the OVA, 3.0 mg/mL of the hyaluronic acid derivative and 10 mM PB (pH 7.4) was obtained. The resultant was incubated at 75°C for one hour to obtain a composition containing a complex of the OVA and the hyaluronic acid derivative.

### [Comparative Example 6-1-1]

A composition containing a complex of an OVA and a hyaluronic acid derivative was obtained using the aqueous solution of the hyaluronic acid derivative obtained in Comparative Example 6-1 by encapsulating an OVA into the hyaluronic acid derivative by the same method as Example 6-1-1.

### [Antibody production test]

Each composition containing the complex of the OVA and the hyaluronic acid derivative prepared in Example 6-1-1 and Comparative Example 6-1-1 was administered subcutaneously to BALB/c mice on Days 0 and 7 such that the amount of OVA administered was 20 µg, followed by collecting the serum therefrom on Day 10. When the complex of the OVA and the hyaluronic acid derivative was administered, 50 µg of CpG1826 (manufactured by InvivoGen under the product code of tlrl-1826-1), which is an adjuvant, was administered subcutaneously. The antibody titer against the OVA was measured using an ELISA kit (manufactured by Conderx, Inc., mouse anti-OVA IgG antibody assay kit, mouse anti-OVA IgG1 antibody assay kit, and mouse anti-OVA IgG2a antibody assay kit) according to the attached protocol. The dilution ratios of the serum were 10, 50, 250, 1250, 6250, and 31250.

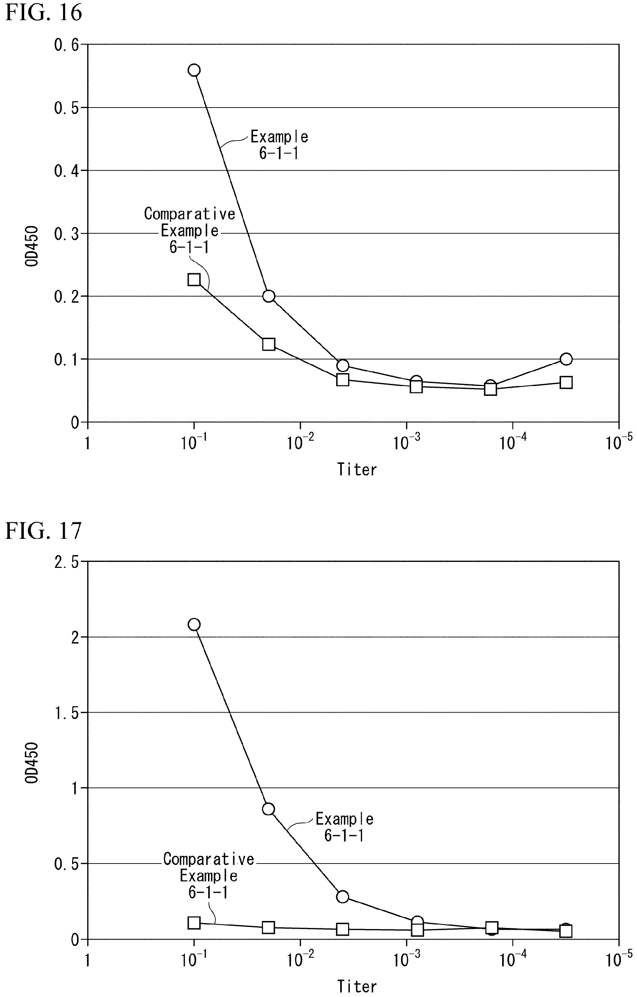

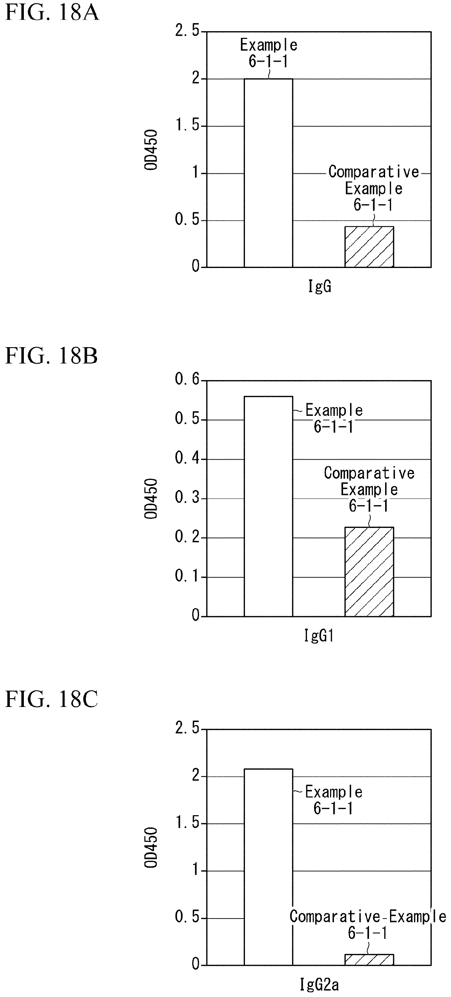

The IgG antibody titer (OD) calculated using the mouse anti-OVA IgG antibody assay kit is shown in Fig. 15, the IgG1 antibody titer (OD) calculated using the mouse anti-OVA IgG1 antibody assay kit is shown in Fig. 16, and the IgG2a antibody titer (OD) calculated using the mouse anti-OVA IgG2a antibody assay kit is shown in FIG. 17. Each antibody titer at a dilution ratio of 10-fold is shown in FIG. 18A (IgG antibody titer), FIG. 18B (IgG1 antibody titer), and FIG. 18C (IgG2a antibody titer).

Although no difference was confirmed in the antibody titer when the dilution ratio of the serum was 1250-fold or more, it was confirmed that the antibody titer of the composition containing the complex of the OVA and the hyaluronic acid derivative of Example 6-1-1 was higher than that of the composition containing the complex of the OVA and the hyaluronic acid derivative of Comparative Example 6-1-1 when the dilution ratio was lower than 1250-fold. Thus, it became apparent that the hyaluronic acid derivative of Example 6-1 (A1/A2=2.03) had a higher ability of producing antibodies against foreign antigens than the hyaluronic acid derivative of Comparative Example 6-1 (A1/A2=0.81), thereby showing that the ability of producing antibodies for treatment in vivo was high.

It has been reported that mature dendritic cells to which antigens are added enhance antigen-specific IgG2a production through induction of type I helper T cells that produce IFN-γ (Reference Document 5: Shigeharu Fujita et al., "Regulatory dendritic cells protect against allergic airway inflammation in a murine asthmatic model.", J Allergy Clin Immunol 2008; 121: 95-104). It was presumed from the report in Reference Document 5 and the results of Test Example 4 that co-stimulatory molecules of DC were activated by the pharmaceutical composition containing the complex of the OVA and the hyaluronic acid derivative of Example 6-1-1, CD80 and CD86 were expressed strongly, and the abilities of inducing subsequent antigen-specific T cells and producing IFN-y were enhanced, thereby promoting the antigen-specific IgG2a production. Therefore, it is expected that the pharmaceutical composition of Example 6-1-1 induces not only humoral immunity but also cell-mediated immunity, thereby inducing a stronger immunoresponse as an infectious disease vaccine.

It was presumed that the use of the pharmaceutical composition of the present embodiment protected not only a peptide but also a protein antigen as a medicinal ingredient from heating conditions, allowed stable and efficient delivery to immune cells (especially, CD1 and macrophages) while maintaining an appropriate three-dimensional structure, allowed the uptake of the medicinal ingredient into immune cells to be improved, promoted, or enhanced, and simultaneously allowed the activity of immune cells (especially, expression of co-stimulatory molecules CD 80 and CD86 on cDC1) to be improved, promoted, maintained or enhanced.

It was presumed that the activation of the helper T cells was promoted, and the activation and expansion of B cells were further promoted, thereby producing the antigen against the epitope.

It is considered that the use of the hyaluronic acid derivatives of the first to third embodiments makes it possible to provide a superior vaccine against foreign antigens (mainly infectious disease vaccines) to the case where a conventional hyaluronic acid derivative is used. At the same time, it is considered that a pharmaceutical product containing an antigen that induces a therapeutic antibody can be provided.

### <Evaluation of properties as a cancer vaccine (antitumor test of cancer vaccine using an administration composition containing hyaluronic acid derivative and antigen)>

### [Test Example 5]

In order to clarify the antitumor effects of cancer vaccines using hyaluronic acid derivatives with different area ratios A1/A2, mice bearing tumor caused by fibrosarcoma cell line CMS5a resisant to an immune checkpoint inhibitor (ICI) were used to prepare cancer vaccines of hyaluronic acid derivatives loaded with long-chain peptide antigens formed by complexing a long-chain peptide containing a CD8 epitope of a mutant ERK2 antigen (mERK2) expressed in the CMS5a with various hyaluronic acid derivatives and treatment effects thereof were investigated.

Materials and methods are shown below.

### (1) Cells

RPMI 1640 medium (to which 2-mercaptoethanol was added) was purchased from Cell Science & Technology Institute, Inc.

Fetal bovine serum (FBS) was purchased from Gibco.

The mouse fibrosarcoma CMS5a cell line was provided by Memorial Sloan Kettering Cancer Institute and passaged at Mie University. The mouse fibrosarcoma CMS5a cell line expresses mutant ERK2 protein. A peptide (QYIHSANVL (SEQ ID No. 4)) containing a mutant portion of the mutant ERK2 protein is recognized by CD8-positive cytotoxic T cells of BALB/c mice. A T cell receptor (TCR) that recognizes this peptide has been isolated, and a transgenic mouse (DUC18 mouse) into which the TCR gene has been introduced has been created.

### (2) Test animals

Female BALB/c mice (CD90.2-positive) aged 6 to 8 weeks were purchased from Japan SLC, Inc.

Both mice were bred at the Mie University Advanced Science Research Promotion Center Animal Experiment Facility. The animal experiment protocol was approved by the Ethics Committee of Faculty of Medicine, Mie University.

### (3) Long-chain peptide antigen

A synthetic long-chain peptide antigen was purchased from Sigma Genosys Inc. The sequence was as follows.
NDHIAYFLYQILRGLQYIHSANVLHRDLKPSNLLLNT (SEQ ID No. 1).

The sequence includes: a nine amino acid sequence (QYIHSANVL (SEQ ID No. 4)) from the 16th Q to the 24th L as the CD8-positive cytotoxic T cell recognition epitope sequence of the mutant ERK2; and a 17 amino acid sequence (RGLQYIHSANVLHRDLK (SEQ ID No. 5)) from the 13th R to the 29th K as the CD4-positive helper T cell recognition epitope sequence.

A hyaluronic acid derivative loaded with the long-chain peptide antigen formed by complexing the long-chain peptide antigen and various hyaluronic acid derivatives is sometimes referred to as a "cancer vaccine based on HA nanogel loaded with the long-chain peptide antigen" or "cancer vaccine based on HA nanogel".

### [Example 7-1]

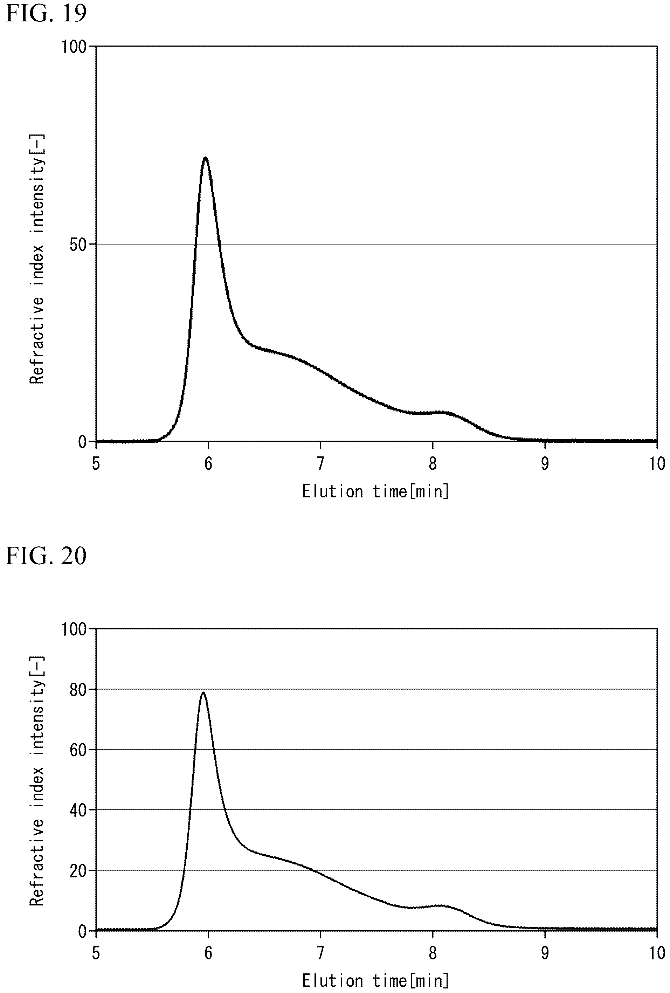

The hyaluronic acid derivative obtained in Comparative Example 6-1 was fractionated as follows. The freeze-dried hyaluronic acid derivative (10k HA-C₆-Chol-41.3%) was dissolved in injection water at 5 mg/mL, and transferred to a dialysis cassette (Float-A-Lyzer G2, MWCO: 300,000, Ieda Boeki Co., Ltd.), and subjected to dialysis with 10 mM phosphate buffer pH 7.4. The hyaluronic acid derivative in the obtained internal dialysate was subjected to a measurement by gel permeation chromatography. Fig. 19 shows the resulting gel permeation chromatogram. The particle size distribution of the hyaluronic acid derivative was evaluated by the same method as Example 1-1 or the like. The results are shown in Table 12.

**[Table 12]**

| | A1/A2 | Da/Db |
|---|---|---|
| Example 7-1 | 6.62 | 0.80 |

### <Production of pharmaceutical composition>

### [Example 7-1-1]

The concentration of the hyaluronic acid derivative in the aqueous solution of the hyaluronic acid derivative obtained in Example 7-1 was 3.8 mg/mL, the concentration being calculated with reference to the area of the hyaluronic acid derivative at a known concentration.

In contrast, an antigenic peptide was dissolved in dimethyl sulfoxide (DMSO) in another container such that the concentration thereof became 50 mg/mL, thereby obtaining a DMSO solution of the antigenic peptide. A peptide manufactured by Biologica, Inc., and having the following amino acid sequence was used as the antigenic peptide.

NDHIAYFLYQILRGLQYIHSANVLHRDLKPSNLLLNT (SEQ ID No. 1)

A complex of the antigenic peptide and the hyaluronic acid was tried to be prepared by mixing the aqueous solution of the hyaluronic acid derivative and the DMSO solution of the antigenic peptide at a ratio of 10 parts by mass of the peptide to 100 parts by mass of the hyaluronic acid derivative at room temperature (about 25°C), and then stirring the mixture at room temperature (about 25°C) for two hours. When the state of the solution at this time was visually confirmed, a clear solution was obtained, as confirmed by the photograph indicated as "hyaluronic acid derivative" in FIG. 11. Thus, it was confirmed that the peptide was reliably encapsulated.

Thereafter, a solid purified sucrose (manufactured by FUJIFILM Wako Pure Chemical Corporation, for production only, under the product number of 198-18385) was added to the composition containing the complex of the peptide and the hyaluronic acid derivative such that the amount of sucrose relative to the total mass of the composition and the sucrose became 10% by mass, and stirred at room temperature (approximately 25°C) for one hour or more. After confirming that the sucrose was sufficiently dissolved, the resultant was diluted with 10 mM phosphate buffer pH 7.4 containing 10% by mass of sucrose such that the concentration of the peptide became about 0.3 mg/mL, followed by subjecting the resultant to sterilization filtration using a 0.2 µm PES (polyethersulfone) (manufactured by Pall Corporation, acrodisc syringe filter, 25 mmΦ) to obtain a pharmaceutical composition containing a complex of the peptide and the hyaluronic acid derivative. The amount of the peptide in the pharmaceutical composition was quantified by a high-performance liquid chromatography measurement in the same manner as in Example 2-1. The amount of the peptide relative to 100 parts by mass of the pharmaceutical composition was 0.383 parts by mass. In addition, the pharmaceutical composition containing the complex of the peptide and the hyaluronic acid derivative was diluted with 10 mM phosphate buffer pH 7.4 containing 10% by mass of sucrose such that the concentration of the peptide became 250 µg/mL to obtain an administration liquid of the pharmaceutical composition. The pharmaceutical composition containing the complex of the peptide and the hyaluronic acid derivative was subjected to measurement by gel permeation chromatography by the method described in Example 1-1 under the following measurement conditions. Fig. 20 shows the resulting gel permeation chromatogram.

### (Measurement conditions)

Device: HLC8420-GPC (manufactured by TOSOH CORPORATION)
Column: G4000SWXL (manufactured by TOSOH CORPORATION, particle size 8 µm, inner diameter 7.8 mm, length 30 cm, product number:8542)
Eluent: 10 mM phosphate buffer (pH 7.4)
Flow rate: 1 mL/min
Injection amount: 50 µL
Detector: RI
Temperature: 30°C

### [Comparative Example 7-1-1]

The peptide used in Example 7-1-1 was dissolved in dimethyl sulfoxide (DMSO) to a concentration of 50 mg/mL. The resultant was diluted with 10 mM phosphate buffer pH 7.4 containing 10% by mass sucrose to a concentration of 0.25mg/mL.

### [Test Example 6]

### Tumor growth test

A mouse fibrosarcoma CMS5a cell line was cultured in RPMI 1640 medium containing 10% FBS using T75 culture flasks (Corning). The cultured cells were detached using PBS containing 0.5% trypsin and suspended in RPMI 1640 medium containing 10% FBS. The suspension was centrifuged (400×g, five minutes, 4°C) and the supernatant was removed. Thereafter, the cells were washed twice with RPMI 1640 medium and suspended in RPMI 1640 medium at a concentration of 1×10⁶ cells/100 µL. The cells were implanted subcutaneously in the right anterior dorsal region of BALB/c mice (five mice per group) at a dose of 100 µL/mouse.

When the cancer vaccine based on HA nanogel loaded with the long-chain peptide antigen was administered, 50 µg of the cancer vaccine based on HA nanogel loaded with the long-chain peptide antigen was administered subcutaneously to the right posterior dorsal region of the mice together with 50 µg of CpG oligo DNA (Ajinomoto Bio-Pharma Services, GeneDesign) dissolved in PBS 7 days, 10 days, 13 days, and 16 days after tumor implantation. Thereafter, the tumor area was measured over time.

When the long-chain peptide antigen of Comparative Example 7-1-1 was administered, 50 µg of the long chain peptide was administered subcutaneously to the right posterior dorsal region of the mice together with 50 µg of CpG oligo DNA (Ajinomoto Bio-Pharma Services, GeneDesign) dissolved in PBS 7 days, 10 days, 13 days, and 16 days after tumor implantation. Thereafter, the tumor area was measured over time.

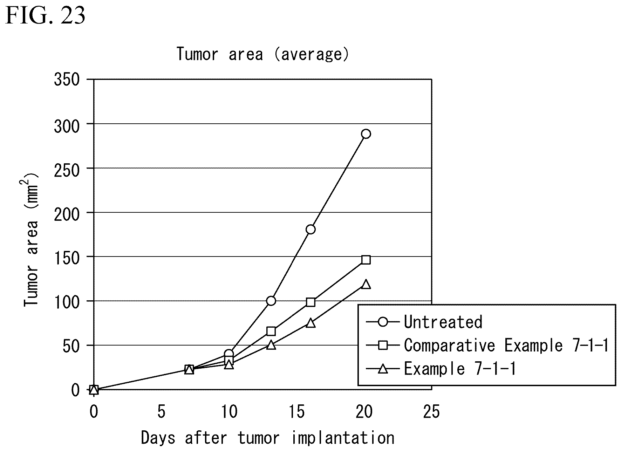

Figure 23 shows the tumor area (average values) over time.

It was revealed that the pharmaceutical composition containing: the cancer vaccine based on HA nanogel loaded with the long-chain peptide antigen obtained by conjugating the hyaluronic acid derivative (A1/A2 = 6.62) of Example 7-1 with the long-chain peptide antigen; and the CpG oligo DNA suppressed the growth of CMS5a tumor in comparison with the pharmaceutical composition containing the long-chain peptide antigen of Comparative Example 7-1 and the CpG oligo DNA. Thus, it is considered that the use of the hyaluronic acid derivative with A1/A2=6.62 makes it possible to provide an excellent cancer vaccine.

### <Influence of retention time at the peak-top (the maximum point of the refractive index intensity) by size elimination chromatography (SEC) on immune cell activation and vaccine properties)>

### [Test Example 7]

### [Peak-top retention time ratio in particle size distribution of hyaluronic acid derivative]

Samples of the hyaluronic acid derivatives obtained in Synthesis Examples 1-1 to 1-3, Examples 1-1 to 1-3, 6-1, and 7-1 and Comparative Examples 1-1 to 1-3, 6-1, 7-1, and 7-2, and a polyacrylic acid (50 kDa) as a standard substance were prepared by the method as described in Example 1-1, and subjected to measurement by gel permeation chromatography under the following conditions.

### (Measurement conditions)

Device: HLC8420-GPC (manufactured by TOSOH CORPORATION)
Column: G4000SWXL (manufactured by TOSOH CORPORATION, particle size 8 µm, inner diameter 7.8 mm, length 30 cm, product number:8542)
Eluent: 10 mM phosphate buffer (pH 7.4)
Flow rate: 1 mL/min
Injection amount: 50 µL
Detector: RI
Temperature: 30°C

The peak-top retention times Pt of the hyaluronic acid derivatives obtained in Synthesis Examples 1-1 to 1-3, Examples 1-1 to 1-3, 6-1, and 7-1, and Comparative Examples 1-1 to 1-3, 6-1, 7-1, and 7-2, and the peak-top retention time Pr of a polyacrylic acid (50 kDa) as a standard substance are shown in the following Table 13. The ratio Pt/Pr of the peak-top retention time Pt of the hyaluronic acid derivative to the peak-top retention time Pr of a polyacrylic acid (50 kDa) as a standard substance was calculated and described in the following Table 13.

**[Table 13]**

| | Peak-top retention time | | Pt/Pr |
|---|---|---|---|
| | Pt (min) | Pr (min) | |
| Synthesis Example 1-1 | 8.61 | - | 1.14 |
| Synthesis Example 1-2 | 8.33 | - | 1.11 |
| Synthesis Example 1-3 | 8.64 | - | 1.15 |
| Example 1-1 | 5.99 | - | 0.80 |
| Example 1-2 | 6.02 | - | 0.80 |
| Example 1-3 | 5.97 | - | 0.79 |
| Comparative Example 1-1 | 8.64 | - | 1.15 |
| Comparative Example 1-2 | 8.42 | - | 1.12 |
| Comparative Example 1-3 | 8.67 | - | 1.15 |
| Example 6-1 | 6.86 | - | 0.91 |
| Comparative Example 6-1 | 8.01 | - | 1.06 |
| Example 7-1 | 5.97 | - | 0.79 |
| Comparative Example 7-1 | 8.01 | - | 1.06 |
| Comparative Example 7-2 | 8.08 | - | 1.07 |
| Polyacrylic acid (50 kDa) | - | 7.53 | - |

In the case of an associated particle size in which the peak-top retention time Pt of the hyaluronic acid derivative was shorter than the peak top retention time Pr of the polyacrylic acid (50 kDa) as a standard substance, it is assumed that a larger amount of associated products having a size suitable to activate co-stimulatory molecules is present, the use of the pharmaceutical composition of the present embodiment makes it possible to stably and efficiently deliver the medicinal ingredient such as a peptide or protein to immune cells (especially cDC1 or macrophages), furthermore improve, promote, or enhance the uptake of the medicinal ingredient into immune cells, and simultaneously improve, promote, maintain or enhance the activation of the immune cells (especially, expression of co-stimulatory molecules CD80 and CD86 on cDC1).

### <Influence of average molecular weight (based on polyacrylic acid) by GPC-SEC on immune cell activation and vaccine properties)>

### [Test Example 8]

The weight-average molecular weight (Mw) and the number-average molecular weight (Mn) (based on polyacrylic acid) of the hyaluronic acid derivatives obtained in Synthesis Examples 1-1 to 1-3, Examples 1-1 to 1-3, 6-1, and 7-1, and Comparative Examples 1-1 to 1-3 and 6-1 were measured under the following conditions. A calibration curve was created using polyacrylic acids as standard substances and each molecular weight of the hyaluronic acid derivatives obtained in Synthesis Examples 1-1 to 1-3, Examples 1-1 to 1-3, 6-1, and 7-1, and Comparative Examples 1-1 to 1-3 and 6-1, was calculated based on polyacrylic acids. As the polyacrylic acid standard substances, PSS-Paa 2k (2 kDa), 4k (4 kDa), 8k (8 kDa), 18k (18 kDa), 40k (40 kDa), and 150k (150 kDa) (manufactured by PSS Polymer Standard service GmbH (sodium polyacrylates)) were used. Results are shown in Table 14.

### (Measurement conditions)

Device: HLC8420-GPC (manufactured by TOSOH CORPORATION)
Column: G4000SWXL (manufactured by TOSOH CORPORATION, particle size 8 µm, inner diameter 7.8 mm, length 30 cm, product number:8542)
Eluent: 10 mM phosphate buffer (pH 7.4)
Flow rate: 1 mL/min
Injection amount: 50 µL
Detector: RI
Sample concentration: The concentration of the hyaluronic acid derivatives obtained in the synthesis examples, examples, and comparative examples was 1 mg/mL, and that of the polyacrylic acids as standard substances was 2 mg/mL.
Calibration curve condition: The cubic equation: At3+Bt2+Ct+D was used as an approximation equation.

**[Table 14]**

| | Mw | Mw/Mn |
|---|---|---|
| Synthesis Example 1-1 | 69201 | 2.784 |
| Synthesis Example 1-2 | 86393 | 2.293 |
| Example 1-1 | 133892 | 2.565 |
| Example 1-2 | 152674 | 2.073 |
| Comparative Example 1-1 | 45799 | 2.209 |
| Comparative Example 1-2 | 43722 | 1.826 |
| Example 6-1 | 137659 | 2.201 |
| Comparative Example 6-1 | 74840 | 1.825 |
| Example 7-1 | 204104 | 2.056 |

When the weight-average molecular weight Mw (based on polyacrylic acid) of the hyaluronic acid derivative was 110,000 or more, the weight-average molecular weight Mw being calculated based on polyacrylic acids as standard substances, it was assumed that a larger amount of associated products having a size suitable to activate co-stimulatory molecules was present, the use of the pharmaceutical composition of the present embodiment made it possible to stably and efficiently deliver the medicinal ingredient such as a peptide to immune cells (especially cDC1 or macrophages), furthermore improve, promote, or enhance the uptake of the medicinal ingredient into immune cells, and simultaneously improve, promote, maintain or enhance the activation of the immune cells (especially, expression of co-stimulatory molecules CD80 and CD86 on cDC1).

### <Fractionation method by SEC (size elimination chromatography)>

### [Example 8]

The fractionation method is not limited to a fractionation method using a dialysis membrane.

The hyaluronic acid derivative obtained in Synthesis Example 1-1 was dissolved in injection water at 20 mg/mL, followed by subjecting to fractionation by gel permeation chromatography under the following conditions. For example, fractionation was carried out every 0.1 minutes.

### (Measurement conditions)

Device: HITACHI, CHROMASTER
Column: G4000SWXL (manufactured by TOSOH CORPORATION, particle size 8 µm, inner diameter 7.8 mm, length 30 cm, product number:8542)
Guard column: TSKGEL GUARDCOLUMN SWXL (6.0 mm I.D.X. 4CM)
Eluent: 10 mM phosphate buffer (pH 7.4)
Flow rate: 1 mL/min
Injection amount: 100 mL
Detector: RI

After the fractionation, GPC analysis was carried out by the method described in Synthesis Example 1-1 to confirm that the fraction had an area ratio A1/A of 0.90 or more, thereby obtaining the target hyaluronic acid derivative.

### <Production conditions of pharmaceutical composition that maintain appropriate particle size distribution>

### [Examples 9-1 to 9-4 and Comparative Examples 9-1 to 9-4]

An aqueous solution containing 5 mg/mL of the hyaluronic acid derivative fractionated by the same procedure as in Example 1-1 was prepared to examine the mixing ratio of the aqueous phase containing the hyaluronic acid derivative and the oil phase in detail.

A peptide drug, cyclosporine (manufactured by Tokyo Chemical Industry Co., Ltd., under the product number of C2408), was weighed in a 6 mL clean vial, and dissolved in DMSO (manufactured by FUJIFILM Wako Pure Chemical Corporation under the product number of 045-24511) such that the concentration of the peptide was 50 mg/mL).

Then, 4 µL of 50 mg/mL of cyclosporin, which was an oil phase containing the drug, was added to 400 µL of the 5 mg/mL of the hyaluronic acid derivative. Thereafter, the oil phase DMSO was mixed as shown in Table 15 below. After mixing sufficiently, injection water was added as shown in Table 15 below such that the final concentration of the hyaluronic acid derivative became constant. Furthermore, after incubation at room temperature (25°C) for 24 hours, the resultant was subjected to measurement by gel permeation chromatography under the following conditions, and the area ratio A1/A2 was calculated in the same manner as in Example 1-1 (Table 15).

### (Measurement conditions)

Device: HLC8420-GPC (manufactured by TOSOH CORPORATION)
Column: G4000SWXL (manufactured by TOSOH CORPORATION, particle size 8 µm, inner diameter 7.8 mm, length 30 cm, product number:8542)
Eluent: 10 mM phosphate buffer (pH 7.4)
Flow rate: 1 mL/min
Injection amount: 50 µL
Detector: RI
Temperature: 30°C

**[Table 15]**

| Example/ Comparative Example | Oil phase DMSO Mixed amount (µL) | Injection water Mixed amount (µL) | A1/A2 |
|---|---|---|---|
| Example 9-1 | 0 | 396 | 1.46 |
| Example 9-2 | 16 | 380 | 1.37 |
| Example 9-3 | 36 | 360 | 1.25 |
| Example 9-4 | 76 | 320 | 1.03 |
| Comparative Example 9-1 | 156 | 240 | 0.69 |
| Comparative Example 9-2 | 236 | 160 | 0.43 |
| Comparative Example 9-3 | 316 | 80 | 0.30 |
| Comparative Example 9-4 | 396 | 0 | 0.17 |

It was revealed from the results of Examples 9-1 to 9-4 and Comparative Examples 9-1 to 9-4 that the particle size of the hyaluronic acid derivative, which exhibited excellent medicinal efficacy, could be maintained through the mixing step of making the mixing ratio, based on volume, of an oil phase and an aqueous phase containing the hyaluronic acid derivative be 20:100 to 0.01:100.

### INDUSTRIAL APPLICABILITY

The hyaluronic acid derivative of the present embodiment makes it possible to provide a hyaluronic acid derivative that has excellent delivery properties to immune cells in lymph nodes and activation abilities against the immune cells when formulated with the medicinal ingredient. The pharmaceutical composition of the present embodiment contains the hyaluronic acid derivative and has excellent delivery properties to immune cells in lymph nodes and activation abilities against the immune cells. The method for producing a pharmaceutical composition of the present embodiment uses the hyaluronic acid derivative and makes it possible to provide a pharmaceutical composition that has excellent delivery properties to immune cells in lymph nodes and activation abilities against the immune cells.

## Claims

1. A hyaluronic acid derivative having an introduced steryl group, wherein
a ratio A1/A2 of areas A1 and A2 calculated by a method mentioned below from chromatograms obtained by gel permeation chromatography measurements is 0.90 or more,
(i) an intersection point Ub is determined by: a perpendicular line drawn from a maximum point Kb of a refractive index intensity on the chromatogram of a 50 kDa polyacrylic acid serving as a standard substance to a baseline B; and the chromatogram of the hyaluronic acid derivative; and
(ii) the area A2 surrounded by: a curve from the intersection point Ub to an end point on the chromatogram of the hyaluronic acid derivative; a perpendicular line drawn from the maximum point Kb of the refractive index intensity to the baseline B; and the baseline is determined, and the area A1 surrounded by: a curve from a starting point to the intersection point Ub on the chromatogram of the hyaluronic acid derivative; the perpendicular line drawn from the maximum point Kb of the refractive index intensity to the baseline B; and the baseline is determined.

2. The hyaluronic acid derivative according to claim 1, wherein a ratio Da/Db of distances Da and Db calculated by a method described below from chromatograms obtained by a gel permeation chromatography measurement is more than 0.00 and 1.20 or less,
(i) an intersection point Ba is determined by: a perpendicular line drawn from a maximum point Ka of a refractive index intensity on the chromatogram of a 150 kDa polyacrylic acid serving as a standard substance to the baseline B; and the baseline, and a length La between the maximum point Ka of the refractive index intensity and the intersection point Ba is determined;
(ii) among two points at which the refractive index intensity on the chromatogram reaches La/20, one point at which an elution time is earlier is determined as a point R1, and another point at which the elution time is later is determined as a point S1;
(iv) an intersection point Ta is determined by: a straight line D1 that connects the point R1 and the point S1; and the perpendicular line drawn from the maximum point Ka of the refractive index intensity to the baseline B, and an intersection point Tb is determined by: the perpendicular line drawn from the maximum point Kb of the refractive index intensity to the baseline B; and the straight line D1; and
(v) the distance Da is determined as a distance between the point R1 and the intersection point Ta, and the distance Db is determined as a distance between the intersection point Ta and the intersection point Tb.

3. The hyaluronic acid derivative according to claim 1 or 2, wherein the ratio A1/A2 of the areas A1 and A2 is 1.70 or more.

4. A hyaluronic acid derivative having an introduced steryl group, wherein
a ratio Pt/Pr of a retention time Pt at a maximum point of a refractive index intensity of the hyaluronic acid derivative relative to a retention time Pr at a maximum point of a refractive index intensity of a 50 kDa polyacrylic acid as a standard substance, calculated from chromatograms obtained by gel permeation chromatography measurements, is 0.5 or more and less than 1.0.

5. A hyaluronic acid derivative having an introduced steryl group, wherein
a weight-average molecular weight of the hyaluronic acid derivative is 110,000 or more and less than 500,000, the weight-average molecular weight being calculated from a chromatogram obtained by a gel permeation chromatography measurement based on a calibration curve obtained using polyacrylic acids having a molecular weight of 2 kDa, 4 kDa, 8 kDa, 18 kDa, 40 kDa, or 150 kDa as standard substances.

6. The hyaluronic acid derivative according to claim 1, 4 or 5, wherein the hyaluronic acid derivative comprises at least one repeating unit of general formula (I): 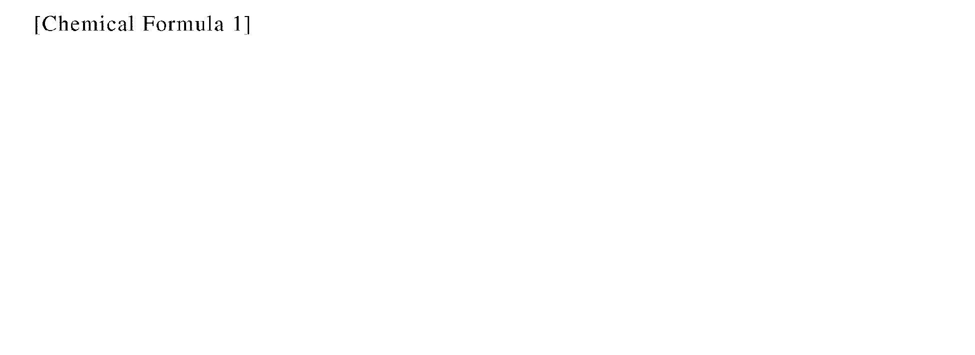 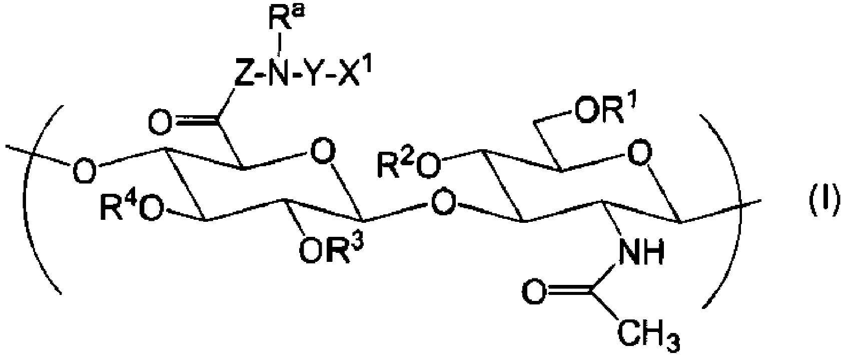
(in the general formula, each of R¹, R², R³, and R⁴ is independently selected from the group consisting of a hydrogen atom, C₁₋₆ alkyls, a formyl and C₁₋₆ alkylcarbonyls;
Z is a direct bond or a peptide linker consisting of 2 to 30 arbitrary amino acid residues;
X¹ is a group selected from the group consisting of:
-NR^{b}-R,
-NR^{b}-COO-R,
-NR^{b}-CO-R,
-NR^{b}-CO-NR^{c}-R,
-COO-R,
-O-COO-R,
-S-R,
-CO-Y^{a}-S-R,
-O-CO-Y^{b}-S-R,
-NR^{b}-CO-Y^{b}-S-R, and
-S-S-R;
each of R^{a}, R^{b} and R^{c} is independently selected from the group consisting of a hydrogen atom, C₁₋₂₀ alkyls, amino C₂₋₂₀ alkyls and hydroxy C₂₋₂₀ alkyls, in an alkyl moiety of which a group selected from the group consisting of -O- and -NR^{f}- may be inserted;
R^{f} is selected from the group consisting of a hydrogen atom, C₁₋₁₂ alkyls, amino C₂₋₁₂ alkyls and hydroxy C₂₋₁₂ alkyls, in an alkyl moiety of which a group selected from the group consisting of -O- and -NH- may be inserted;
R is a steryl group;
Y is C₂₋₃₀ alkylene or (CH₂CH ₂O)ₘ-CH₂CH₂-, and a group selected from the group consisting of -O-, -NR^{g}- and -S-S- may be inserted in the alkylene;
R^{g} is selected from the group consisting of a hydrogen atom, C₁₋₂₀ alkyls, amino C₂₋₂₀ alkyls and hydroxy C₂₋₂₀ alkyls, in an alkyl moiety of which a group selected from the group consisting of -O- and -NH- may be inserted;
Y^{a} is a C₁₋₅ alkylene;
Y^{b} is a C₂₋₈ alkylene or a C₂₋₈ alkenylene; and
m is an integer of 1 or more and 100 or less.)

7. The hyaluronic acid derivative according to claim 1, 4 or 5, wherein the steryl group is a cholesteryl group.

8. The hyaluronic acid derivative according to claim 1, 4 or 5, wherein an introduction ratio of the steryl group relative to a disaccharide repeating unit that constitutes the hyaluronic acid derivative is 30% or more and 60% or less.

9. The hyaluronic acid derivative according to claim 1, 4 or 5, wherein the weight-average molecular weight (absolute molecular weight) of the hyaluronic acid derivative is 4,000 or more and 1,000,000 or less.

10. The hyaluronic acid derivative according to claim 9, wherein the weight-average molecular weight (absolute molecular weight) of the hyaluronic acid derivative is 5,000 or more and 25,000 or less.

11. A pharmaceutical composition comprising: a hyaluronic acid derivative of clam 1, 4 or 5; and a medicinal ingredient.

12. The pharmaceutical composition according to claim 11, wherein the pharmaceutical composition is used to prevent or treat at least one disease selected from the group consisting of cancers, infectious diseases and immune diseases.

13. The pharmaceutical composition according to claim 11, wherein the medicinal ingredient comprises at least one selected from the group consisting of cancer antigens, antigens derived from infectious diseases, and autoantigens in immune diseases, and the pharmaceutical composition further comprises an adjuvant.

14. The pharmaceutical composition according to claim 13, wherein the medicinal ingredient comprises a cancer antigen or an antigen derived from infectious diseases.

15. A method for producing a pharmaceutical composition comprising a hyaluronic acid derivative of claim 1, 4 or 5 and a medicinal ingredient, the method comprising:
a preparation step in which the medicinal ingredient is dissolved in an organic solvent or an organic solvent containing water to prepare an oil phase comprising the medicinal ingredient; and
a mixing step in which the oil phase and an aqueous phase comprising the hyaluronic acid derivative are mixed such that a mixing ratio of the oil phase and the aqueous phase, based on volume, becomes 20:100 to 0.01:100.

16. The method for producing a pharmaceutical composition according to claim 15, wherein a pH of the aqueous phase comprising the hyaluronic acid derivative is 6.00 or more and 11.00 or less.
